# EUROPEAN PATENT APPLICATION

(11) **EP 4 660 201 A1**
(43) Date of publication of application: **10.12.2025**
(21) Application number: 24741343.8
(22) Date of filing: 12.01.2024
(51) Int. Cl.: C07K 16/28, C12N 5/10, A61K 38/17, A61K 39/00, A61P 35/00, C12N 15/13

(54) **ANTIGEN BINDING PROTEIN AND USE THEREOF**

(30) Priority: 13.01.2023 CN 202310065643; 13.01.2023 CN 202310067716; 29.01.2023 CN 202310104552; 17.02.2023 CN 202310131538; 17.02.2023 CN 202310131539
(71) Applicant: Corregene Biotechnology Co., Ltd., Beijing 102200 (CN)
(72) Inventor: XIE, Xingwang, Beijing 102206 (CN); JIANG, Dong, Beijing 102206 (CN); ZHAI, Jiahui, Beijing 102206 (CN); YAN, Baoqi, Beijing 102206 (CN); WANG, Jianghua, Beijing 102206 (CN); BI, Jinglei, Beijing 102206 (CN); SHI, Yunqiang, Beijing 102206 (CN); FU, Shuangli, Beijing 102206 (CN)
(74) Representative: J A Kemp LLP
(86) International application number: PCT/CN2024/071910
(87) International publication number: WO 2024/149347

(57) **Abstract**

Disclosed are an antigen-binding protein, a multispecific antibody, a nucleic acid encoding an antigen-binding protein or a multispecific antibody, a vector comprising the nucleic acid, a cell comprising an antigen-binding protein, a multispecific antibody, a nucleic acid or a vector, and a method for preparing a cell. Further disclosed are a conjugate or composition comprising an antigen-binding protein or a multispecific antibody, a method for preventing and/or treating a disorder by using an antigen-binding protein, a multispecific antibody or a cell, and a method for detecting the presence of a disorder in a subject.

## Description

This application claims the benefit of priority to Chinese Patent Applications No. 202310065643.3 filed on January 13, 2023; Chinese Patent Applications No. 202310067716.2 filed on January 13, 2023; Chinese Patent Applications No. 2023101045526 filed on January 29, 2023; Chinese Patent Applications No. 202310131538.5 filed on February 17, 2023; and Chinese Patent Applications No. 202310131539.X filed on February 17, 2023, each of which is incorporated herein by reference in its entirety for all purposes.

### Technical Field

The present disclosure relates to the field of immunology, in particular to T cell receptors (TCRs) and uses thereof, especially the use in the prevention, treatment, or detection of KRAS mutation- positive diseases and/or disorders.

### Background Art

The RAS gene family includes three genes: NRAS, HRAS, and KRAS, which encode small GTPase membrane-associated proteins that play a major role in signal transduction regulating cell growth. RAS acts as a central mediator in cell signaling pathways: at the molecular level, it regulates processes such as transcription and translation; at the cellular level, it modulates cell proliferation, differentiation, senescence, and apoptosis.

As early as 1982, researchers identified a mutated RAS gene in human bladder cancer cells, making RAS the first discovered human oncogene. Among the RAS genes, KRAS has the greatest impact on human cancers. When in an activated state, KRAS transmits upstream signals from cell surface receptors to downstream pathways, thereby regulating normal cellular functions and proliferation. The most common activation of KRAS occurs through point mutations, which are among the most frequent mutations in solid tumors. These mutations are almost exclusively located at codon 12 of exon 2, with the most common types being G12D, G12V, and G12C.

Although the critical role of KRAS mutations in tumors has been widely recognized, no drug targeting KRAS has been approved to date. In recent years, some progress has been made with novel drugs specifically targeting KRAS G12C mutations: small molecule drugs targeting KRAS can directly inhibit KRAS protein activity. However, because KRAS protein is widely expressed, indiscriminate inhibition of both wild-type and mutant KRAS proteins may result in unacceptable toxicity. Another method to directly inhibit KRAS activity is to interfere with its peripheral membrane association, but this also carries the risk of toxicity due to the difficulty in distinguishing mutant from wild-type proteins. KRAS regulates numerous downstream effector molecules, mainly via the RAF/MEK/ERK and PI3K/AKT/mTOR pathways. However, due to extensive feedback mechanisms, clinical trials have shown that the efficacy of various downstream inhibitors is limited.

A wealth of research suggests that KRAS mutant antigen epitopes represent targets that can be efficiently recognized by TCRs, and TCRs specific for KRAS mutant antigens are a promising approach for the treatment of KRAS-mutant solid tumors. The development of KRAS mutation-specific TCR-T products holds significant clinical relevance.

### Disclosure of the Invention

The objective of the present disclosure is to provide a novel antigen binding protein that specifically binds to a KRAS mutant antigen, particularly to a KRAS G12 mutant epitope or a complex of said epitope with an MHC molecule, such as a complex of a KRAS G12 mutant epitope with HLA-A*11. The antigen-binding protein of the present disclosure may be in the form of a T cell receptor (TCR) or an antigen-binding fragment thereof. The antigen-binding protein of the present disclosure is capable of binding the target antigen peptide with high affinity, exhibits excellent expression stability and high membrane stability, and is capable of mediating the specific killing of antigen-positive target cells by effector cells, while exhibiting no alloreactivity toward different HLA subtypes.

Accordingly, in one aspect, the present disclosure provides an antigen-binding protein comprising an antigen binding domain or a fragment thereof of a T cell receptor (TCR), the antigen binding domain or fragment thereof comprising a T cell receptor (TCR) alpha chain variable region (Va) and a TCR beta chain variable region (Vβ), wherein:
the Vα comprises a CDR3 having an amino acid sequence of X1X2X3X4X5X6X7X8X9X10X11X12X13LX15 (SEQ ID NO:1), wherein: X1 is A or V; X2 is E or V; X3 is R, P, N or null; X4 is D, G, S, L or null; X5 is I, G, D or null; X6 is E, T or null; X7 is G or null; X8 is A, G, T or null; X9 is G, S, A or null; X10 is N, Y or G; X11 is N, G, A or null; X12 is R or null; X13 is K or R; X15 is I, T or M; or
the Vα comprises a CDR3 having an amino acid sequence of X1X2X3X4X5X6GX8X9X10X11X12KLX15 (SEQ ID NO:2), wherein: X1 is A or V; X2 is E or V; X3 is R, P or N; X4 is D, G or S; X5 is I, G or D; X6 is E, T or null; X8 is A, G or T; X9 is G or S; X10 is N or Y; X11 is N, G or null; X12 is R or null; X15 is I or T; or
the Vα comprises a CDR3 having an amino acid sequence of X1VX3X4X6X7X8X9X10X11X13LX15 (SEQ ID NO:3), wherein: X1 is A or V; X3 is N or null; X4 is L or null; X6 is T or null; X7 is G or null; X8 is A or null; X9 is A or null; X10 is N or G; X11 is N or A; X13 is K or R; X15 is T or M; or
the Vα comprises a CDR3 having an amino acid sequence of SEQ ID NOs: 22, 23, 24, 25 or 26, or a functional variant thereof formed by insertion, deletion or substitution of one or more amino acids;
   and/or
the Vβ comprises a CDR3 having an amino acid sequence of ASX3X4X5X6X7X8X9X10X11X12X13X14 (SEQ ID NO:10), wherein: X3 is S or T; X4 is E, H, L, F or S; X5 is G, W, V, S or I; X6 is F, G, D or V; X7 is Y, A or S; X8 is T, Q, P, Y or S; X9 is E, D, N or G; X10 is Y, S, R or null; X11 is G or null; X12 is T, E, P or null; X13 is A, Q or L; X14 is F, Y or H; or
the Vβ comprises a CDR3 having an amino acid sequence of ASSX4X5X6X7X8X9X10X12X13X14 (SEQ ID NO:11), wherein: X4 is E, H or L; X5 is G, W or V; X6 is F or G; X7 is Y, A or S; X8 is T, Q or P; X9 is E or D; X10 is Y or null; X12 is T, E or null; X13 is A or Q; X14 is F or Y; or
the Vβ comprises a CDR3 having an amino acid sequence of ASX3X4X5X6SX8X9X10X11X12X13X14 (SEQ ID NO:12), wherein: X3 is S or T; X4 is F or S; X5 is S or I; X6 is D or V; X8 is Y or S; X9 is N or G; X10 is S or R; X11 is G or null; X12 is E or P; X13 is Q or L; X14 is F or H; or
the Vβ comprises a CDR3 having an amino acid sequence of SEQ ID NOs: 37, 38, 39, 40 or 41, or a functional variant thereof formed by insertion, deletion, or substitution of one or more amino acids;
and wherein the antigen binding domain or fragment thereof is antigen-specific for a mutant KRAS G12 epitope.

In some embodiments, the Vα comprises a CDR1 having an amino acid sequence of X1X2X3X4X5X6 (SEQ ID NO:4), wherein X1 is V, N or D; X2 is S or R; X3 is G or A; X4 is N or S; X5 is P, Q or D; X6 is Y or S.

In some embodiments, the Vα comprises a CDR1 having an amino acid sequence of XlSX3X4X5X6 (SEQ ID NO:5), wherein X1 is V or N; X3 is G or A; X4 is N or S; X5 is P, Q or D; X6 is Y or S.

In some embodiments, the Vα comprises a CDR1 having an amino acid sequence of X1X2X3SQS (SEQ ID NO:6), wherein X1 is N or D; X2 is S or R; X3 is G or A.

In some embodiments, the Vα comprises a CDR1 having an amino acid sequence of SEQ ID NOs: 27, 28, 29, 30 or 31, or a functional variant thereof formed by insertion, deletion or substitution of one or more amino acids.

In some embodiments, the Vα comprises a CDR2 having an amino acid sequence of X1X2X3X4X5NX7X8X9 (SEQ ID NO:7), wherein X1 is Y, V or null; X2 is I or Y; X3 is T, S, R or Y; X4 is G or S; X5 is D, G or null; X7 is L, M, G or null; X8 is V, D or null; X9 is K or null.

In some embodiments, the Vα comprises a CDR2 having an amino acid sequence of X1X2X3X4X5NX7X8X9 (SEQ ID NO:8), wherein X1 is Y, V or null; X2 is I or Y; X3 is T, S or R; X4 is G or S; X5 is D, G or null; X7 is L, M or null; X8 is V, D or null; X9 is K or null.

In some embodiments, the Vα comprises a CDR2 having an amino acid sequence of X1X2X3SX5NX7X8 (SEQ ID NO:9), wherein X1 is V or null; X2 is I or Y; X3 is S or Y; X5 is G or null; X7 is G or null; X8 is D or null.

In some embodiments, the Vα comprises a CDR2 having an amino acid sequence of SEQ ID NOs: 32, 33, 34, 35 or 36, or a functional variant thereof formed by insertion, deletion or substitution of one or more amino acids.

In some embodiments, the Vβ comprises a CDR1 having an amino acid sequence of X1X2HX4X5 (SEQ ID NO:13), wherein X1 is S, L or M; X2 is G or N; X4 is N, D, A or E; X5 is S, T or Y.

In some embodiments, the Vβ comprises a CDR1 having an amino acid sequence of X1GHX4X5 (SEQ ID NO:14), wherein X1 is S or L; X4 is N, D or A; X5 is S or T.

In some embodiments, the Vβ comprises a CDR1 having an amino acid sequence of X1X2HX4X5 (SEQ ID NO:15), wherein X1 is S or M; X2 is G or N; X4 is A or E; X5 is T or Y.

In some embodiments, the Vβ comprises a CDR1 having an amino acid sequence of SEQ ID NOs: 42, 43, 44, 45 or 46, or a functional variant thereof formed by insertion, deletion or substitution of one or more amino acids.

In some embodiments, the Vβ comprises a CDR2 having an amino acid sequence of X1X2NX4X5X6 (SEQ ID NO:16), wherein X1 is F, Y or S; X2 is N, Q or M; X4 is N, K or V; X5 is V, E or G; X6 is P, L or V.

In some embodiments, the Vβ comprises a CDR2 having an amino acid sequence of X1X2NX4X5X6 (SEQ ID NO:17), wherein X1 is F or Y; X2 is N or Q; X4 is N or K; X5 is V, E or G; X6 is P, L or V.

In some embodiments, the Vβ comprises a CDR2 having an amino acid sequence of X1X2NX4X5V (SEQ ID NO:18), wherein X1 is F or S; X2 is Q or M; X4 is N or V; X5 is E or G.

In some embodiments, the Vβ comprises a CDR2 having an amino acid sequence of SEQ ID NOs: 47, 48, 49, 50 or 51, or a functional variant thereof formed by insertion, deletion or substitution of one or more amino acids.

In some embodiments, the present disclosure provides a TCR or an antigen-binding fragment thereof, comprising a TCR α variable region (Vα) and a TCR β variable region (Vβ), wherein:
The Vα comprises a CDR3 having an amino acid sequence of X1X2X3X4X5X6X7X8X9X10X11X12X13LX15 (SEQ ID NO:1), wherein: X1 is A or V; X2 is E or V; X3 is R, P, N or null; X4 is D, G, S, L or null; X5 is I, G, D or null; X6 is E, T or null; X7 is G or null; X8 is A, G, T or null; X9 is G, S, A or null; X10 is N, Y or G; X11 is N, G, A or null; X12 is R or null; X13 is K or R; X15 is I, T or M; and
the Vα comprises a CDR1 having an amino acid sequence of X1X2X3X4X5X6 (SEQ ID NO:4), wherein: X1 is V, N or D; X2 is S or R; X3 is G or A; X4 is N or S; X5 is P, Q or D; X6 is Y or S; and
the Vα comprises a CDR2 having an amino acid sequence of X1X2X3X4X5NX7X8X9 (SEQ ID NO:7), wherein: X1 is Y, V or null; X2 is I or Y; X3 is T, S, R or Y; X4 is G or S; X5 is D, G or null; X7 is L, M, G or null; X8 is V, D or null; X9 is K or null;
   and/or
the Vβ comprises a CDR3 having an amino acid sequence of ASX3X4X5X6X7X8X9X10X11X12X13X14 (SEQ ID NO:10), wherein: X3 is S or T; X4 is E, H, L, F or S; X5 is G, W, V, S or I; X6 is F, G, D or V; X7 is Y, A or S; X8 is T, Q, P, Y or S; X9 is E, D, N or G; X10 is Y, S, R or null; X11 is G or null; X12 is T, E, P or null; X13 is A, Q or L; X14 is F, Y or H; and
the Vβ comprises a CDR1 having an amino acid sequence of X1X2HX4X5 (SEQ ID NO:13), wherein: X1 is S, L or M; X2 is G or N; X4 is N, D, A or E; X5 is S, T or Y; and
the Vβ comprises a CDR2 having an amino acid sequence of X1X2NX4X5X6 (SEQ ID NO:16), wherein: X1 is F, Y or S; X2 is N, Q or M; X4 is N, K or V; X5 is V, E or G; X6 is P, L or V; and
wherein the antigen binding domain or fragment thereof is antigen-specific for a mutant KRAS G12 epitope.

In some embodiments, the present disclosure provides a TCR or an antigen binding fragment thereof, comprising a TCR α variable region (Vα) and a TCR β variable region (Vβ), wherein:
the Vα comprises a CDR3 having an amino acid sequence of X1X2X3X4X5X6GX8X9X10X11X12KLX15 (SEQ ID NO:2), wherein X1 is A or V; X2 is E or V; X3 is R, P or N; X4 is D, G or S; X5 is 1, G or D; X6 is E, T or null; X8 is A, G or T; X9 is G or S; X10 is N or Y; X11 is N, G or null; X12 is R or null; X15 is I or T; or the Vα comprises a CDR3 having an amino acid sequence of SEQ ID NOs: 22, 23 or 24, or a functional variant thereof formed by insertion, deletion or substitution of one or more amino acids; and
the Vα comprises a CDR1 having an amino acid sequence of X1SX3X4X5X6 (SEQ ID NO:5), wherein X1 is V or N; X3 is G or A; X4 is N or S; X5 is P, Q or D; X6 is Y or S; or the Vα comprises a CDR1 having an amino acid sequence of SEQ ID NOs: 27, 28 or 29, or a functional variant thereof formed by insertion, deletion or substitution of one or more amino acids; and
the Vα comprises a CDR2 having an amino acid sequence of X1X2X3X4X5NX7X8X9 (SEQ ID NO:8), wherein X1 is Y, V or null; X2 is I or Y; X3 is T, S or R; X4 is G or S; X5 is D, G or null; X7 is L, M or null; X8 is V, D or null; X9 is K or null; or the Vα comprises a CDR2 having an amino acid sequence of SEQ ID NOs: 32, 33 or 34, or a functional variant thereof formed by insertion, deletion or substitution of one or more amino acids;
   and/or
the Vβ comprises a CDR3 having an amino acid sequence of ASSX4X5X6X7X8X9X10X12X13X14 (SEQ ID NO:11), wherein X4 is E, H or L; X5 is G, W or V; X6 is F or G; X7 is Y, A or S; X8 is T, Q or P; X9 is E or D; X10 is Y or null; X12 is T, E or null; X13 is A or Q; X14 is F or Y; or the Vβ comprises a CDR3 having an amino acid sequence of SEQ ID NOs: 37, 38 or 39, or a functional variant thereof formed by insertion, deletion or substitution of one or more amino acids; and
the Vβ comprises a CDR1 having an amino acid sequence of X1GHX4X5 (SEQ ID NO:14), wherein X1 is S or L; X4 is N, D or A; X5 is S or T; or the Vβ comprises a CDR1 having an amino acid sequence of SEQ ID NOs: 42, 43 or 44, or a functional variant thereof formed by insertion, deletion or substitution of one or more amino acids; and
the Vβ comprises a CDR2 having an amino acid sequence of X1X2NX4X5X6 (SEQ ID NO:17), wherein X1 is F or Y; X2 is N or Q; X4 is N or K; X5 is V, E or G; X6 is P, L or V; or the Vβ comprises a CDR2 having an amino acid sequence of SEQ ID NOs: 47, 48 or 49, or a functional variant thereof formed by insertion, deletion or substitution of one or more amino acids;
and wherein the antigen binding domain or fragment thereof is antigen-specific for a mutant KRAS G12V epitope.

In some embodiments, the Vα comprises a CDR3 having an amino acid sequence of X1VX3X4X6X7X8X9X10X11X13LX15 (SEQ ID NO:3), wherein X1 is A or V; X3 is N or null; X4 is L or null; X6 is T or null; X7 is G or null; X8 is A or null; X9 is A or null; X10 is N or G; X11 is N or A; X13 is K or R; X15 is T or M; or the Vα comprises a CDR3 having an amino acid sequence of SEQ ID NOs: 25 or 26, or a functional variant thereof formed by insertion, deletion, or substitution of one or more amino acids; and
the Vα comprises a CDR1 having an amino acid sequence of X1X2X3SQS (SEQ ID NO:6), wherein X1 is N or D; X2 is S or R; X3 is G or A; or the Vα comprises a CDR1 having an amino acid sequence of SEQ ID NOs: 30 or 31, or a functional variant thereof formed by insertion, deletion, or substitution of one or more amino acids; and
the Vα comprises a CDR2 having an amino acid sequence of X1X2X3SX5NX7X8 (SEQ ID NO:9), wherein: X1 is V or null; X2 is I or Y; X3 is S or Y; X5 is G or null; X7 is G or null; X8 is D or null; or the Vα comprises a CDR2 having an amino acid sequence of SEQ ID NOs: 35 or 36, or a functional variant thereof formed by insertion, deletion, or substitution of one or more amino acids;
   and/or
the Vβ comprises a CDR3 having an amino acid sequence of ASX3X4X5X6SX8X9X10X11X12X13X14 (SEQ ID NO:12), wherein X3 is S or T; X4 is For S; X5 is S or I; X6 is D or V; X8 is Y or S; X9 is N or G; X10 is S or R; X11 is G or null; X12 is E or P; X13 is Q or L; X14 is F or H; or the Vβ comprises a CDR3 having an amino acid sequence of SEQ ID NOs: 40 or 41, or a functional variant thereof formed by insertion, deletion, or substitution of one or more amino acids; and
the Vβ comprises a CDR1 having an amino acid sequence of X1X2HX4X5 (SEQ ID NO:15), wherein X1 is S or M; X2 is G or N; X4 is A or E; X5 is T or Y; or the Vβ comprises a CDR1 having an amino acid sequence of SEQ ID NOs: 45 or 46, or a functional variant thereof formed by insertion, deletion, or substitution of one or more amino acids; and
the Vβ comprises a CDR2 having an amino acid sequence of X1X2NX4X5V (SEQ ID NO:18), wherein X1 is F or S; X2 is Q or M; X4 is N or V; X5 is E or G; or the Vβ comprises a CDR2 having an amino acid sequence of SEQ ID NOs: 50 or 51, or a functional variant thereof formed by insertion, deletion, or substitution of one or more amino acids;
and wherein the antigen binding domain or fragment thereof is antigen-specific for a mutant KRAS G12D epitope.

In another aspect, the present disclosure provides an antigen-binding protein comprising an antigen binding domain or a fragment thereof of a T cell receptor (TCR), the antigen binding domain or fragment thereof comprising a T cell receptor (TCR) alpha chain variable region (Va) and a TCR beta chain variable region (Vβ), wherein:
the Vα comprises a CDR3 having an amino acid sequence as shown in SEQ ID NOs: 22, 23, 24, 25 or 26, or a functional variant thereof formed by insertion, deletion, or substitution of one or more amino acids; and/or the Vβ comprises a CDR3 having an amino acid sequence as shown in SEQ ID NOs: 37, 38, 39, 40 or 41, or a functional variant thereof formed by insertion, deletion, or substitution of one or more amino acids;
and wherein the antigen binding domain or fragment thereof is antigen-specific for a mutant KRAS G12 epitope.

In some embodiments, the Vα comprises a CDR1 having an amino acid sequence as shown in SEQ ID NOs: 27, 28, 29, 30 or 31, or a functional variant thereof formed by insertion, deletion, or substitution of one or more amino acids.

In some embodiments, the Vα comprises a CDR2 having an amino acid sequence as shown in SEQ ID NOs: 32, 33, 34, 35 or 36, or a functional variant thereof formed by insertion, deletion, or substitution of one or more amino acids.

In some embodiments, the Vβ comprises a CDR1 having an amino acid sequence as shown in SEQ ID NOs: 42, 43, 44, 45 or 46, or a functional variant thereof formed by insertion, deletion, or substitution of one or more amino acids.

In some embodiments, the Vβ comprises a CDR2 having an amino acid sequence as shown in SEQ ID NOs: 47, 48, 49, 50 or 51, or a functional variant thereof formed by insertion, deletion, or substitution of one or more amino acids.

In yet another aspect, the present disclosure provides an antigen-binding protein comprising an antigen binding domain or a fragment thereof of a T cell receptor (TCR), the antigen binding domain or fragment thereof comprising a T cell receptor (TCR) alpha chain variable region (Va) and a TCR beta chain variable region (Vβ), wherein:
the Vα comprises: a CDR1 having an amino acid sequence as shown in SEQ ID NOs: 27, 28, 29, 30 or 31, or a functional variant thereof formed by insertion, deletion, or substitution of one or more amino acids; a CDR2 having an amino acid sequence as shown in SEQ ID NOs: 32, 33, 34, 35 or 36, or a functional variant thereof formed by insertion, deletion, or substitution of one or more amino acids; a CDR3 having an amino acid sequence as shown in SEQ ID NOs: 22, 23, 24, 25 or 26, or a functional variant thereof formed by insertion, deletion, or substitution of one or more amino acids; and/or
the Vβ comprises: a CDR1 having an amino acid sequence as shown in SEQ ID NOs: 42, 43, 44, 45 or 46, or a functional variant thereof formed by insertion, deletion, or substitution of one or more amino acids; a CDR2 having an amino acid sequence as shown in SEQ ID NOs: 47, 48, 49, 50 or 51, or a functional variant thereof formed by insertion, deletion, or substitution of one or more amino acids; a CDR3 having an amino acid sequence as shown in SEQ ID NOs: 37, 38, 39, 40 or 41, or a functional variant thereof formed by insertion, deletion, or substitution of one or more amino acids.

In another aspect, the present disclosure provides an antigen-binding protein comprising an antigen binding domain or a fragment thereof of a T cell receptor (TCR), the antigen binding domain or fragment thereof comprising a T cell receptor (TCR) alpha chain variable region (Va) and a TCR beta chain variable region (Vβ), wherein:
the Vα comprises CDR1, CDR2, and CDR3 having amino acid sequences of SEQ ID NOs: 27, 32, and 22, respectively; and the Vβ comprises CDR1, CDR2, and CDR3 having amino acid sequences of SEQ ID NOs: 42, 47, and 37, respectively;
the Vα comprises CDR1, CDR2, and CDR3 having amino acid sequences of SEQ ID NOs: 28, 33, and 23, respectively; and the Vβ comprises CDR1, CDR2, and CDR3 having amino acid sequences of SEQ ID NOs: 43, 48, and 38, respectively;
the Vα comprises CDR1, CDR2, and CDR3 having amino acid sequences of SEQ ID NOs: 29, 34, and 24, respectively; and the Vβ comprises CDR1, CDR2, and CDR3 having amino acid sequences of SEQ ID NOs: 44, 49, and 39, respectively;
the Vα comprises CDR1, CDR2, and CDR3 having amino acid sequences of SEQ ID NOs: 30, 35, and 25, respectively; and the Vβ comprises CDR1, CDR2, and CDR3 having amino acid sequences of SEQ ID NOs: 45, 50, and 40, respectively;
or the Vα comprises CDR1, CDR2, and CDR3 having amino acid sequences of SEQ ID NOs: 31, 36, and 26, respectively; and the Vβ comprises CDR1, CDR2, and CDR3 having amino acid sequences of SEQ ID NOs: 46, 51, and 41, respectively.

In some embodiments, the Vα comprises an amino acid sequence having at least 80%, at least 85%, at least 90%, at least 95%, or 100% sequence identity to SEQ ID NOs: 52, 53, 54, 55, or 56; and/or the Vβ comprises an amino acid sequence having at least 80%, at least 85%, at least 90%, at least 95%, or 100% sequence identity to SEQ ID NOs: 57, 58, 59, 60, or 61. In some embodiments, the Vα comprises an amino acid sequence as shown in SEQ ID NOs: 52, 53, 54, 55, or 56; and/or the Vβ comprises an amino acid sequence as shown in SEQ ID NOs: 57, 58, 59, 60, or 61.

In some embodiments, the Vα comprises an amino acid sequence having at least 80%, at least 85%, at least 90%, at least 95%, or 100% sequence identity to SEQ ID NO: 52; and/or the Vβ comprises an amino acid sequence having at least 80%, at least 85%, at least 90%, at least 95%, or 100% sequence identity to SEQ ID NO: 57. Preferably, the Vα comprises an amino acid sequence as shown in SEQ ID NO: 52; and/or the Vβ comprises an amino acid sequence as shown in SEQ ID NO: 57.

In some embodiments, the Vα comprises an amino acid sequence having at least 80%, at least 85%, at least 90%, at least 95%, or 100% sequence identity to SEQ ID NO: 53; and/or the Vβ comprises an amino acid sequence having at least 80%, at least 85%, at least 90%, at least 95%, or 100% sequence identity to SEQ ID NO: 58. Preferably, the Vα comprises an amino acid sequence as shown in SEQ ID NO: 53; and/or the Vβ comprises an amino acid sequence as shown in SEQ ID NO: 58.

In some embodiments, the Vα comprises an amino acid sequence having at least 80%, at least 85%, at least 90%, at least 95%, or 100% sequence identity to SEQ ID NO: 54; and/or the Vβ comprises an amino acid sequence having at least 80%, at least 85%, at least 90%, at least 95%, or 100% sequence identity to SEQ ID NO: 59. Preferably, the Vα comprises an amino acid sequence as shown in SEQ ID NO: 54; and/or the Vβ comprises an amino acid sequence as shown in SEQ ID NO: 59.

In some embodiments, the Vα comprises an amino acid sequence having at least 80%, at least 85%, at least 90%, at least 95%, or 100% sequence identity to SEQ ID NO: 55; and/or the Vβ comprises an amino acid sequence having at least 80%, at least 85%, at least 90%, at least 95%, or 100% sequence identity to SEQ ID NO: 60. Preferably, the Vα comprises an amino acid sequence as shown in SEQ ID NO: 55; and/or the Vβ comprises an amino acid sequence as shown in SEQ ID NO: 60.

In some embodiments, the Vα comprises an amino acid sequence having at least 80%, at least 85%, at least 90%, at least 95%, or 100% sequence identity to SEQ ID NO: 56; and/or the Vβ comprises an amino acid sequence having at least 80%, at least 85%, at least 90%, at least 95%, or 100% sequence identity to SEQ ID NO: 61. Preferably, the Vα comprises an amino acid sequence as shown in SEQ ID NO: 56; and/or the Vβ comprises an amino acid sequence as shown in SEQ ID NO: 61.

In some embodiments, the antigen-binding protein binds to a KRAS G12 mutant epitope or a complex of the epitope with an MHC molecule. In some embodiments, the KRAS mutant epitope comprises at least one of G12V and G12D.

In some embodiments, the MHC molecule is HLA-A*11 type, for example, HLA-A*11:01.

In some embodiments, the antigen-binding protein exhibits one or more of the following properties:
1) capable of binding to the target antigenic peptide with an EC50 of less than or equal to about 1.0*10⁻⁷ M;
2) high membrane stability;
3) specific killing activity against antigen-positive tumor cells; and
4) absence of alloreactivity to different HLA subtypes.

In some embodiments, the Vα is comprised on a first polypeptide and the Vβ is comprised on a different second polypeptide.

In some embodiments, the Vα and Vβ are comprised on a single polypeptide.

In some embodiments, the antigen-binding protein is soluble or membrane-bound.

In some embodiments, the antigen-binding protein is selected from a TCR, a chimeric antigen receptor (CAR), an Fc polypeptide, or an antigen-binding fragment thereof.

In some embodiments, the antigen-binding protein is a TCR or an antigen-binding fragment thereof, and further the antigen-binding protein comprises a TCR constant region or a fragment thereof.

In some embodiments, the antigen-binding protein further comprises a transmembrane region and/or a cytoplasmic region.

In some embodiments, the antigen-binding protein further comprises an intracellular signaling domain.

In some embodiments, the TCR constant region is a murine constant region or a human constant region.

In some embodiments, the TCR constant region comprises a TCR α chain constant region and/or a TCR β chain constant region.

In some embodiments, the TCR α chain constant region and/or the TCR β chain constant region comprises at least one cysteine mutation relative to a wild-type sequence, so as to form a disulfide bond between the TCR α chain and the TCR β chain.

In some embodiments, the TCR α chain constant region comprises an amino acid sequence having at least 80%, at least 85%, at least 90%, at least 95%, or 100% sequence identity to any one of SEQ ID NOs: 62-66 and 105, and/or the TCR β chain constant region comprises an amino acid sequence having at least 80%, at least 85%, at least 90%, at least 95%, or 100% sequence identity to any one of SEQ ID NOs: 67-76.

In some embodiments, the TCR α chain constant region comprises an amino acid sequence as shown in SEQ ID NO: 66, and the TCR β chain constant region comprises an amino acid sequence as shown in SEQ ID NO: 72.

In some embodiments, the fragment of the TCR constant region is the extracellular segment of the TCR constant region.

In some embodiments, the antigen-binding protein comprises a TCR α chain comprising an amino acid sequence having at least 80%, at least 85%, at least 90%, at least 95%, or 100% sequence identity to any one of SEQ ID NOs: 77-81; and/or a TCR β chain comprising an amino acid sequence having at least 80%, at least 85%, at least 90%, at least 95%, or 100% sequence identity to any one of SEQ ID NOs: 82-86.

In some embodiments, the antigen-binding protein comprises a TCR α chain with an amino acid sequence having at least 80%, at least 85%, at least 90%, at least 95%, or 100% sequence identity to SEQ ID NO: 77; and/or a TCR β chain with an amino acid sequence having at least 80%, at least 85%, at least 90%, at least 95%, or 100% sequence identity to SEQ ID NO: 82. Preferably, the antigen-binding protein comprises a TCR α chain with an amino acid sequence as shown in SEQ ID NO: 77, and/or a TCR β chain with an amino acid sequence as shown in SEQ ID NO: 82.

In some embodiments, the antigen-binding protein comprises a TCR α chain with an amino acid sequence having at least 80%, at least 85%, at least 90%, at least 95%, or 100% sequence identity to SEQ ID NO: 78; and/or a TCR β chain with an amino acid sequence having at least 80%, at least 85%, at least 90%, at least 95%, or 100% sequence identity to SEQ ID NO: 83. Preferably, the antigen-binding protein comprises a TCR α chain with an amino acid sequence as shown in SEQ ID NO: 78, and/or a TCR β chain with an amino acid sequence as shown in SEQ ID NO: 83.

In some embodiments, the antigen-binding protein comprises a TCR α chain with an amino acid sequence having at least 80%, at least 85%, at least 90%, at least 95%, or 100% sequence identity to SEQ ID NO: 79; and/or a TCR β chain with an amino acid sequence having at least 80%, at least 85%, at least 90%, at least 95%, or 100% sequence identity to SEQ ID NO: 84. Preferably, the antigen-binding protein comprises a TCR α chain with an amino acid sequence as shown in SEQ ID NO: 79, and/or a TCR β chain with an amino acid sequence as shown in SEQ ID NO: 84.

In some embodiments, the antigen-binding protein comprises a TCR α chain with an amino acid sequence having at least 80%, at least 85%, at least 90%, at least 95%, or 100% sequence identity to SEQ ID NO: 80; and/or a TCR β chain with an amino acid sequence having at least 80%, at least 85%, at least 90%, at least 95%, or 100% sequence identity to SEQ ID NO: 85. Preferably, the antigen-binding protein comprises the TCR α chain with an amino acid sequence as shown in SEQ ID NO: 80, and/or the TCR β chain with an amino acid sequence as shown in SEQ ID NO: 85.

In some embodiments, the antigen-binding protein comprises a TCR α chain with an amino acid sequence having at least 80%, at least 85%, at least 90%, at least 95%, or 100% sequence identity to SEQ ID NO: 81; and/or a TCR β chain with an amino acid sequence having at least 80%, at least 85%, at least 90%, at least 95%, or 100% sequence identity to SEQ ID NO: 86. Preferably, the antigen-binding protein comprises the TCR α chain with an amino acid sequence as shown in SEQ ID NO: 81 and/or the TCR β chain with an amino acid sequence as shown in SEQ ID NO: 86.

In some embodiments, the antigen-binding protein further comprises one or more antigen binding regions that bind to other antigens or epitopes.

In some embodiments, the antigen-binding protein is isolated or purified.

In another aspect, the present disclosure provides a multispecific antibody, wherein the multispecific antibody comprises the antigen-binding protein of the present disclosure.

In some embodiments, the multispecific antibody comprises the antigen-binding protein of the present disclosure (e.g., the TCR of the present disclosure) and an antibody (e.g., an anti-CD3 antibody) fused to the C-terminus or N-terminus of the antigen-binding protein of the present disclosure.

In another aspect, the present disclosure further provides a nucleic acid encoding the antigen-binding protein of the present disclosure or the multispecific antibody of the present disclosure.

In some embodiments, the nucleic acid comprises a nucleotide sequence having at least 80%, at least 85%, at least 90%, at least 95%, or 100% sequence identity to any one of SEQ ID NOs: 87-91 and/or comprises a nucleotide sequence having at least 80%, at least 85%, at least 90%, at least 95%, or 100% sequence identity to any one of SEQ ID NOs: 92-96.

In another aspect, the present disclosure provides a vector comprising the nucleic acid of the present disclosure.

In some embodiments, the vector is selected from the group consisting of lentiviral vectors, retroviral vectors, plasmids, DNA vectors, mRNA vectors, transposon-based vectors, and artificial chromosomes.

In another aspect, the present disclosure provides a cell comprising the antigen binding protein of the present disclosure, the multispecific antibody of the present disclosure, the nucleic acid of the present disclosure, or the vector of the present disclosure.

In some embodiments, the cell is selected from the group consisting of lymphocytes (e.g., T cells, NK cells), monocytes (e.g., PBMCs), and stem cells.

In some embodiments, the stem cells are a lymphoid progenitor cells or induced pluripotent stem cells (iPSCs).

In some embodiments, the cell is a T cell.

In some embodiments, the T cell does not express an endogenous TCR.

In another aspect, the present disclosure provides a method for preparing the cell of the present disclosure, comprising a step of transducing or transfecting a cell with the vector of the present disclosure.

In some embodiments, the method further comprises a step of amplifying and/or activating the cell prior to or after said transduction or transfection.

In another aspect, the present disclosure provides a conjugate comprising the antigen binding protein of the present disclosure or the multispecific antibody of the present disclosure and an active agent conjugated or coupled to the antigen-binding protein or the multispecific antibody.

In some embodiments, the active agent is selected from the group consisting of a detectable marker, an immunostimulatory molecule and a therapeutic agent.

In some embodiments, the detectable maker is selected from the group consisting of biotins, streptavidin, enzymes or catalytically active fragments thereof, radionuclides, nanoparticles, paramagnetic metal ions, nucleic acid probes, contrast agents, and fluorescent, phosphorescent or chemiluminescent molecules.

In some embodiments, the immunostimulatory molecule is selected from the group consisting of cytokines, chemokines, platelet factors and complement initiators.

In some embodiments, the therapeutic agent is selected from the group consisting of immunomodulators, radioactive compounds (e.g. nuclide), enzymes, chemotherapeutic agents and toxins.

In another aspect, the present disclosure provides a composition comprising the antigen-binding protein the present disclosure, the multispecific antibody the present disclosure, the nucleic acid the present disclosure, the vector the present disclosure, or the cell of the present disclosure.

In some embodiments, the composition further comprises a pharmaceutically acceptable carrier or excipient.

In some embodiments, the composition further comprises a second therapeutic agent.

In some embodiments, the second therapeutic agent is selected from the group consisting of antibodies, chemotherapeutic agents, and small molecule drugs.

In another aspect, the present disclosure provides a method for treating or preventing a KRAS mutation-positive disease and/or disorder in a subject, comprising administering to the subject an effective amount of at least one of the antigen-binding protein of the present disclosure, the multispecific antibody of the present disclosure, and the cell of the present disclosure.

In some embodiments, the cell is autologous or allogeneic to the subject.

In some embodiments, the method comprises steps of: (i) isolating a sample containing cells from the subject; (ii) transducing or transfecting the cells with the vector of the present disclosure; and (iii) administering the cells obtained in step (ii) to the subject.

In some embodiments, the method further comprises knocking out the endogenous TCR in the cells after step (i) and prior to step (ii).

In some embodiments, the KRAS mutation is KRAS G12 mutation, such as comprising at least one of G12V and G12D.

In some embodiments, the KRAS mutation-positive disease and/or disorder is selected from the group consisting of KRAS mutation-driven tumors, precancerous lesions , and cancers. In some embodiments, the tumor is selected from solid tumors and hematologic tumors. More preferably, the tumor comprises at least one of pancreatic cancer, colon cancer, rectal cancer, colorectal cancer, endometrial cancer, lung cancer, melanoma, prostate cancer, cholangiocarcinoma, cervical cancer, bladder cancer, liver cancer, and breast cancer.

In some embodiments, the method further comprises administering a second therapeutic agent.

In some embodiments, the second therapeutic agent is selected from the group consisting of antibodies, chemotherapeutic agents, and small molecule drugs.

In some embodiments, the subject has the HLA-A*11 type, for example, HLA-A*11:01.

In another aspect, the present disclosure provides a method for detecting a KRAS mutation-positive disease and/or disorder in a subject, comprising: (i) contacting a sample obtained from the subject with the antigen-binding protein of the present disclosure, the multispecific antibody of the present disclosure, or the conjugate of the present disclosure; and (ii) detecting the presence of the KRAS mutant antigen in the sample, wherein the presence of the KRAS mutant antigen indicates the KRAS mutation-positive disease and/or disorder.

In another aspect, the present disclosure provides a method for detecting a KRAS mutation-positive disease and/or disorder in a subject, comprising administering to the subject an effective amount of a detectable label (e.g., radionuclide) conjugated antigen-binding protein of the present disclosure or multispecific antibody of the present disclosure.

In some embodiments, the KRAS mutation is KRAS G12, such as comprising at least one of G12V and G12D.

In some embodiments, the KRAS mutation-positive disease and/or disorder is selected from the group consisting of KRAS mutation-driven tumors, precancerous lesions, and cancers. In some embodiments, the cancer is selected from solid tumors and hematologic tumors. More preferably, the tumor comprises at least one of pancreatic cancer, colon cancer, rectal cancer, colorectal cancer, endometrial cancer, lung cancer, melanoma, prostate cancer, cholangiocarcinoma, cervical cancer, bladder cancer, liver cancer, and breast cancer.

In another aspect, the present disclosure provides a kit comprising the antigen-binding protein of the present disclosure, multispecific antibody of the present disclosure, or conjugate of the present disclosure.

In a specific embodiment, the kit is for detecting (e.g., diagnosing) a KRAS mutation-positive disease and/or disorder in a subject, and the kit comprises the antigen-binding protein of the present disclosure, multispecific antibody of the present disclosure, or conjugate of the present disclosure.

In some embodiments of the kit of the present disclosure, the KRAS positive mutation is KRAS G12, such as comprising at least one of G12V and G12C.

In some embodiments, the KRAS mutation-positive disease and/or disorder is selected from the group consisting of KRAS mutation-driven tumors, precancerous lesions, and cancers. In some embodiments, the cancer is selected from solid tumors and hematologic tumors. More preferably, the tumor comprises at least one of pancreatic cancer, colon cancer, rectal cancer, colorectal cancer, endometrial cancer, lung cancer, melanoma, prostate cancer, cholangiocarcinoma, cervical cancer, bladder cancer, liver cancer, and breast cancer.

In another aspect, the present disclosure provides use of the antigen-binding protein, multispecific antibody, nucleic acid, vector, cell, conjugate, or composition of the present disclosure in the preparation of a medicament for treating or preventing KRAS mutation-positive diseases and/or disorders in a subject.

In another aspect, the present disclosure provides the antigen-binding protein, multispecific antibody, nucleic acid, vector, cell, conjugate, or composition of the present disclosure for use in treating or preventing KRAS mutation-positive diseases and/or disorders in a subject.

In another aspect, the present disclosure provides use of the antigen-binding protein, multispecific antibody, or conjugate of the present disclosure in the preparation of a product for detecting (e.g., diagnosing) the presence of KRAS-positive epitopes in a sample to be tested, for example, for use in a kit for detecting (e.g., diagnosing) KRAS mutation-positive diseases and/or disorders in a subject.

In another aspect, the present disclosure provides the antigen-binding protein, multispecific antibody, or conjugate of the present disclosure for detecting (e.g., diagnosing) the presence of KRAS-positive epitopes in a sample to be tested, for example, for detecting (e.g., diagnosing) KRAS mutation-positive diseases and/or disorders in a subject.

In some embodiments of the use of the present disclosure, the KRAS positive mutation is KRAS G12, such as comprising at least one of G12V and G12C.

In some embodiments of the use of the present disclosure, the KRAS mutation-positive disease and/or disorder is selected from the group consisting of KRAS mutation-driven tumors, precancerous lesions, and cancers. In some embodiments, the cancer is selected from solid tumors and hematologic tumors. More preferably, the tumor comprises at least one of pancreatic cancer, colon cancer, rectal cancer, colorectal cancer, endometrial cancer, lung cancer, melanoma, prostate cancer, cholangiocarcinoma, cervical cancer, bladder cancer, liver cancer, and breast cancer.

In some embodiments of the present disclosure, the subject has HLA-A*11 type, for example, HLA-A*11:01.

The antigen-binding protein of the present disclosure is capable of binding the target antigen peptide with high affinity, exhibits good expression stability and high membrane stability, and capable of mediating specific killing of antigen-positive target cells by effector cells, and does not exhibit alloreactivity to different HLA subtypes.

### Brief Description of the Drawings

Figure 1 shows the KRAS G12-related T cell immune epitope described in the present disclosure.
Figures 2A to 2E show the process of obtaining antigen-specific TCR clones described in the present disclosure.
Figure 3 shows that the KDA11-N02 TCR protein molecule provided by the present disclosure exhibits high affinity.
Figures 4A to 4E show that the TCR protein molecules provided by the present disclosure exhibit high membrane stability.
Figure 5 shows specific cytotoxicity against antigen-positive tumor cells of the KRAS G12V-specific TCR-T cells provided by the present disclosure.
Figure 6 shows the cytokine release upon specific recognition of tumor antigens by the TCR-T cells provided by the present invention.
Figures 7A to 7E show the specific killing activity of the TCR-T cells provided by the present disclosure against different antigen-positive tumor cells.
Figure 8 shows the specific response of the TCR-T cells provided by the present disclosure to antigen-positive target cells.
Figures 9A and 9B show that the KRAS G12D-specific TCR-T cells provided by the present disclosure can efficiently mediate antigen-specific T cell proliferation.
Figures 10A to 10E show that the KRAS G12V-specific TCRs provided by the present disclosure do not exhibit non-specific activation against T2KO-TAP1 cell lines expressing different HLA subtypes.
Figure 11 shows that T2 cells themselves express KRAS G12D.
Figure 12 shows that KVA11-N02 does not induce non-specific activation against cells derived from different tissues.
Figure 13 shows the strategy for researching non-specific responses of the KVA11-N02 to the target antigen peptide thereof.
Figure 14 shows the non-specific responses of KVA11-N02 to peptides in the human body.
Figure 15 shows the strategy for researching non-specific responses of the KVA11-N04 to the target antigen peptide thereof.
Figure 16 shows the non-specific responses of KVA11-N04 to peptides in the human body.
Figure 17 shows the in vivo anti-tumor activity of KVA11-N02 TCR-T and KVA11-N04 TCR-T cells.

### Detailed Description of the Invention

Unless otherwise defined, all technical and scientific terms used herein have the same meaning as commonly understood by those skilled in the art. For example, terms used herein are as defined in Janeway CA Jr, Travers P, Walport M et al., Immunobiology, 5th edition, New York: GarlandScience (2001), and Leuenberger, H.G.W, Nagel, B. and Kölbl, H. eds (1995), A multilingual glossary of biotechnological terms: (IUPAC Recommendations), Helvetica Chimica Acta, CH-4010 Basel, Switzerland.

It should be noted that, as used herein and in the appended claims, the singular forms "a", "an" and "the/the" include plural forms, unless the context clearly defined otherwise. Thus, the terms "a", "an", "one or more" and "at least one" can be used interchangeably. Similarly, the terms "comprise", "include" and "have" can be used interchangeably.

When the term "comprise" is used herein and in the appended claims, it does not exclude other elements. For the purposes of the present disclosure, the term "consist of" is considered to be a preferred embodiment of the term "comprise". If a group is defined below as including or comprising at least a certain number of embodiments, it is also to be understood as disclosing a group that preferably consists of only those embodiments.

As used here, X1, X2, X3, X4, X5, X6, X7, X8, X9, X10, X11, X12, X13, X14, and X15 are collectively refer to X in the sequence listing.

In one aspect, the present disclosure provides an antigen-binding protein comprising an antigen-binding domain or a fragment thereof of a T cell receptor (TCR), wherein the antigen-binding domain or fragment thereof comprises a TCR α-chain variable region (Vα) and a TCR β-chain variable region (Vβ), wherein:
The Vα comprises a CDR3 having an amino acid sequence of X1X2X3X4X5X6X7X8X9X10X11X12X13LX15 (SEQ ID NO:1), wherein X1 is A or V, X2 is E or V, X3 is R, P, N, or null, X4 is D, G, S, L, or null, X5 is I, G, D, or null, X6 is E, T, or null, X7 is G or null, X8 is A, G, T, or null, X9 is G, S, A, or null, X10 is N, Y, or G, X11 is N, G, A, or null, X12 is R or null, X13 is K or R, and X15 is I, T, or M; or
the Vα comprises a CDR3 having an amino acid sequence of X1X2X3X4X5X6GX8X9X10X11X12KLX15 (SEQ ID NO:2), wherein X1 is A or V, X2 is E or V, X3 is R, P, or N, X4 is D, G, or S, X5 is I, G, or D, X6 is E, T, or null, X8 is A, G, or T, X9 is G or S, X10 is N or Y, X11 is N, G, or null, X12 is R or null, and X15 is I or T; or
the Vα comprises a CDR3 having an amino acid sequence of X1VX3X4X6X7X8X9X10X11X13LX15 (SEQ ID NO:3), wherein X1 is A or V, X3 is N or null, X4 is L or null, X6 is T or null, X7 is G or null, X8 is A or null, X9 is A or null, X10 is N or G, X11 is N or A, X13 is K or R, and X15 is T or M; or
the Vα comprises a CDR3 having an amino acid sequence of SEQ ID NOs: 22, 23, 24, 25, or 26, or a functional variant thereof formed by insertion, deletion, or substitution of one or more amino acids;
   and/or
the Vβ comprises a CDR3 having an amino acid sequence of ASX3X4X5X6X7X8X9X10X11X12X13X14 (SEQ ID NO:10), wherein: X3 is S or T, X4 is E, H, L, F, or S, X5 is G, W, V, S, or I, X6 is F, G, D, or V, X7 is Y, A, or S, X8 is T, Q, P, Y, or S, X9 is E, D, N, or G, X10 is Y, S, R, or null, X11 is G or null, X12 is T, E, P, or null, X13 is A, Q, or L, and X14 is F, Y, or H; or
the Vβ comprises a CDR3 having an amino acid sequence of ASSX4X5X6X7X8X9X10X12X13X14 (SEQ ID NO:11), wherein: X4 is E, H, or L, X5 is G, W, or V, X6 is F or G, X7 is Y, A, or S, X8 is T, Q, or P, X9 is E or D, X10 is Y or null, X12 is T, E, or null, X13 is A or Q, and X14 is F or Y; or
the Vβ comprises a CDR3 having an amino acid sequence of ASX3X4X5X6SX8X9X10X11X12X13X14 (SEQ ID NO:12), wherein X3 is S or T, X4 is F or S, X5 is S or I, X6 is D or V, X8 is Y or S, X9 is N or G, X10 is S or R, X11 is G or null, X12 is E or P, X13 is Q or L, and X14 is F or H; or
the Vβ comprises a CDR3 having an amino acid sequence of SEQ ID NOs: 37, 38, 39, 40, or 41, or a functional variant thereof formed by insertion, deletion, or substitution of one or more amino acids;
and wherein the antigen-binding domain or fragment thereof is antigen-specific for a mutated KRAS G12 epitope.

As used herein, the term "antigen binding protein" refers to a protein or polypeptide comprising at least one TCR alpha chain CDR3 (CDR3α) and/or at least one TCR beta chain CDR3 (CDR3β) as of the present disclosure and capable of binding to an antigenic target KRAS G12 (e.g. G12V, G12D, G12C). Further contemplated herein is an antigen binding protein comprising at least one of CDR1α, CDR2α, CDR1β, CDR2β, alpha chain variable region, beta chain variable region, alpha chain and/or beta chain, or the combination thereof, optionally in combination with other protein domains or portions listed herein.

As used herein, the term "functional variant" refers to a polypeptide that has significant sequence identity to the parent polypeptide and retains the biological activity of the parent polypeptide. A functional variant encompasses, for example, a variant of the polypeptide or protein described herein that retains the ability to specifically bind to the KRAS G12 antigen to a similar extent to the parent polypeptide, to the same extent as the parent polypeptide, or to a higher extent than the parent polypeptide. The amino acid sequence of the functional variant may, for example, have at least about 50%, 75%, 80%, 90%, 95%, 96%, 97%, 98%, 98.2%, 98.4%, 98.6%, 98.8%, 99%, 99.1%, 99.2%, 99.3%, 99.4%, 99.5%, 99.6%, 99.7%, 99.8%, 99.9% or higher sequence identity to that of the parent polypeptide.

As used herein, the term "functional variant thereof formed by insertion, deletion or substitution of one or more amino acids" refers to a functional variant thereof formed by the insertion, deletion or substitution of 1, 2, 3, 4, 5, 6, 7, 8, 9, 10, 11, 12, 13, 14 or 15 amino acids.

The term "epitope" usually refers to a site on an antigen, usually a (poly)peptide recognized by a binding domain. The term "binding domain" in its broadest sense refers to an "antigen binding site", i.e. a domain characterizing a molecule that binds to/interacts with a specific epitope on an antigenic target. The antigenic target may comprise a single epitope, but typically comprises at least two epitopes, and depending on the size, conformation and type of the antigen, the antigenic target may include any number of epitopes. The term "epitope" typically includes both linear epitopes and conformational epitopes. A linear epitope is a contiguous epitope contained in a primary sequence of amino acids and typically comprises at least 2 or more amino acids. A conformational epitope is formed by non-contiguous amino acids juxtaposed by folding of a target antigen, in particular, a target (poly)peptide.

In the context of the present disclosure, the term "binding domain" refers in particular to the variable regions of TCR alpha chain and/or beta chain, in particular the CDR3α and CDR3β of the TCR.

As used herein, the term "T cell receptor" or "TCR" includes natural TCRs as well as TCR variants, fragments and constructs. The term thus includes heterodimers as well as multimers and single chain constructs comprising TCR alpha chain and TCR beta chain; optionally comprising other domains and/or portions, provided that the antigen binding protein retains its ability to recognize an antigenic target (preferably its complex with HLA-A*02).

In a natural form, the TCR exists as a complex of several proteins on the surface of T cells. The T cell receptor consists of two (separate) protein chains that are produced by separate T cell receptor alpha and beta (TCRα and TCRβ) genes and are referred to as alpha chain and beta chain. Each chain of the TCR has an N-terminal immunoglobulin-like (Ig)-variable (V) region/domain, an Ig-constant (C) region/domain, a transmembrane/cytomembrane spanning region that anchors the chain into the plasma membrane, and a C-terminal short cytoplasmic tail.

Antigen specificity is conferred by the variable regions ofα chain and β chain of TCR. Both variable regions of TCR α and β chains contain three highly variable or complementary determining regions (CDR1α/β, CDR2α/β, and CDR3α/β) surrounded by framework (FR) regions. CDR3 is the major determinant for antigen recognition and specificity (i.e., the ability to recognize and interact with the specific antigen), whereas CDR1 and CDR2 interact primarily with MHC molecules that present antigenic peptides.

The natural TCR recognizes an antigenic peptide that binds to a major histocompatibility complex (MHC) molecule at the surface of the antigen-presenting cell ("presenting/exhibiting on the MHC molecule"). The antigenic peptide presented on the MHC molecule is also referred to herein as a "complex of epitope with MHC molecule", "epitope-MHC complex" or "target antigenic peptide-MHC complex". There are two different classes of MHC molecules: MHC I and MHC II, which present peptides from different cellular compartments. MHC class I molecules are expressed on the surface of all nucleated cells in the human and present peptides or protein fragments from intracellular compartments to cytotoxic T cells. In human, MHC is also known as human leukocyte antigen (HLA). There are three main types of MHC class I molecules: HLA-A, HLA-B, and HLA-C. Once the TCR binds to its specific epitope-MHC complex, the T cells are activated and exert a biological effector function.

As will be discussed in detail below, the TCRs provided herein are capable of advantageously (specifically) recognizing a KRAS G12 mutant antigen, in particular KRAS G12V or G12D epitopes or a complex of the epitope with a MHC molecule, such as a complex of KRAS G12V or G12D epitope with HLA-A*11.

In some embodiments of the antigen-binding protein of the present disclosure, the MHC molecule is HLA-A*11 type, for example, HLA-A*11:01.

As used herein, "G12" is defined with reference to the wild-type human KRAS amino acid sequence, the epitope sequence of the wild-type human KRAS amino acid sequence typically includes a 9-mer (VVGA***G***GVGK, SEQ ID NO: 99) or 10-mer (VVVGA***G***GVGK, SEQ ID NO: 100), in which the underlined, bold and italicized amino acid represents the G12 position. Therefore, in some embodiments, "KRAS G12V" as used herein includes an amino acid sequence of VVGA***V***GVGK (SEQ ID NO: 101) or VVVGA***V***GVGK (SEQ ID NO: 102). In some embodiments, "KRAS G12D" as used herein includes an amino acid sequence of VVGA***D***GVGK (SEQ ID NO: 103) or VVVGA***D***GVGK (SEQ ID NO: 104).

In some embodiments, the antigen-binding proteins of the present disclosure do not exhibit antigen specificity to a wild-type human KRAS amino acid sequence.

In some embodiments, the present disclosure provides an antigen-binding protein comprising an antigen-binding domain or a fragment thereof of a T cell receptor (TCR), wherein the antigen-binding domain or fragment thereof comprises a TCR α chain variable region (Vα) and a TCR β chain variable region (Vβ), wherein
the Vα comprises a CDR3 having an amino acid sequence of X1X2X3X4X5X6X7X8X9X10X11X12X13LX15 (SEQ ID NO:1), wherein X1 is A or V, X2 is E or V, X3 is R, P, N, or null, X4 is D, G, S, L, or null, X5 is I, G, D, or null, X6 is E, T, or null, X7 is G or null, X8 is A, G, T, or null, X9 is G, S, A, or null, X10 is N, Y, or G, X11 is N, G, A, or null, X12 is R or null, X13 is K or R, and X15 is I, T, or M;
   and/or
the Vβ comprises a CDR3 having an amino acid sequence of ASX3X4X5X6X7X8X9X10X11X12X13X14 (SEQ ID NO:10), wherein X3 is S or T, X4 is E, H, L, F, or S, X5 is G, W, V, S, or I, X6 is F, G, D, or V, X7 is Y, A, or S, X8 is T, Q, P, Y, or S, X9 is E, D, N, or G, X10 is Y, S, R, or null, X11 is G or null, X12 is T, E, P, or null, X13 is A, Q, or L, and X14 is F, Y, or H;
and wherein the antigen-binding domain or fragment thereof is antigen-specific for a mutated KRAS G12 epitope.

In some embodiments, the present disclosure provides an antigen-binding protein comprising an antigen-binding domain or a fragment thereof of a T cell receptor (TCR), wherein the antigen-binding domain or fragment thereof comprises a TCR α chain variable region (Vα) and a TCR β chain variable region (Vβ), wherein
the Vα comprises a CDR3 having an amino acid sequence of X1X2X3X4X5X6GX8X9X10X11X12KLX15 (SEQ ID NO:2), wherein:
X1 is A or V, X2 is E or V, X3 is R, P, or N, X4 is D, G, or S, X5 is I, G, or D, X6 is E, T, or null, X8 is A, G, or T, X9 is G or S, X10 is N or Y, X11 is N, G, or null, X12 is R or null, and X15 is I or T;
   and/or
the Vβ comprises a CDR3 having an amino acid sequence of ASSX4X5X6X7X8X9X10X12X13X14 (SEQ ID NO:11), wherein:
   X4 is E, H, or L, X5 is G, W, or V, X6 is F or G, X7 is Y, A, or S, X8 is T, Q, or P, X9 is E or D, X10 is Y or null, X12 is T, E, or null, X13 is A or Q, and X14 is F or Y;
   and wherein the antigen-binding domain or fragment thereof is antigen-specific for the mutated KRAS G12V epitope.

In some embodiments, the present disclosure provides an antigen-binding protein comprising an antigen-binding domain or a fragment thereof of a T cell receptor (TCR), wherein the antigen-binding domain or fragment thereof comprises a TCR α chain variable region (Vα) and a TCR β chain variable region (Vβ), wherein
the Vα comprises a CDR3 having an amino acid sequence of X1VX3X4X6X7X8X9X10X11X13LX15 (SEQ ID NO:3), wherein:
X1 is A or V, X3 is N or null, X4 is L or null, X6 is T or null, X7 is G or null, X8 is A or null, X9 is A or null, X10 is N or G, X11 is N or A, X13 is K or R, and X15 is T or M;
   and/or
the Vβ comprises a CDR3 having an amino acid sequence of ASX3X4X5X6SX8X9X10X11X12X13X14 (SEQ ID NO:12), wherein:
   X3 is S or T, X4 is F or S, X5 is S or I, X6 is D or V, X8 is Y or S, X9 is N or G, X10 is S or R, X11 is G or null, X12 is E or P, X13 is Q or L, and X14 is F or H;
   and wherein the antigen-binding domain or fragment thereof is antigen-specific for the mutated KRAS G12D epitope.

In some embodiments, the Vα of the antigen-binding protein provided by the present disclosure further comprises a CDR1 having an amino acid sequence of X1X2X3X4X5X6 (SEQ ID NO:4), wherein X1 is V, N, or D; X2 is S or R; X3 is G or A; X4 is N or S; X5 is P, Q, or D; and X6 is Y or S.

In some embodiments, the Vα of the antigen-binding protein provided by the present disclosure further comprises a CDR1 having an amino acid sequence of X1SX3X4X5X6 (SEQ ID NO:5), wherein X1 is V or N; X3 is G or A; X4 is N or S; X5 is P, Q, or D; and X6 is Y or S. In some embodiments, the antigen-binding domain or fragment thereof is antigen-specific for the mutated KRAS G12V epitope.

In some embodiments, the Vα of the antigen-binding protein provided by the present disclosure further comprises a CDR1 having an amino acid sequence of X1X2X3SQS (SEQ ID NO:6), wherein X1 is N or D; X2 is S or R; and X3 is G or A. In some embodiments, the antigen-binding domain or fragment thereof is antigen-specific for the mutated KRAS G12D epitope.

In some embodiments, the Vα of the antigen-binding protein provided by the present disclosure further comprises a CDR1 having an amino acid sequence as shown in SEQ ID NOs: 27, 28, 29, 30, or 31, or a functional variant thereof formed by insertion, deletion, or substitution of one or more amino acids.

In some embodiments, the Vα of the antigen-binding protein provided by the present disclosure further comprises a CDR2 having an amino acid sequence of X1X2X3X4X5NX7X8X9 (SEQ ID NO:7), wherein X1 is Y, V, or null; X2 is I or Y; X3 is T, S, R, or Y; X4 is G or S; X5 is D, G, or null; X7 is L, M, G, or null; X8 is V, D, or null; and X9 is K or null.

In some embodiments, the Vα of the antigen-binding protein provided by the present disclosure further comprises a CDR2 having an amino acid sequence of X1X2X3X4X5NX7X8X9 (SEQ ID NO:8), wherein X1 is Y, V, or null; X2 is I or Y; X3 is T, S, or R; X4 is G or S; X5 is D, G, or null; X7 is L, M, or null; X8 is V, D, or null; and X9 is K or null. In some embodiments, the antigen-binding domain or fragment thereof is antigen-specific for the mutated KRAS G12V epitope.

In some embodiments, the Vα of the antigen-binding protein provided by the present disclosure further comprises a CDR2 having an amino acid sequence of X1X2X3SX5NX7X8 (SEQ ID NO:9), wherein X1 is V or null; X2 is I or Y; X3 is S or Y; X5 is G or null; X7 is G or null; and X8 is D or null. In some embodiments, the antigen-binding domain or fragment thereof is antigen-specific for the mutated KRAS G12D epitope.

In some embodiments, the Vα of the antigen-binding protein provided by the present disclosure further comprises a CDR2 having an amino acid sequence as shown in SEQ ID NOs: 32, 33, 34, 35, or 36, or a functional variant thereof formed by insertion, deletion, or substitution of one or more amino acids.

In some embodiments, the Vα of the antigen-binding protein provided by the present disclosure further comprises a CDR1 having an amino acid sequence of X1X2X3X4X5X6 (SEQ ID NO:4), wherein X1 is V, N, or D; X2 is S or R; X3 is G or A; X4 is N or S; X5 is P, Q, or D; and X6 is Y or S; and a CDR2 having an amino acid sequence of X1X2X3X4X5NX7X8X9 (SEQ ID NO:7), wherein X1 is Y, V, or null; X2 is I or Y; X3 is T, S, R, or Y; X4 is G or S; X5 is D, G, or null; X7 is L, M, G, or null; X8 is V, D, or null; and X9 is K or null.

In some embodiments, the Vα of the antigen-binding protein provided by the present disclosure further comprises a CDR1 having an amino acid sequence of X1SX3X4X5X6 (SEQ ID NO:5), wherein X1 is V or N; X3 is G or A; X4 is N or S; X5 is P, Q, or D; and X6 is Y or S; and a CDR2 having an amino acid sequence of X1X2X3X4X5NX7X8X9 (SEQ ID NO:8), wherein X1 is Y, V, or null; X2 is I or Y; X3 is T, S, or R; X4 is G or S; X5 is D, G, or null; X7 is L, M, or null; X8 is V, D, or null; and X9 is K or null. In some embodiments, the antigen-binding domain or fragment thereof is antigen-specific for the mutated KRAS G12V epitope.

In some embodiments, the Vα of the antigen-binding protein provided by the present disclosure further comprises a CDR1 having an amino acid sequence of X1X2X3SQS (SEQ ID NO:6), wherein X1 is N or D; X2 is S or R; and X3 is G or A; and a CDR2 having an amino acid sequence of X1X2X3SX5NX7X8 (SEQ ID NO:9), wherein X1 is V or null; X2 is I or Y; X3 is S or Y; X5 is G or null; X7 is G or null; and X8 is D or null. In some embodiments, the antigen-binding domain or fragment thereof is antigen-specific for the mutated KRAS G12D epitope.

In some embodiments, the Vα of the antigen-binding protein provided by the present disclosure further comprises a CDR1 having an amino acid sequence as shown in SEQ ID NOs: 27, 28, 29, 30, or 31, or a functional variant thereof formed by insertion, deletion, or substitution of one or more amino acids; and a CDR2 having an amino acid sequence as shown in SEQ ID NOs: 32, 33, 34, 35, or 36, or a functional variant thereof formed by insertion, deletion, or substitution of one or more amino acids.

In some embodiments, the Vα of the antigen-binding protein provided by the present disclosure further comprises a CDR1 having an amino acid sequence as shown in SEQ ID NOs: 27, 28, or 29, or a functional variant thereof formed by insertion, deletion, or substitution of one or more amino acids; and a CDR2 having an amino acid sequence as shown in SEQ ID NOs: 32, 33, or 34, or a functional variant thereof formed by insertion, deletion, or substitution of one or more amino acids. In some embodiments, the antigen-binding domain or fragment thereof is antigen-specific for the mutated KRAS G12V epitope.

In some embodiments, the Vα of the antigen-binding protein of the present disclosure further comprises a CDR1 having an amino acid sequence as shown in SEQ ID NOs: 30 or 31, or a functional variant thereof formed by insertion, deletion, or substitution of one or more amino acids; and a CDR2 having an amino acid sequence as shown in SEQ ID NOs: 35 or 36, or a functional variant thereof formed by insertion, deletion, or substitution of one or more amino acids. In some embodiments, the antigen-binding domain or fragment thereof is antigen-specific for the mutated KRAS G12D epitope.

In some embodiments, the Vβ of the antigen-binding protein of the present disclosure further comprises a CDR1 having an amino acid sequence of X1X2HX4X5 (SEQ ID NO: 13), wherein X1 is S, L, or M; X2 is G or N; X4 is N, D, A, or E; and X5 is S, T, or Y.

In some embodiments, the Vβ of the antigen-binding protein of the present disclosure further comprises a CDR1 having an amino acid sequence of X1GHX4X5 (SEQ ID NO:14), wherein X1 is S or L; X4 is N, D, or A; and X5 is S or T. In some embodiments, the antigen-binding domain or fragment thereof is antigen-specific for the mutated KRAS G12V epitope.

In some embodiments, the Vβ of the antigen-binding protein of the present disclosure further comprises a CDR1 having an amino acid sequence of X1X2HX4X5 (SEQ ID NO:15), wherein X1 is S or M; X2 is G or N; X4 is A or E; and X5 is T or Y. In some embodiments, the antigen-binding domain or fragment thereof is antigen-specific for the mutated KRAS G12D epitope.

In some embodiments, the Vβ of the antigen-binding protein of the present disclosure further comprises a CDR1 having an amino acid sequence as shown in SEQ ID NOs: 42, 43, 44, 45, or 46, or a functional variant thereof formed by insertion, deletion, or substitution of one or more amino acids.

In some embodiments, the Vβ of the antigen-binding protein of the present disclosure further comprises a CDR2 having an amino acid sequence of X1X2NX4X5X6 (SEQ ID NO:16), wherein X1 is F, Y, or S; X2 is N, Q, or M; X4 is N, K, or V; X5 is V, E, or G; and X6 is P, L, or V.

In some embodiments, the Vβ of the antigen-binding protein of the present disclosure further comprises a CDR2 having an amino acid sequence of X1X2NX4X5X6 (SEQ ID NO:17), wherein X1 is F or Y; X2 is N or Q; X4 is N or K; X5 is V, E, or G; and X6 is P, L, or V. In some embodiments, the antigen-binding domain or fragment thereof is antigen-specific for the mutated KRAS G12V epitope.

In some embodiments, the Vβ of the antigen-binding protein of the present disclosure further comprises a CDR2 having an amino acid sequence of X1X2NX4X5V (SEQ ID NO:18), wherein X1 is F or S; X2 is Q or M; X4 is N or V; and X5 is E or G. In some embodiments, the antigen-binding domain or fragment thereof is antigen-specific for the mutated KRAS G12D epitope.

In some embodiments, the Vβ of the antigen-binding protein of the present disclosure further comprises a CDR2 having an amino acid sequence as shown in SEQ ID NOs: 47, 48, 49, 50, or 51, or a functional variant thereof formed by insertion, deletion, or substitution of one or more amino acids.

In some embodiments, the Vβ of the antigen-binding protein of the present disclosure further comprises a CDR1 having an amino acid sequence of X1X2HX4X5 (SEQ ID NO:13), wherein X1 is S, L, or M; X2 is G or N; X4 is N, D, A, or E; and X5 is S, T, or Y; and a CDR2 having an amino acid sequence of X1X2NX4X5X6 (SEQ ID NO:16), wherein X1 is F, Y, or S; X2 is N, Q, or M; X4 is N, K, or V; X5 is V, E, or G; and X6 is P, L, or V.

In some embodiments, the Vβ of the antigen-binding protein of the present disclosure further comprises a CDR1 having an amino acid sequence of X1GHX4X5 (SEQ ID NO:14), wherein X1 is S or L; X4 is N, D, or A; and X5 is S or T; and a CDR2 having an amino acid sequence of X1X2NX4X5X6 (SEQ ID NO:17), wherein X1 is F or Y; X2 is N or Q; X4 is N or K; X5 is V, E, or G; and X6 is P, L, or V. In some embodiments, the antigen-binding domain or fragment thereof is antigen-specific for the mutated KRAS G12V epitope.

In some embodiments, the Vβ of the antigen-binding protein of the present disclosure further comprises a CDR1 having an amino acid sequence of X1X2HX4X5 (SEQ ID NO:15), wherein X1 is S or M; X2 is G or N; X4 is A or E; and X5 is T or Y; and a CDR2 having an amino acid sequence of X1X2NX4X5V (SEQ ID NO:18), wherein X1 is F or S; X2 is Q or M; X4 is N or V; and X5 is E or G. In some embodiments, the antigen-binding domain or fragment thereof is antigen-specific for the mutated KRAS G12D epitope.

In some embodiments, the Vβ of the antigen-binding protein of the present disclosure further comprises a CDR1 having an amino acid sequence as shown in SEQ ID NOs: 42, 43, 44, 45, or 46, or a functional variant thereof formed by insertion, deletion, or substitution of one or more amino acids; and a CDR2 having an amino acid sequence as shown in SEQ ID NOs: 47, 48, 49, 50, or 51, or a functional variant thereof formed by insertion, deletion, or substitution of one or more amino acids.

In some embodiments, the Vβ of the antigen-binding protein of the present disclosure further comprises a CDR1 having an amino acid sequence as shown in SEQ ID NOs: 42, 43, or 44, or a functional variant thereof formed by insertion, deletion, or substitution of one or more amino acids; and a CDR2 having an amino acid sequence as shown in SEQ ID NOs: 47, 48, or 49, or a functional variant thereof formed by insertion, deletion, or substitution of one or more amino acids. In some embodiments, the antigen-binding domain or fragment thereof is antigen-specific for the mutated KRAS G12V epitope.

In some embodiments, the Vβ of the antigen-binding protein of the present disclosure further comprises a CDR1 having an amino acid sequence as shown in SEQ ID NOs: 45 or 46, or a functional variant thereof formed by insertion, deletion, or substitution of one or more amino acids; and a CDR2 having an amino acid sequence as shown in SEQ ID NOs: 50 or 51, or a functional variant thereof formed by insertion, deletion, or substitution of one or more amino acids. In some embodiments, the antigen-binding domain or fragment thereof is antigen-specific for the mutated KRAS G12D epitope.

As described above, it is believed that CDR1 and CDR2 of the Vα and Vβ chains are mainly involved in MHC recognition. There exists a limited "pool" of known CDR1 and CDR2 sequences involved in HLA-A*11-restricted antigen recognition. In principle, the CDR3 domain of the present disclosure can be combined with any one of the Vα chain CDR1 sequences shown in SEQ ID NOs: 4-6 and 27-31, any one of the Vα chain CDR2 sequences shown in SEQ ID NOs: 7-9 and 32-36, any one of the Vβ chain CDR1 sequences shown in SEQ ID NOs: 13-15 and 42-46, and any one of the Vβ chain CDR2 sequences shown in SEQ ID NOs: 16-18 and 47-51, provided that the antigen-binding protein retains its ability to recognize an antigen target (preferably as a complex with HLA-A*11) to a degree similar to, the same as, or even greater than the TCRs evaluated in the appended examples.

As previously described, it is identified that CDR1 and CDR2 of TCRα and TCRβ chains are primarily involved in MHC recognition. There is a limited "pool" of CDR1 and CDR2 sequences known to be involved in HLA-A*11-restricted antigen recognition. It is contemplated that the CDR3 domain of the present disclosure can in principle be combined with any one of the Vα chain CDR1 as shown in SEQ ID NOs: 4-6 and 27-31, any one of the Vα chain CDR2 as shown in SEQ ID NOs: 7-9 and 32-36, any one of the Vβ chain CDR1 as shown in SEQ ID NOs: 13-15 and 42-46, and any one of the Vβ chain CDR2 as shown in SEQ ID NOs: 16-18 and 47-51, provided that the antigen binding protein retains its ability to recognize an antigenic target (preferably its complex with HLA-A*11) to a similar, identical, or even greater extent than the TCR assessed in the appended examples.

In some embodiments, the Vα comprises a CDR3 having an amino acid sequence of X1X2X3X4X5X6X7X8X9X10X11X12X13LX15 (SEQ ID NO:1), wherein:
X1 is A or V, X2 is E or V, X3 is R, P, N or null, X4 is D, G, S, L or null, X5 is I, G, D or null, X6 is E, T or null, X7 is G or null, X8 is A, G, T or null, X9 is G, S, A or null, X10 is N, Y or G, X11 is N, G, A or null, X12 is R or null, X13 is K or R, and X15 is I, T or M; or
a CDR3 having an amino acid sequence of X1X2X3X4X5X6GX8X9X10X11X12KLX15 (SEQ ID NO:2), wherein:
   X1 is A or V, X2 is E or V, X3 is R, P or N, X4 is D, G or S, X5 is I, G or D, X6 is E, T or null, X8 is A, G or T, X9 is G or S, X10 is N or Y, X11 is N, G or null, X12 is R or null, and X15 is I or T; or
   a CDR3 having an amino acid sequence of X1VX3X4X6X7X8X9X10X11X13LX15 (SEQ ID NO:3), wherein:
      X1 is A or V, X3 is N or null, X4 is L or null, X6 is T or null, X7 is G or null, X8 is A or null, X9 is A or null, X10 is N or G, X11 is N or A, X13 is K or R, and X15 is T or M;
      or a CDR3 having an amino acid sequence as shown in SEQ ID NOs: 22, 23, 24, 25 or 26 or a functional variant thereof formed by insertion, deletion or substitution of one or more amino acids; and
      the Vα comprises a CDR1 having an amino acid sequence of X1X2X3X4X5X6 (SEQ ID NO:4), wherein X1 is V, N or D; X2 is S or R; X3 is G or A; X4 is N or S; X5 is P, Q or D; and X6 is Y or S; or
      a CDR1 having an amino acid sequence of X1SX3X4X5X6 (SEQ ID NO:5), wherein X1 is V or N; X3 is G or A; X4 is N or S; X5 is P, Q or D; and X6 is Y or S; or
      a CDR1 having an amino acid sequence of X1X2X3SQS (SEQ ID NO:6), wherein X1 is N or D; X2 is S or R; and X3 is G or A; or
      a CDR1 having an amino acid sequence as shown in SEQ ID NOs: 27, 28, 29, 30 or 31 or a functional variant thereof formed by insertion, deletion or substitution of one or more amino acids; and
      the Vα comprises a CDR2 having an amino acid sequence of X1X2X3X4X5NX7X8X9 (SEQ ID NO:7), wherein X1 is Y, V or null; X2 is I or Y; X3 is T, S, R or Y; X4 is G or S; X5 is D, G or null; X7 is L, M, G or null; X8 is V, D or null; and X9 is K or null; or
      a CDR2 having an amino acid sequence of X1X2X3X4X5NX7X8X9 (SEQ ID NO:8), wherein X1 is Y, V or null; X2 is I or Y; X3 is T, S or R; X4 is G or S; X5 is D, G or null; X7 is L, M or null; X8 is V, D or null; and X9 is K or null; or
      a CDR2 having an amino acid sequence of X1X2X3SX5NX7X8 (SEQ ID NO:9), wherein X1 is V or null; X2 is I or Y; X3 is S or Y; X5 is G or null; X7 is G or null; and X8 is D or null; or
      a CDR2 having an amino acid sequence as shown in SEQ ID NOs: 32, 33, 34, 35 or 36 or a functional variant thereof formed by insertion, deletion or substitution of one or more amino acids;
         and/or
      the Vβ comprises a CDR3 having an amino acid sequence of ASX3X4X5X6X7X8X9X10X11X12X13X14 (SEQ ID NO:10), wherein X3 is S or T; X4 is E, H, L, F or S; X5 is G, W, V, S or I; X6 is F, G, D or V; X7 is Y, A or S; X8 is T, Q, P, Y or S; X9 is E, D, N or G; X10 is Y, S, R or null; X11 is G or null; X12 is T, E, P or null; X13 is A, Q or L; and X14 is F, Y or H; or
      a CDR3 having an amino acid sequence of ASSX4X5X6X7X8X9X10X12X13X14 (SEQ ID NO:11), wherein X4 is E, H or L; X5 is G, W or V; X6 is F or G; X7 is Y, A or S; X8 is T, Q or P; X9 is E or D; X10 is Y or null; X12 is T, E or null; X13 is A or Q; and X14 is F or Y; or
      a CDR3 having an amino acid sequence of ASX3X4X5X6SX8X9X10X11X12X13X14 (SEQ ID NO:12), wherein X3 is S or T; X4 is F or S; X5 is S or I; X6 is D or V; X8 is Y or S; X9 is N or G; X10 is S or R; X11 is G or null; X12 is E or P; X13 is Q or L; and X14 is F or H; or
      a CDR3 having an amino acid sequence as shown in SEQ ID NOs: 37, 38, 39, 40 or 41 or a functional variant thereof formed by insertion, deletion or substitution of one or more amino acids; and
      the Vβ comprises a CDR1 having an amino acid sequence of X1X2HX4X5 (SEQ ID NO:13), wherein X1 is S, L or M; X2 is G or N; X4 is N, D, A or E; and X5 is S, T or Y; or
      a CDR1 having an amino acid sequence of X1GHX4X5 (SEQ ID NO:14), wherein X1 is S or L; X4 is N, D or A; and X5 is S or T; or
      a CDR1 having an amino acid sequence of X1X2HX4X5 (SEQ ID NO:15), wherein X1 is S or M; X2 is G or N; X4 is A or E; and X5 is T or Y; or
      a CDR1 having an amino acid sequence as shown in SEQ ID NOs: 42, 43, 44, 45 or 46 or a functional variant thereof formed by insertion, deletion or substitution of one or more amino acids; and
      the Vβ comprises a CDR2 having an amino acid sequence of X1X2NX4X5X6 (SEQ ID NO:16), wherein X1 is F, Y or S; X2 is N, Q or M; X4 is N, K or V; X5 is V, E or G; and X6 is P, L or V; or
      a CDR2 having an amino acid sequence of X1X2NX4X5X6 (SEQ ID NO:17), wherein X1 is F or Y; X2 is N or Q; X4 is N or K; X5 is V, E or G; and X6 is P, L or V; or
      a CDR2 having an amino acid sequence of X1X2NX4X5V (SEQ ID NO:18), wherein X1 is F or S; X2 is Q or M; X4 is N or V; and X5 is E or G; or
      a CDR2 having an amino acid sequence as shown in SEQ ID NOs: 47, 48, 49, 50 or 51 or a functional variant thereof formed by insertion, deletion or substitution of one or more amino acids.

In some embodiments, the Vα comprises a CDR3 having an amino acid sequence of X1X2X3X4X5X6X7X8X9X10X11X12X13LX15 (SEQ ID NO: 1), wherein: X1 is A or V, X2 is E or V, X3 is R, P, N, or null, X4 is D, G, S, L, or null, X5 is I, G, D, or null, X6 is E, T, or null, X7 is G or null, X8 is A, G, T, or null, X9 is G, S, A, or null, X10 is N, Y, or G, X11 is N, G, A, or null, X12 is R or null, X13 is K or R, and X15 is I, T, or M; and
the Vα comprises a CDR1 having an amino acid sequence of X1X2X3X4X5X6 (SEQ ID NO: 4), wherein X1 is V, N, or D; X2 is S or R; X3 is G or A; X4 is N or S; X5 is P, Q, or D; and X6 is Y or S;
and the Vα comprises a CDR2 having an amino acid sequence of X1X2X3X4X5NX7X8X9 (SEQ ID NO: 7), wherein X1 is Y, V, or null; X2 is I or Y; X3 is T, S, R, or Y; X4 is G or S; X5 is D, G, or null; X7 is L, M, G, or null; and X8 is V, D, or null, and X9 is K or null;
   and/or
the Vβ comprises a CDR3 having an amino acid sequence of ASX3X4X5X6X7X8X9X10X11X12X13X14 (SEQ ID NO: 10), wherein X3 is S or T, X4 is E, H, L, F, or S, X5 is G, W, V, S, or I, X6 is F, G, D, or V, X7 is Y, A, or S, X8 is T, Q, P, Y, or S, X9 is E, D, N, or G, X10 is Y, S, R, or null, X11 is G or null, X12 is T, E, P, or null, X13 is A, Q, or L, and X14 is F, Y, or H; and
the Vβ comprises a CDR1 having an amino acid sequence of X1X2HX4X5 (SEQ ID NO: 13), wherein X1 is S, L, or M; X2 is G or N; X4 is N, D, A, or E; and X5 is S, T, or Y;
and the Vβ comprises a CDR2 having an amino acid sequence of X1X2NX4X5X6 (SEQ ID NO: 16), wherein X1 is F, Y, or S; X2 is N, Q, or M; X4 is N, K, or V; X5 is V, E, or G; and X6 is P, L, or V.

In some embodiments, the Vα comprises a CDR3 having an amino acid sequence of X1X2X3X4X5X6GX8X9X10X11X12KLX15 (SEQ ID NO: 2), wherein X1 is A or V, X2 is E or V, X3 is R, P, or N, X4 is D, G, or S, X5 is I, G, or D, X6 is E, T, or null, X8 is A, G, or T, X9 is G or S, X10 is N or Y, X11 is N, G, or null, X12 is R or null, and X15 is I or T; or the Vα comprises a CDR3 having an amino acid sequence as shown in SEQ ID NOs: 22, 23, or 24, or a functional variant thereof formed by insertion, deletion, or substitution of one or more amino acids; and
the Vα comprises a CDR1 having an amino acid sequence of X1SX3X4X5X6 (SEQ ID NO: 5), wherein X1 is V or N; X3 is G or A; X4 is N or S; X5 is P, Q, or D; and X6 is Y or S; or the Vα comprises a CDR1 having an amino acid sequence as shown in SEQ ID NOs: 27, 28, or 29, or a functional variant thereof formed by insertion, deletion, or substitution of one or more amino acids; and
the Vα comprises a CDR2 having an amino acid sequence of X1X2X3X4X5NX7X8X9 (SEQ ID NO: 8), wherein X1 is Y, V, or null; X2 is I or Y; X3 is T, S, or R; X4 is G or S; X5 is D, G, or null; X7 is L, M, or null; X8 is V, D, or null; and X9 is K or null; or the Vα comprises a CDR2 having an amino acid sequence as shown in SEQ ID NOs: 32, 33, or 34, or a functional variant thereof formed by insertion, deletion, or substitution of one or more amino acids;
   and/or
the Vβ comprises a CDR3 having an amino acid sequence of ASSX4X5X6X7X8X9X10X12X13X14 (SEQ ID NO: 11), wherein: X4 is E, H, or L, X5 is G, W, or V, X6 is F or G, X7 is Y, A, or S, X8 is T, Q, or P, X9 is E or D, X10 is Y or null, X12 is T, E, or null, X13 is A or Q, and X14 is F or Y; or the Vβ comprises a CDR3 having an amino acid sequence as shown in SEQ ID NOs: 37, 38, or 39, or a functional variant thereof formed by insertion, deletion, or substitution of one or more amino acids; and
the Vβ comprises a CDR1 having an amino acid sequence of X1GHX4X5 (SEQ ID NO: 14), wherein X1 is S or L; X4 is N, D, or A; X5 is S or T; or the Vβ comprises a CDR1 having an amino acid sequence as shown in SEQ ID NOs: 42, 43, or 44, or a functional variant thereof formed by insertion, deletion, or substitution of one or more amino acids; and
the Vβ comprises a CDR2 having an amino acid sequence of X1X2NX4X5X6 (SEQ ID NO: 17), wherein X1 is F or Y; X2 is N or Q; X4 is N or K; X5 is V, E, or G; and X6 is P, L, or V; and X6 is P, L, or V; or the Vβ comprises a CDR2 having an amino acid sequence as shown in SEQ ID NOs: 47, 48, or 49, or a functional variant thereof formed by insertion, deletion, or substitution of one or more amino acids;
and wherein the antigen-binding domain or fragment thereof is antigen-specific for the mutant KRAS G12V epitope.

In some embodiments, the Vα comprises a CDR3 having an amino acid sequence of X1X2X3X4X5X6GX8X9X10X11X12KLX15 (SEQ ID NO: 2), wherein X1 is A or V, X2 is E or V, X3 is R, P, or N, X4 is D, G, or S, X5 is I, G, or D, X6 is E, T, or null, X8 is A, G, or T, X9 is G or S, X10 is N or Y, X11 is N, G, or null, X12 is R or null, and X15 is I or T; and
the Vα comprises a CDR1 having an amino acid sequence of X1SX3X4X5X6 (SEQ ID NO: 5), wherein X1 is V or N; X3 is G or A; X4 is N or S; X5 is P, Q, or D; and X6 is Y or S; and
the Vα comprises a CDR2 having an amino acid sequence of X1X2X3X4X5NX7X8X9 (SEQ ID NO: 8), wherein X1 is Y, V, or null; X2 is I or Y; X3 is T, S, or R; X4 is G or S; X5 is D, G, or null; X7 is L, M, or null; X8 is V, D, or null; and X9 is K or null;
   and/or
the Vβ comprises a CDR3 having an amino acid sequence of ASSX4X5X6X7X8X9X10X12X13X14 (SEQ ID NO: 11), wherein X4 is E, H, or L; X5 is G, W, or V; X6 is F or G; X7 is Y, A, or S; X8 is T, Q, or P; X9 is E or D; X10 is Y or null; X12 is T, E, or null; X13 is A or Q; and X14 is F or Y; and
the Vβ comprises a CDR1 having an amino acid sequence of X1GHX4X5 (SEQ ID NO: 14), wherein X1 is S or L; X4 is N, D, or A; and X5 is S or T; and
the Vβ comprises a CDR2 having an amino acid sequence of X1X2NX4X5X6 (SEQ ID NO: 17), wherein X1 is F or Y; X2 is N or Q; X4 is N or K; X5 is V, E, or G;
and wherein the antigen-binding domain or fragment thereof is antigen-specific for the mutant KRAS G12V epitope.

In some embodiments, the Vα comprises a CDR3 having an amino acid sequence of X1VX3X4X6X7X8X9X10X11X13LX15 (SEQ ID NO: 3), wherein: X1 is A or V; X3 is N or null; X4 is L or null; X6 is T or null; X7 is G or null; X8 is A or null; X9 is A or null; X10 is N or G; X11 is N or A; X13 is K or R; and X15 is T or M; or the Vα comprises a CDR3 having an amino acid sequence of as shown in SEQ ID NOs: 25 or 26, or a functional variant thereof formed by insertion, deletion, or substitution of one or more amino acids; and
the Vα comprises a CDR1 having an amino acid sequence of X1X2X3SQS (SEQ ID NO: 6), wherein X1 is N or D; X2 is S or R; X3 is G or A; or the Vα comprises a CDR1 having an amino acid sequence of as shown in SEQ ID NOs: 30 or 31, or a functional variant thereof formed by insertion, deletion, or substitution of one or more amino acids; and
the Vα comprises a CDR2 having an amino acid sequence of X1X2X3SX5NX7X8 (SEQ ID NO: 9), wherein X1 is V or null; X2 is I or Y; X3 is S or Y; X5 is G or null; X7 is G or null; and X8 is D or null; or the Vα comprises a CDR2 having an amino acid sequence of as shown in SEQ ID NOs: 35 or 36, or a functional variant thereof formed by insertion, deletion, or substitution of one or more amino acids;
   and/or
the Vβ comprises a CDR3 having an amino acid sequence of ASX3X4X5X6SX8X9X10X11X12X13X14 (SEQ ID NO: 12), wherein: X3 is S or T; X4 is F or S; X5 is S or I; X6 is D or V; X8 is Y or S; X9 is N or G; X10 is S or R; X11 is G or null; X12 is E or P; X13 is Q or L; and X14 is F or H; or the Vβ comprises a CDR3 having an amino acid sequence of as shown in SEQ ID NOs: 40 or 41, or a functional variant thereof formed by insertion, deletion, or substitution of one or more amino acids; and
the Vβ comprises a CDR1 having an amino acid sequence of X1X2HX4X5 (SEQ ID NO: 15), wherein X1 is S or M; X2 is G or N; X4 is A or E; X5 is T or Y; or the Vβ comprises a CDR1 having an amino acid sequence as shown in SEQ ID NOs: 45 or 46, or a functional variant thereof formed by insertion, deletion, or substitution of one or more amino acids; and
the Vβ comprises a CDR2 having an amino acid sequence of X1X2NX4X5V (SEQ ID NO: 18), wherein X1 is F or S; X2 is Q or M; X4 is N or V; and X5 is E or G; or the Vβ comprises a CDR2 having an amino acid sequence as shown in SEQ ID NOs: 50 or 51, or a functional variant thereof formed by insertion, deletion, or substitution of one or more amino acids;
and wherein the antigen-binding domain or fragment thereof is antigen-specific for the mutant KRAS G12D epitope.

In some embodiments, the Vα comprises a CDR3 having an amino acid sequence of X1VX3X4X6X7X8X9X10X11X13LX15 (SEQ ID NO: 3), wherein X1 is A or V, X3 is N or null, X4 is L or null, X6 is T or null, X7 is G or null, X8 is A or null, X9 is A or null, X10 is N or G, X11 is N or A, X13 is K or R, and X15 is T or M; and
the Vα comprises a CDR1 having an amino acid sequence of X1X2X3SQS (SEQ ID NO: 6), wherein X1 is N or D; X2 is S or R; X3 is G or A; and
the Vα comprises a CDR2 having an amino acid sequence of X1X2X3SX5NX7X8 (SEQ ID NO: 9), wherein X1 is V or null; X2 is I or Y; X3 is S or Y; X5 is G or null; X7 is G or null; and X8 is D or null;
   and/or
the Vβ comprises a CDR3 having an amino acid sequence of ASX3X4X5X6SX8X9X10X11X12X13X14 (SEQ ID NO: 12), wherein X3 is S or T, X4 is F or S, X5 is S or I, X6 is D or V, X8 is Y or S, X9 is Nor G, X10 is S or R, X11 is G or null, X12 is E or P, X13 is Q or L, and X14 is F or H; and
the Vβ comprises a CDR1 having an amino acid sequence of X1X2HX4X5 (SEQ ID NO: 15), wherein X1 is S or M; X2 is G or N; X4 is A or E; X5 is T or Y; and
the Vβ comprises a CDR2 having an amino acid sequence of X1X2NX4X5V (SEQ ID NO: 18), wherein X1 is F or S; X2 is Q or M; X4 is N or V;
and wherein the antigen-binding domain or fragment thereof is antigen-specific for the mutant KRAS G12D epitope.

According to a second aspect, the present disclosure provides an antigen-binding protein comprising an antigen-binding domain or a fragment thereof of a T cell receptor (TCR), the antigen-binding domain or fragment thereof comprising a TCR α chain variable region (Vα) and a TCR β chain variable region (Vβ), wherein:
the Vα comprises a CDR3 having an amino acid sequence as shown in SEQ ID NOs: 22, 23, 24, 25, or 26, or a functional variant thereof formed by insertion, deletion, or substitution of one or more amino acids; and/or the Vβ comprises a CDR3 having an amino acid sequence as shown in SEQ ID NOs: 37, 38, 39, 40, or 41, or a functional variant thereof formed by insertion, deletion, or substitution of one or more amino acids;
and wherein the antigen-binding domain or fragment thereof is antigen-specific for the mutant KRAS G12 epitope.

In some embodiments, the Vα comprises a CDR3 having an amino acid sequence as shown in SEQ ID NOs: 22, 23, or 24, or a functional variant thereof formed by insertion, deletion, or substitution of one or more amino acids; and/or the Vβ comprises a CDR3 having an amino acid sequence as shown in SEQ ID NOs: 37, 38, or 39, or a functional variant thereof formed by insertion, deletion, or substitution of one or more amino acids. In some embodiments, the antigen-binding domain or fragment thereof is antigen-specific for the mutant KRAS G12V epitope.

In some embodiments, the Vα comprises a CDR3 having an amino acid sequence as shown in SEQ ID NOs: 25 or 26, or a functional variant thereof formed by insertion, deletion, or substitution of one or more amino acids; and/or the Vβ comprises a CDR3 having an amino acid sequence as shown in SEQ ID NOs: 40 or 41, or a functional variant thereof formed by insertion, deletion, or substitution of one or more amino acids. In some embodiments, the antigen-binding domain or fragment thereof is antigen-specific for the mutant KRAS G12D epitope.

In some embodiments, the Vα comprises a CDR3 having an amino acid sequence as shown in SEQ ID NO: 22, or a functional variant thereof formed by insertion, deletion, or substitution of one or more amino acids; and/or the Vβ comprises a CDR3 having an amino acid sequence as shown in SEQ ID NO: 37, or a functional variant thereof formed by insertion, deletion, or substitution of one or more amino acids. In some embodiments, the Vα comprises a CDR3 having an amino acid sequence as shown in SEQ ID NO: 22; and/or the Vβ comprises a CDR3 having an amino acid sequence as shown in SEQ ID NO: 37. In some embodiments, the Vα comprises a CDR3 having an amino acid sequence as shown in SEQ ID NO: 22; and the Vβ comprises a CDR3 having an amino acid sequence as shown in SEQ ID NO: 37. In some embodiments, the antigen-binding domain or fragment thereof is antigen-specific for the mutant KRAS G12V epitope.

In some embodiments, the Vα comprises a CDR3 having an amino acid sequence as shown in SEQ ID NO: 23, or a functional variant thereof formed by insertion, deletion, or substitution of one or more amino acids; and/or the Vβ comprises a CDR3 having an amino acid sequence as shown in SEQ ID NO: 38, or a functional variant thereof formed by insertion, deletion, or substitution of one or more amino acids. In some embodiments, the Vα comprises a CDR3 having an amino acid sequence as shown in SEQ ID NO: 23; and/or the Vβ comprises a CDR3 having an amino acid sequence as shown in SEQ ID NO: 38. In some embodiments, the Vα comprises a CDR3 having an amino acid sequence as shown in SEQ ID NO: 23; and the Vβ comprises a CDR3 having an amino acid sequence as shown in SEQ ID NO: 38. In some embodiments, the antigen-binding domain or fragment thereof is antigen-specific for the mutant KRAS G12V epitope.

In some embodiments, the Vα comprises a CDR3 having an amino acid sequence as shown in SEQ ID NO: 24, or a functional variant thereof formed by insertion, deletion, or substitution of one or more amino acids; and/or the Vβ comprises a CDR3 having an amino acid sequence as shown in SEQ ID NO: 39, or a functional variant thereof formed by insertion, deletion, or substitution of one or more amino acids. In some embodiments, the Vα comprises a CDR3 having an amino acid sequence as shown in SEQ ID NO: 24; and/or the Vβ comprises a CDR3 having an amino acid sequence as shown in SEQ ID NO: 39. In some embodiments, the Vα comprises a CDR3 having an amino acid sequence as shown in SEQ ID NO: 24; and the Vβ comprises a CDR3 having an amino acid sequence as shown in SEQ ID NO: 39. In some embodiments, the antigen-binding domain or fragment thereof is antigen-specific for the mutant KRAS G12V epitope.

In some embodiments, the Vα comprises a CDR3 having an amino acid sequence as shown in SEQ ID NO: 25, or a functional variant thereof formed by insertion, deletion, or substitution of one or more amino acids; and/or the Vβ comprises a CDR3 having an amino acid sequence as shown in SEQ ID NO: 40, or a functional variant thereof formed by insertion, deletion, or substitution of one or more amino acids. In some embodiments, the Vα comprises a CDR3 having an amino acid sequence as shown in SEQ ID NO: 25; and/or the Vβ comprises a CDR3 having an amino acid sequence as shown in SEQ ID NO: 40. In some embodiments, the Vα comprises a CDR3 having an amino acid sequence as shown in SEQ ID NO: 25; and the Vβ comprises a CDR3 having an amino acid sequence as shown in SEQ ID NO: 40. In some embodiments, the antigen-binding domain or fragment thereof is antigen-specific for the mutant KRAS G12D epitope.

In some embodiments, the Vα comprises a CDR3 having an amino acid sequence as shown in SEQ ID NO: 26, or a functional variant thereof formed by insertion, deletion, or substitution of one or more amino acids; and/or the Vβ comprises a CDR3 having an amino acid sequence as shown in SEQ ID NO: 41, or a functional variant thereof formed by insertion, deletion, or substitution of one or more amino acids. In some embodiments, the Vα comprises a CDR3 having an amino acid sequence as shown in SEQ ID NO: 26; and/or the Vβ comprises a CDR3 having an amino acid sequence as shown in SEQ ID NO: 41. In some embodiments, the Vα comprises a CDR3 having an amino acid sequence as shown in SEQ ID NO: 26; and the Vβ comprises a CDR3 having an amino acid sequence as shown in SEQ ID NO: 41. In some embodiments, the antigen-binding domain or fragment thereof is antigen-specific for the mutant KRAS G12D epitope.

In some embodiments, the Vα comprises a CDR1 having an amino acid sequence as shown in SEQ ID NOs: 27, 28, 29, 30, or 31, or a functional variant thereof formed by insertion, deletion, or substitution of one or more amino acids.

In some embodiments, the Vα comprises a CDR2 having an amino acid sequence as shown in SEQ ID NOs: 32, 33, 34, 35, or 36, or a functional variant thereof formed by insertion, deletion, or substitution of one or more amino acids.

In some embodiments, the Vα comprises a CDR1 having an amino acid sequence as shown in SEQ ID NOs: 27, 28, or 29, or a functional variant thereof formed by insertion, deletion, or substitution of one or more amino acids; and a CDR2 having an amino acid sequence as shown in SEQ ID NOs: 32, 33, or 34, or a functional variant thereof formed by insertion, deletion, or substitution of one or more amino acids. In some embodiments, the antigen-binding domain or fragment thereof is antigen-specific for the mutant KRAS G12V epitope.

In some embodiments, the Vα comprises a CDR1 having an amino acid sequence as shown in SEQ ID NO: 27, or a functional variant thereof formed by insertion, deletion, or substitution of one or more amino acids; and a CDR2 having an amino acid sequence as shown in SEQ ID NO: 32, or a functional variant thereof formed by insertion, deletion, or substitution of one or more amino acids. In some embodiments, the antigen-binding domain or fragment thereof is antigen-specific for the mutant KRAS G12V epitope.

In some embodiments, the Vα comprises a CDR1 having an amino acid sequence as shown in SEQ ID NO: 28, or a functional variant thereof formed by insertion, deletion, or substitution of one or more amino acids; and a CDR2 having an amino acid sequence as shown in SEQ ID NO: 33, or a functional variant thereof formed by insertion, deletion, or substitution of one or more amino acids. In some embodiments, the antigen-binding domain or fragment thereof is antigen-specific for the mutant KRAS G12V epitope.

In some embodiments, the Vα comprises a CDR1 having an amino acid sequence as shown in SEQ ID NO: 29, or a functional variant thereof formed by insertion, deletion, or substitution of one or more amino acids; and a CDR2 having an amino acid sequence as shown in SEQ ID NO: 34, or a functional variant thereof formed by insertion, deletion, or substitution of one or more amino acids. In some embodiments, the antigen-binding domain or fragment thereof is antigen-specific for the mutant KRAS G12V epitope.

In some embodiments, the Vα comprises a CDR1 having an amino acid sequence as shown in SEQ ID NOs: 30 or 31, or a functional variant thereof formed by insertion, deletion, or substitution of one or more amino acids; and a CDR2 having an amino acid sequence as shown in SEQ ID NOs: 35 or 36, or a functional variant thereof formed by insertion, deletion, or substitution of one or more amino acids. In some embodiments, the antigen-binding domain or fragment thereof is antigen-specific for the mutant KRAS G12D epitope.

In some embodiments, the Vα comprises a CDR1 having an amino acid sequence as shown in SEQ ID NO: 30, or a functional variant thereof formed by insertion, deletion, or substitution of one or more amino acids; and a CDR2 having an amino acid sequence as shown in SEQ ID NO: 35, or a functional variant thereof formed by insertion, deletion, or substitution of one or more amino acids. In some embodiments, the antigen-binding domain or fragment thereof is antigen-specific for the mutant KRAS G12D epitope.

In some embodiments, the Vα comprises a CDR1 having an amino acid sequence as shown in SEQ ID NO: 31, or a functional variant thereof formed by insertion, deletion, or substitution of one or more amino acids; and a CDR2 having an amino acid sequence as shown in SEQ ID NO: 36, or a functional variant thereof formed by insertion, deletion, or substitution of one or more amino acids. In some embodiments, the antigen-binding domain or fragment thereof is antigen-specific for the mutant KRAS G12D epitope.

In some embodiments, the Vβ comprises a CDR1 having an amino acid sequence as shown in SEQ ID NOs: 42, 43, 44, 45, or 46, or a functional variant thereof formed by insertion, deletion, or substitution of one or more amino acids.

In some embodiments, the Vβ comprises a CDR2 having an amino acid sequence as shown in SEQ ID NOs: 47, 48, 49, 50, or 51, or a functional variant thereof formed by insertion, deletion, or substitution of one or more amino acids.

In some embodiments, the Vβ comprises a CDR1 having an amino acid sequence as shown in SEQ ID NOs: 42, 43, or 44, or a functional variant thereof formed by insertion, deletion, or substitution of one or more amino acids; and a CDR2 having an amino acid sequence as shown in SEQ ID NOs: 47, 48, or 49, or a functional variant thereof formed by insertion, deletion, or substitution of one or more amino acids. In some embodiments, the antigen-binding domain or fragment thereof is antigen-specific for the mutant KRAS G12V epitope.

In some embodiments, the Vβ comprises a CDR1 having an amino acid sequence as shown in SEQ ID NO: 42, or a functional variant thereof formed by insertion, deletion, or substitution of one or more amino acids; and a CDR2 having an amino acid sequence as shown in SEQ ID NO: 47, or a functional variant thereof formed by insertion, deletion, or substitution of one or more amino acids. In some embodiments, the antigen-binding domain or fragment thereof is antigen-specific for the mutant KRAS G12V epitope.

In some embodiments, the Vβ comprises a CDR1 having an amino acid sequence as shown in SEQ ID NO: 43, or a functional variant thereof formed by insertion, deletion, or substitution of one or more amino acids; and a CDR2 having an amino acid sequence as shown in SEQ ID NO: 48, or a functional variant thereof formed by insertion, deletion, or substitution of one or more amino acids. In some embodiments, the antigen-binding domain or fragment thereof is antigen-specific for the mutant KRAS G12V epitope.

In some embodiments, the Vβ comprises a CDR1 having an amino acid sequence as shown in SEQ ID NO: 44, or a functional variant thereof formed by insertion, deletion, or substitution of one or more amino acids; and a CDR2 having an amino acid sequence as shown in SEQ ID NO: 49, or a functional variant thereof formed by insertion, deletion, or substitution of one or more amino acids. In some embodiments, the antigen-binding domain or fragment thereof is antigen-specific for the mutant KRAS G12V epitope.

In some embodiments, the Vβ comprises a CDR1 having an amino acid sequence as shown in SEQ ID NOs: 45 or 46, or a functional variant thereof formed by insertion, deletion, or substitution of one or more amino acids; and a CDR2 having an amino acid sequence as shown in SEQ ID NOs: 50 or 51, or a functional variant thereof formed by insertion, deletion, or substitution of one or more amino acids. In some embodiments, the antigen-binding domain or fragment thereof is antigen-specific for the mutant KRAS G12D epitope.

In some embodiments, the Vβ comprises a CDR1 having an amino acid sequence as shown in SEQ ID NO: 45, or a functional variant thereof formed by insertion, deletion, or substitution of one or more amino acids; and a CDR2 having an amino acid sequence as shown in SEQ ID NO: 50, or a functional variant thereof formed by insertion, deletion, or substitution of one or more amino acids. In some embodiments, the antigen-binding domain or fragment thereof is antigen-specific for the mutant KRAS G12D epitope.

In some embodiments, the Vβ comprises a CDR1 having an amino acid sequence as shown in SEQ ID NO: 46, or a functional variant thereof formed by insertion, deletion, or substitution of one or more amino acids; and a CDR2 having an amino acid sequence as shown in SEQ ID NO: 51, or a functional variant thereof formed by insertion, deletion, or substitution of one or more amino acids. In some embodiments, the antigen-binding domain or fragment thereof is antigen-specific for the mutant KRAS G12D epitope.

In some embodiments, the Vα comprises a CDR1 having an amino acid sequence as shown in SEQ ID NOs: 27, 28, 29, 30, or 31, or a functional variant thereof formed by insertion, deletion, or substitution of one or more amino acids; and a CDR2 having an amino acid sequence as shown in SEQ ID NOs: 32, 33, 34, 35, or 36, or a functional variant thereof formed by insertion, deletion, or substitution of one or more amino acids; and the Vβ comprises a CDR1 having an amino acid sequence as shown in SEQ ID NOs: 42, 43, 44, 45, or 46, or a functional variant thereof formed by insertion, deletion, or substitution of one or more amino acids; and a CDR2 having an amino acid sequence as shown in SEQ ID NOs: 47, 48, 49, 50, or 51, or a functional variant thereof formed by insertion, deletion, or substitution of one or more amino acids.

In some embodiments, the Vα comprises a CDR1 having an amino acid sequence as shown in SEQ ID NOs: 27, 28, or 29, or a functional variant thereof formed by insertion, deletion, or substitution of one or more amino acids; and a CDR2 having an amino acid sequence as shown in SEQ ID NOs: 32, 33, or 34, or a functional variant thereof formed by insertion, deletion, or substitution of one or more amino acids; and the Vβ comprises a CDR1 having an amino acid sequence as shown in SEQ ID NOs: 42, 43, or 44, or a functional variant thereof formed by insertion, deletion, or substitution of one or more amino acids; and a CDR2 having an amino acid sequence as shown in SEQ ID NOs: 47, 48, or 49, or a functional variant thereof formed by insertion, deletion, or substitution of one or more amino acids. In some embodiments, the antigen-binding domain or fragment thereof is antigen-specific for the mutant KRAS G12V epitope.

In some embodiments, the Vα comprises a CDR1 having an amino acid sequence as shown in SEQ ID NO: 27 or a functional variant thereof formed by insertion, deletion, or substitution of one or more amino acids, and a CDR2 having an amino acid sequence as shown in SEQ ID NO: 32 or a functional variant thereof formed by insertion, deletion, or substitution of one or more amino acids; and the Vβ comprises a CDR1 having an amino acid sequence as shown in SEQ ID NO: 42 or a functional variant thereof formed by insertion, deletion, or substitution of one or more amino acids, and a CDR2 having an amino acid sequence as shown in SEQ ID NO: 47 or a functional variant thereof formed by insertion, deletion, or substitution of one or more amino acids. In some embodiments, the antigen-binding domain or fragment thereof is antigen-specific for the mutant KRAS G12V epitope.

In some embodiments, the Vα comprises a CDR1 having an amino acid sequence as shown in SEQ ID NO: 28 or a functional variant thereof formed by insertion, deletion, or substitution of one or more amino acids, and a CDR2 having an amino acid sequence as shown in SEQ ID NO: 33 or a functional variant thereof formed by insertion, deletion, or substitution of one or more amino acids; and the Vβ comprises a CDR1 having an amino acid sequence as shown in SEQ ID NO: 43 or a functional variant thereof formed by insertion, deletion, or substitution of one or more amino acids, and a CDR2 having an amino acid sequence as shown in SEQ ID NO: 48 or a functional variant thereof formed by insertion, deletion, or substitution of one or more amino acids. In some embodiments, the antigen-binding domain or fragment thereof is antigen-specific for the mutant KRAS G12V epitope.

In some embodiments, the Vα comprises a CDR1 having an amino acid sequence as shown in SEQ ID NO: 29 or a functional variant thereof formed by insertion, deletion, or substitution of one or more amino acids, and a CDR2 having an amino acid sequence as shown in SEQ ID NO: 34 or a functional variant thereof formed by insertion, deletion, or substitution of one or more amino acids; and the Vβ comprises a CDR1 having an amino acid sequence as shown in SEQ ID NO: 44 or a functional variant thereof formed by insertion, deletion, or substitution of one or more amino acids, and a CDR2 having an amino acid sequence as shown in SEQ ID NO: 49 or a functional variant thereof formed by insertion, deletion, or substitution of one or more amino acids. In some embodiments, the antigen-binding domain or fragment thereof is antigen-specific for the mutant KRAS G12V epitope.

In some embodiments, the Vα comprises a CDR1 having an amino acid sequence as shown in SEQ ID NOs: 30 or 31 or a functional variant thereof formed by insertion, deletion, or substitution of one or more amino acids, and a CDR2 having an amino acid sequence as shown in SEQ ID NOs: 35 or 36 or a functional variant thereof formed by insertion, deletion, or substitution of one or more amino acids; and the Vβ comprises a CDR1 having an amino acid sequence as shown in SEQ ID NOs: 45 or 46 or a functional variant thereof formed by insertion, deletion, or substitution of one or more amino acids, and a CDR2 having an amino acid sequence as shown in SEQ ID NOs: 50 or 51 or a functional variant thereof formed by insertion, deletion, or substitution of one or more amino acids. In some embodiments, the antigen-binding domain or fragment thereof is antigen-specific for the mutant KRAS G12D epitope.

In some embodiments, the Vα comprises a CDR1 having an amino acid sequence as shown in SEQ ID NO: 30 or a functional variant thereof formed by insertion, deletion, or substitution of one or more amino acids, and a CDR2 having an amino acid sequence as shown in SEQ ID NO: 35 or a functional variant thereof formed by insertion, deletion, or substitution of one or more amino acids; and the Vβ comprises a CDR1 having an amino acid sequence as shown in SEQ ID NO: 45 or a functional variant thereof formed by insertion, deletion, or substitution of one or more amino acids, and a CDR2 having an amino acid sequence as shown in SEQ ID NO: 50 or a functional variant thereof formed by insertion, deletion, or substitution of one or more amino acids. In some embodiments, the antigen-binding domain or fragment thereof is antigen-specific for the mutant KRAS G12D epitope.

In some embodiments, the Vα comprises a CDR1 having an amino acid sequence as shown in SEQ ID NO: 31 or a functional variant thereof formed by insertion, deletion, or substitution of one or more amino acids, and a CDR2 having an amino acid sequence as shown in SEQ ID NO: 36 or a functional variant thereof formed by insertion, deletion, or substitution of one or more amino acids; and the Vβ comprises a CDR1 having an amino acid sequence as shown in SEQ ID NO: 46 or a functional variant thereof formed by insertion, deletion, or substitution of one or more amino acids, and a CDR2 having an amino acid sequence as shown in SEQ ID NO: 51 or a functional variant thereof formed by insertion, deletion, or substitution of one or more amino acids. In some embodiments, the antigen-binding domain or fragment thereof is antigen-specific for the mutant KRAS G12D epitope.

In some embodiments, the Vα comprises a CDR3 having an amino acid sequence as shown in SEQ ID NOs: 22, 23, 24, 25, or 26, or a functional variant thereof formed by insertion, deletion, or substitution of one or more amino acids; and
a CDR1 having an amino acid sequence as shown in SEQ ID NOs: 27, 28, 29, 30, or 31, or a functional variant thereof formed by insertion, deletion, or substitution of one or more amino acids;
   and/or
the Vβ comprises a CDR3 having an amino acid sequence as shown in SEQ ID NOs: 37, 38, 39, 40, or 41, or a functional variant thereof formed by insertion, deletion, or substitution of one or more amino acids; and
a CDR1 having an amino acid sequence as shown in SEQ ID NOs: 42, 43, 44, 45, or 46, or a functional variant thereof formed by insertion, deletion, or substitution of one or more amino acids.

In some embodiments, the Vα comprises a CDR3 having an amino acid sequence as shown in SEQ ID NOs: 22, 23, or 24, or a functional variant thereof formed by insertion, deletion, or substitution of one or more amino acids; and
a CDR1 having an amino acid sequence as shown in SEQ ID NOs: 27, 28, or 29, or a functional variant thereof formed by insertion, deletion, or substitution of one or more amino acids;
   and/or
the Vβ comprises a CDR3 having an amino acid sequence as shown in SEQ ID NOs: 37, 38, or 39, or a functional variant thereof formed by insertion, deletion, or substitution of one or more amino acids; and
a CDR1 having an amino acid sequence as shown in SEQ ID NOs: 42, 43, or 44, or a functional variant thereof formed by insertion, deletion, or substitution of one or more amino acids;
and wherein the antigen-binding domain or fragment thereof is antigen-specific for the mutated KRAS G12V epitope.

In some embodiments, the Vα comprises a CDR3 having an amino acid sequence as shown in SEQ ID NOs: 25 or 26, or a functional variant thereof formed by insertion, deletion, or substitution of one or more amino acids; and
a CDR1 having an amino acid sequence as shown in SEQ ID NOs: 30 or 31, or a functional variant thereof formed by insertion, deletion, or substitution of one or more amino acids;
   and/or
the Vβ comprises a CDR3 having an amino acid sequence as shown in SEQ ID NOs: 40 or 41, or a functional variant thereof formed by insertion, deletion, or substitution of one or more amino acids; and
a CDR1 having an amino acid sequence as shown in SEQ ID NOs: 45 or 46, or a functional variant thereof formed by insertion, deletion, or substitution of one or more amino acids;
and wherein the antigen-binding domain or fragment thereof is antigen-specific for the mutated KRAS G12D epitope.

In some embodiments, the Vα comprises a CDR3 having an amino acid sequence as shown in SEQ ID NOs: 22, 23, 24, 25, or 26, or a functional variant thereof formed by insertion, deletion, or substitution of one or more amino acids; and
a CDR2 having an amino acid sequence as shown in SEQ ID NOs: 32, 33, 34, 35, or 36, or a functional variant thereof formed by insertion, deletion, or substitution of one or more amino acids;
   and/or
the Vβ comprises a CDR3 having an amino acid sequence as shown in SEQ ID NOs: 37, 38, 39, 40, or 41, or a functional variant thereof formed by insertion, deletion, or substitution of one or more amino acids; and
a CDR2 having an amino acid sequence as shown in SEQ ID NOs: 47, 48, 49, 50, or 51, or a functional variant thereof formed by insertion, deletion, or substitution of one or more amino acids.

In some embodiments, the Vα comprises a CDR3 having an amino acid sequence as shown in SEQ ID NOs: 22, 23, or 24, or a functional variant thereof formed by insertion, deletion, or substitution of one or more amino acids; and
a CDR2 having an amino acid sequence as shown in SEQ ID NOs: 32, 33, or 34, or a functional variant thereof formed by insertion, deletion, or substitution of one or more amino acids;
   and/or
the Vβ comprises a CDR3 having an amino acid sequence as shown in SEQ ID NOs: 37, 38, or 39, or a functional variant thereof formed by insertion, deletion, or substitution of one or more amino acids; and
a CDR2 having an amino acid sequence as shown in SEQ ID NOs: 47, 48, or 49, or a functional variant thereof formed by insertion, deletion, or substitution of one or more amino acids;
and wherein the antigen-binding domain or fragment thereof is antigen-specific for the mutated KRAS G12V epitope.

In some embodiments, the Vα comprises a CDR3 having an amino acid sequence as shown in SEQ ID NOs: 25 or 26, or a functional variant thereof formed by insertion, deletion, or substitution of one or more amino acids; and
a CDR2 having an amino acid sequence as shown in SEQ ID NOs: 35 or 36, or a functional variant thereof formed by insertion, deletion, or substitution of one or more amino acids;
   and/or
the Vβ comprises a CDR3 having an amino acid sequence as shown in SEQ ID NOs: 40 or 41, or a functional variant thereof formed by insertion, deletion, or substitution of one or more amino acids; and
a CDR2 having an amino acid sequence as shown in SEQ ID NOs: 50 or 51, or a functional variant thereof formed by insertion, deletion, or substitution of one or more amino acids;
and wherein the antigen-binding domain or fragment thereof is antigen-specific for the mutated KRAS G12D epitope.

In a third aspect, the present disclosure provides an antigen-binding protein comprising an antigen-binding domain or a fragment thereof of a T-cell receptor (TCR), wherein the antigen-binding domain or fragment thereof comprises a TCR α chain variable region (Vα) and a TCR β chain variable region (Vβ); wherein
the Vα comprises a CDR1 having an amino acid sequence selected from the group consisting of SEQ ID NOs: 27, 28, 29, 30, or 31 or a functional variant thereof formed by insertion, deletion, or substitution of one or more amino acids; and a CDR2 having an amino acid sequence selected from the group consisting of SEQ ID NOs: 32, 33, 34, 35, or 36 or a functional variant thereof formed by insertion, deletion, or substitution of one or more amino acids; and a CDR3 having an amino acid sequence selected from the group consisting of SEQ ID NOs: 22, 23, 24, 25, or 26 or a functional variant thereof formed by insertion, deletion, or substitution of one or more amino acids;
   and/or
the Vβ comprises a CDR1 having an amino acid sequence selected from the group consisting of SEQ ID NOs: 42, 43, 44, 45, or 46 or a functional variant thereof formed by insertion, deletion, or substitution of one or more amino acids; and a CDR2 having an amino acid sequence selected from the group consisting of SEQ ID NOs: 47, 48, 49, 50, or 51 or a functional variant thereof formed by insertion, deletion, or substitution of one or more amino acids; and a CDR3 having an amino acid sequence selected from the group consisting of SEQ ID NOs: 37, 38, 39, 40, or 41 or a functional variant thereof formed by insertion, deletion, or substitution of one or more amino acids.

In some embodiments, the Vα comprises a CDR3 having an amino acid sequence selected from the group consisting of SEQ ID NOs: 22, 23, or 24 or a functional variant thereof formed by insertion, deletion, or substitution of one or more amino acids; and a CDR1 having an amino acid sequence selected from the group consisting of SEQ ID NOs: 27, 28, or 29 or a functional variant thereof formed by insertion, deletion, or substitution of one or more amino acids; and a CDR2 having an amino acid sequence selected from the group consisting of SEQ ID NOs: 32, 33, or 34 or a functional variant thereof formed by insertion, deletion, or substitution of one or more amino acids;
and/or
the Vβ comprises a CDR3 having an amino acid sequence selected from the group consisting of SEQ ID NOs: 37, 38, or 39 or a functional variant thereof formed by insertion, deletion, or substitution of one or more amino acids; and a CDR1 having an amino acid sequence selected from the group consisting of SEQ ID NOs: 42, 43, or 44 or a functional variant thereof formed by insertion, deletion, or substitution of one or more amino acids; and a CDR2 having an amino acid sequence selected from the group consisting of SEQ ID NOs: 47, 48, or 49 or a functional variant thereof formed by insertion, deletion, or substitution of one or more amino acids;
preferably, the antigen-binding domain or fragment thereof is antigen-specific for the mutated KRAS G12V epitope.

In some embodiments, the Vα comprises a CDR3 having an amino acid sequence as shown in SEQ ID NOs: 25 or 26 or a functional variant thereof formed by insertion, deletion, or substitution of one or more amino acids; and a CDR1 having an amino acid sequence as shown in SEQ ID NOs: 30 or 31 or a functional variant thereof formed by insertion, deletion, or substitution of one or more amino acids; and a CDR2 having an amino acid sequence as shown in SEQ ID NOs: 35 or 36 or a functional variant thereof formed by insertion, deletion, or substitution of one or more amino acids;
and/or
the Vβ comprises a CDR3 having an amino acid sequence as shown in SEQ ID NOs: 40 or 41 or a functional variant thereof formed by insertion, deletion, or substitution of one or more amino acids; and a CDR1 having an amino acid sequence as shown in SEQ ID NOs: 45 or 46 or a functional variant thereof formed by insertion, deletion, or substitution of one or more amino acids; and a CDR2 having an amino acid sequence as shown in SEQ ID NOs: 50 or 51 or a functional variant thereof formed by insertion, deletion, or substitution of one or more amino acids;
wherein the antigen-binding domain or fragment thereof is antigen-specific for the mutated KRAS G12D epitope.

In some embodiments, the Vα comprises CDR1, CDR2, and CDR3 having amino acid sequences as shown in SEQ ID NOs: 27, 32, and 22, respectively, or functional variants thereof formed by insertion, deletion, or substitution of one or more amino acids, and/or the Vβ comprises CDR1, CDR2, and CDR3 having amino acid sequences as shown in SEQ ID NOs: 42, 47, and 37, respectively, or functional variants thereof formed by insertion, deletion, or substitution of one or more amino acids. In some embodiments, the Vα comprises CDR1, CDR2, and CDR3 having amino acid sequences as shown in SEQ ID NOs: 27, 32, and 22, respectively, and/or the Vβ comprises CDR1, CDR2, and CDR3 having amino acid sequences as shown in SEQ ID NOs: 42, 47, and 37, respectively. In some embodiments, the Vα comprises CDR1, CDR2, and CDR3 having amino acid sequences as shown in SEQ ID NOs: 27, 32, and 22, respectively, and the Vβ comprises CDR1, CDR2, and CDR3 having amino acid sequences as shown in SEQ ID NOs: 42, 47, and 37, respectively. In some embodiments, the antigen-binding domain or fragment thereof is antigen-specific for the mutated KRAS G12V epitope.

In some embodiments, the Vα comprises CDR1, CDR2, and CDR3 having amino acid sequences as shown in SEQ ID NOs: 28, 33, and 23, respectively, or functional variants thereof formed by insertion, deletion, or substitution of one or more amino acids, and/or the Vβ comprises CDR1, CDR2, and CDR3 having amino acid sequences as shown in SEQ ID NOs: 43, 48, and 38, respectively, or functional variants thereof formed by insertion, deletion, or substitution of one or more amino acids. In some embodiments, the Vα comprises CDR1, CDR2, and CDR3 having amino acid sequences as shown in SEQ ID NOs: 28, 33, and 23, respectively, and/or the Vβ comprises CDR1, CDR2, and CDR3 having amino acid sequences as shown in SEQ ID NOs: 43, 48, and 38, respectively. In some embodiments, the Vα comprises CDR1, CDR2, and CDR3 having amino acid sequences as shown in SEQ ID NOs: 28, 33, and 23, respectively, and the Vβ comprises CDR1, CDR2, and CDR3 having amino acid sequences as shown in SEQ ID NOs: 43, 48, and 38, respectively. In some embodiments, the antigen-binding domain or fragment thereof is antigen-specific for a mutated KRAS G12V epitope.

In some embodiments, the Vα comprises CDR1, CDR2, and CDR3 having amino acid sequences as shown in SEQ ID NOs: 29, 34, and 24, respectively, or functional variants thereof formed by insertion, deletion, or substitution of one or more amino acids, and/or the Vβ comprises CDR1, CDR2, and CDR3 having amino acid sequences as shown in SEQ ID NOs: 44, 49, and 39, respectively, or functional variants thereof formed by insertion, deletion, or substitution of one or more amino acids. In some embodiments, the Vα comprises CDR1, CDR2, and CDR3 having amino acid sequences as shown in SEQ ID NOs: 29, 34, and 24, respectively, and/or the Vβ comprises CDR1, CDR2, and CDR3 having amino acid sequences as shown in SEQ ID NOs: 44, 49, and 39, respectively. In some embodiments, the Vα comprises CDR1, CDR2, and CDR3 having amino acid sequences as shown in SEQ ID NOs: 29, 34, and 24, respectively, and the Vβ comprises CDR1, CDR2, and CDR3 having amino acid sequences as shown in SEQ ID NOs: 44, 49, and 39, respectively. In some embodiments, the antigen-binding domain or fragment thereof is antigen-specific for a mutated KRAS G12V epitope.

In some embodiments, the Vα comprises CDR1, CDR2, and CDR3 having amino acid sequences as shown in SEQ ID NOs: 30, 35, and 25, respectively, or functional variants thereof formed by insertion, deletion, or substitution of one or more amino acids, and/or the Vβ comprises CDR1, CDR2, and CDR3 having amino acid sequences as shown in SEQ ID NOs: 45, 50, and 40, respectively, or functional variants thereof formed by insertion, deletion, or substitution of one or more amino acids. In some embodiments, the Vα comprises CDR1, CDR2, and CDR3 having amino acid sequences as shown in SEQ ID NOs: 30, 35, and 25, respectively, and/or the Vβ comprises CDR1, CDR2, and CDR3 having amino acid sequences as shown in SEQ ID NOs: 45, 50, and 40, respectively. In some embodiments, the Vα comprises CDR1, CDR2, and CDR3 having amino acid sequences as shown in SEQ ID NOs: 30, 35, and 25, respectively, and the Vβ comprises CDR1, CDR2, and CDR3 having amino acid sequences as shown in SEQ ID NOs: 45, 50, or 40, respectively. In some embodiments, the antigen-binding domain or fragment thereof is antigen-specific for the mutated KRAS G12D epitope.

In some embodiments, the Vα comprises CDR1, CDR2, and CDR3 having amino acid sequences as shown in SEQ ID NOs: 31, 36, and 26, respectively, or functional variants thereof formed by insertion, deletion, or substitution of one or more amino acids, and/or the Vβ comprises CDR1, CDR2, and CDR3 having amino acid sequences as shown in SEQ ID NOs: 46, 51, and 41, respectively, or functional variants thereof formed by insertion, deletion, or substitution of one or more amino acids. In some embodiments, the Vα comprises CDR1, CDR2, and CDR3 having amino acid sequences as shown in SEQ ID NOs: 31, 36, and 26, respectively, and/or the Vβ comprises CDR1, CDR2, and CDR3 having amino acid sequences as shown in SEQ ID NOs: 46, 51, or 41, respectively. In some embodiments, the Vα comprises CDR1, CDR2, and CDR3 having amino acid sequences as shown in SEQ ID NOs: 31, 36, and 26, respectively, and the Vβ comprises CDR1, CDR2, and CDR3 having amino acid sequences as shown in SEQ ID NOs: 46, 51, or 41, respectively. In some embodiments, the antigen-binding domain or fragment thereof is antigen-specific for a mutated KRAS G12D epitope.

In some embodiments, the Vα comprises an amino acid sequence having at least 80%, at least 85%, at least 90%, at least 95%, or 100% sequence identity to an amino acid sequence as shown in SEQ ID NOs: 52, 53, 54, 55, or 56, and/or the Vβ comprises an amino acid sequence having at least 80%, at least 85%, at least 90%, at least 95%, or 100% sequence identity to an amino acid sequence as shown in SEQ ID NOs: 57, 58, 59, 60, or 61. In some embodiments, the Vα comprises an amino acid sequence as shown in SEQ ID NOs: 52, 53, 54, 55, or 56, and/or the Vβ comprises an amino acid sequence as shown in SEQ ID NOs: 57, 58, 59, 60, or 61. In some embodiments, the Vα comprises an amino acid sequence as shown in SEQ ID NOs: 52, 53, 54, 55, or 56, and the Vβ comprises an amino acid sequence as shown in SEQ ID Nos: 57, 58, 59, 60, or 61.

In some embodiments, the Vα comprises an amino acid sequence having at least 80%, at least 85%, at least 90%, at least 95%, or 100% sequence identity to an amino acid sequence as shown in SEQ ID NO: 52, and/or the Vβ comprises an amino acid sequence having at least 80%, at least 85%, at least 90%, at least 95%, or 100% sequence identity to an amino acid sequence as shown in SEQ ID NO: 57. In some embodiments, the Vα comprises an amino acid sequence as shown in SEQ ID NO: 52, and/or the Vβ comprises an amino acid sequence as shown in SEQ ID NO: 57. In some embodiments, the Vα comprises an amino acid sequence as shown in SEQ ID NO: 52, and the Vβ comprises an amino acid sequence as shown in SEQ ID NO: 57. In some embodiments, the antigen-binding domain or fragment thereof is antigen-specific for a mutated KRAS G12V epitope.

In some embodiments, the Vα comprises an amino acid sequence having at least 80%, at least 85%, at least 90%, at least 95%, or 100% sequence identity to an amino acid sequence as shown in SEQ ID NO: 53, and/or the Vβ comprises an amino acid sequence having at least 80%, at least 85%, at least 90%, at least 95%, or 100% sequence identity to an amino acid sequence as shown in SEQ ID NO: 58. In some embodiments, the Vα comprises an amino acid sequence as shown in SEQ ID NO: 53, and/or the Vβ comprises an amino acid sequence as shown in SEQ ID NO: 58. In some embodiments, the Vα comprises an amino acid sequence as shown in SEQ ID NO: 53, and the Vβ comprises an amino acid sequence as shown in SEQ ID NO: 58. In some embodiments, the antigen-binding domain or fragment thereof is antigen-specific for a mutated KRAS G12V epitope.

In some embodiments, the Vα comprises an amino acid sequence having at least 80%, at least 85%, at least 90%, at least 95%, or 100% sequence identity to an amino acid sequence as shown in SEQ ID NO: 54, and/or the Vβ comprises an amino acid sequence having at least 80%, at least 85%, at least 90%, at least 95%, or 100% sequence identity to an amino acid sequence as shown in SEQ ID NO: 59. In some embodiments, the Vα comprises an amino acid sequence as shown in SEQ ID NO: 54, and/or the Vβ comprises an amino acid sequence as shown in SEQ ID NO: 59. In some embodiments, the Vα comprises an amino acid sequence as shown in SEQ ID NO: 54, and the Vβ comprises an amino acid sequence as shown in SEQ ID NO: 59. In some embodiments, the antigen-binding domain or fragment thereof is antigen-specific for the mutated KRAS G12V epitope.

In some embodiments, the Vα comprises an amino acid sequence having at least 80%, at least 85%, at least 90%, at least 95%, or 100% sequence identity to an amino acid sequence as shown in SEQ ID NO: 55, and/or the Vβ comprises an amino acid sequence having at least 80%, at least 85%, at least 90%, at least 95%, or 100% sequence identity to an amino acid sequence as shown in SEQ ID NO: 60. In some embodiments, the Vα comprises an amino acid sequence as shown in SEQ ID NO: 55, and/or the Vβ comprises an amino acid sequence as shown in SEQ ID NO: 60. In some embodiments, the Vα comprises an amino acid sequence as shown in SEQ ID NO: 55, and the Vβ comprises an amino acid sequence as shown in SEQ ID NO: 60. In some embodiments, the antigen-binding domain or fragment thereof is antigen-specific for the mutated KRAS G12D epitope.

In some embodiments, the Vα comprises an amino acid sequence having at least 80%, at least 85%, at least 90%, at least 95%, or 100% sequence identity to an amino acid sequence as shown in SEQ ID NO: 56, and/or the Vβ comprises an amino acid sequence having at least 80%, at least 85%, at least 90%, at least 95%, or 100% sequence identity to an amino acid sequence as shown in SEQ ID NO: 61. In some embodiments, the Vα comprises an amino acid sequence as shown in SEQ ID NO: 56, and/or the Vβ comprises an amino acid sequence as shown in SEQ ID NO: 61. In some embodiments, the Vα comprises an amino acid sequence as shown in SEQ ID NO: 56, and the Vβ comprises an amino acid sequence as shown in SEQ ID NO: 61. In some embodiments, the antigen-binding domain or fragment thereof is antigen-specific for the mutated KRAS G12D epitope.

As used herein, the term "sequence identity" indicates the extent to which two (nucleotide or amino acid) sequences have the same residues at the same positions in alignment, and is typically expressed as a percentage. Preferably, identity is determined over the overall length of the sequences being compared. Thus, two copies with the identical sequence have 100% identity, while sequences that are less highly conserved and have deletions, additions or substitutions may have a lower degree of identity. Those skilled in the art will recognize that a number of algorithms can be used to determine sequence identity using standard parameters, such as Blast (Altschul et al. (1997) Nucleic Acids Res. 25: 3389-3402), Blast2 (Altschul et al. (1990) J. Mol. Biol. 215: 403-410), Smith-Waterman (Smith et al. (1981) J. Mol. Biol. 147: 195-197), and ClustalW.

Accordingly, the amino acid sequences of SEQ ID NOs: 52, 53, 54, 55, or 56 may serve, for example, as the "subject sequences" or "reference sequences" of Vα, and the amino acid sequences of SEQ ID NOs: 57, 58, 59, 60, or 61 may serve, for example, as the "subject sequences" or "reference sequences" of Vβ, while different Vα or Vβ chain amino acid sequences may serve as "query sequences."

In some embodiments of the antigen-binding proteins as described above in the present disclosure, the antigen-binding protein binds to a KRAS G12 mutant epitope or a complex of the epitope with an MHC molecule. In some embodiments, the KRAS mutant epitope includes at least one of G12V and G12D. In some embodiments, the MHC molecule is HLA-A*11 type, for example, HLA-A*11:01.

In some embodiments, the Vα is contained on a first polypeptide and the Vβ is contained on a different second polypeptide. In some embodiments, the Vα and Vβ are contained on a single polypeptide.

In some embodiments, the antigen-binding protein is soluble. In some embodiments, the antigen-binding protein is membrane-bound.

The antigen-binding protein of the present disclosure may be provided in soluble form, for example, provided in the form of a soluble TCR. Soluble TCRs (sTCRs) may be used as diagnostic tools or as vectors or "bridging molecules" for specifically targeting therapeutic agents or effector cells to cancer cells that for example express an antigen target recognized by the soluble TCR. Soluble TCRs are typically fragments or constructs comprising the TCR α chain and/or β chain or their variable regions or CDRs, optionally stabilized by disulfide bond(s) or covalently linked by suitable linker(s). Typically, soluble TCRs do not include, for example, transmembrane domains.

The antigen-binding protein of the present disclosure may also be provided in membrane-bound form, for example, provided in the form of a membrane-bound TCR. Typically, the membrane-bound TCRs include a transmembrane domain to anchor the TCR to the cell membrane.

In some embodiments, the antigen-binding protein is selected from the group consisting of a TCR, a chimeric antigen receptor (CAR), an Fc polypeptide, or antigen-binding fragments thereof.

As used herein, the term "constant region" may be a human constant region or derived from another species, resulting in a "chimeric" TCR. For example, human α chain and/or β chain may be replaced by their murine counterparts ("murine-derived"), and the murine counterparts have been found to enhance surface expression of the human TCR and enhance binding stability of the human TCR to CD3 co-receptors by supporting preferential pairing of TCR α and β chains.

In some embodiments, the TCR constant region is a murine constant region or a human constant region.

It is reported that the addition of disulfide bonds to the constant region may promote the correct pairing of TCR α and β chains (Kuball J et al. Blood. 2007 Mar 15;109(6):2331-8). Therefore, the present disclosure also envisages the addition of one or more cysteine modifications to the constant region to form disulfide bonds between the TCR α and TCR β chains.

In some embodiments, the TCR constant region comprises a TCR alpha chain constant region and/or a TCR beta chain constant region; preferably, the TCR alpha chain constant region and/or TCR beta chain constant region comprises at least one cysteine mutation relative to a wild-type sequence to form a disulfide bond between the TCR alpha chain and the TCR beta chain.

In some embodiments, the cysteine mutation is at one or more of the following positions: position 48 of the wild-type human TCR alpha chain constant region, position 48 of the wild-type murine TCR alpha chain constant region, position 57 of the wild-type human TCR beta chain constant region, and position 57 of the wild-type murine TCR beta chain constant region.

The sequence of the wild-type TCR constant region can be found in the public database of the International Immunogenetics Information System (IMGT). For example, the sequence of the TCR alpha chain constant domain is "TRAC*01" and the sequence of TCR beta chain constant domain is "TRBC1*01 " or "TRBC2*01".

To facilitate the description of the location of the cysteine mutation, the positions of the amino acid sequences of the wild-type TCR constant region in the present disclosure are numbered according to the nomenclature of the International Immunogenetics Information System (IMGT). For example, if an amino acid in the TCR alpha chain constant region (TRAC) is designated with a position number of 48 in the IMGT, it is described herein as the amino acid at position 48 of the TCR alpha chain constant region (TRAC); if an amino acid in the TCR beta chain constant region (TRBC) is designated with a position number of 57 in the IMGT, it is described herein as the amino acid at position 57 of the TCR beta chain constant region ( TRBC); and so on. Herein, the position numbering of the amino acid sequences of the variable regions TRAV and TRBV is based on the position numbering listed in IMGT. If an amino acid in TRAV is designated with a position number of 46 in IMGT, it is described herein as the amino acid at position 46 of TRAV; and so on. When the sequence position numbering of other amino acids is specifically described in the present disclosure, they are numbered as specifically described.

In some embodiments, the TCR alpha chain constant region further comprises an LVL mutation or an LIV mutation such that the constant region (and/or the transmembrane region) comprises the amino acid sequence L**L**VI**VL**RIL. For example, when the TCR alpha chain comprises a human constant region, the human constant region may comprise an LVL mutation such that the constant region (and/or the transmembrane region) comprises the amino acid sequence L**L**VI**VL**RIL. When the TCR alpha chain contains a murine constant region, the murine constant region may comprise an LIV mutation such that the constant region (and/or the transmembrane region) comprises the amino acid sequence L**L**VI**VL**RIL.

In some embodiments, the TCR α chain constant region comprises an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to any of SEQ ID NOs: 62-66, and/or the TCR β chain constant region comprises an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to any of SEQ ID NOs: 67-76.

In some embodiments, the TCR α chain constant region comprises an amino acid sequence as shown in SEQ ID NO: 66, and the TCR β chain constant region comprises an amino acid sequence as shown in SEQ ID NO: 72.

In some embodiments, the fragment of the TCR constant region is the extracellular segment of the TCR constant region.

In some embodiments, the antigen-binding protein is a TCR comprising an α chain and a β chain. In some instances, the TCR α chain and/or β chain may include a leader sequence. For example, the TCR α chain leader sequence may have an amino acid sequence as shown in SEQ ID NOs: 19, 21, 107, or 109. The TCR β chain leader sequence may have an amino acid sequence as shown in SEQ ID NOs: 20, 106, 108, or 110. In some embodiments, the TCR α chain leader sequence may have an amino acid sequence as shown in SEQ ID NO: 19 and the TCR β chain leader sequence may have an amino acid sequence as shown in SEQ ID NO: 20. In some embodiments, the TCR α chain leader sequence may have an amino acid sequence as shown in SEQ ID NO: 21 and the TCR β chain leader sequence may have an amino acid sequence as shown in SEQ ID NO: 106. In some embodiments, the TCR α chain leader sequence may have an amino acid sequence as shown in SEQ ID NO: 107 and the TCR β chain leader sequence may have an amino acid sequence as shown in SEQ ID NO: 108. In some embodiments, the TCR α chain leader sequence may have an amino acid sequence as shown in SEQ ID NO: 109 and the TCR β chain leader sequence may have an amino acid sequence as shown in SEQ ID NO: 108. In some embodiments, the TCR α chain leader sequence may have an amino acid sequence as shown in SEQ ID NO: 21 and the TCR β chain leader sequence may have an amino acid sequence as shown in SEQ ID NO: 110.

In some embodiments, the antigen-binding protein further comprises a transmembrane region and/or a cytoplasmic region.

In some embodiments, the antigen-binding protein comprises a TCR α chain comprising an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to any one of SEQ ID NOs: 77-81; and/or a TCR β chain comprising an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to any one of SEQ ID NOs: 82-86.

In some embodiments, the antigen-binding protein comprises a TCR α chain comprising an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 77; and/or a TCR β chain comprising an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 82. In some embodiments, the antigen-binding protein comprises a TCR α chain as shown in SEQ ID NO: 77 and a TCR β chain as shown in SEQ ID NO: 82.

In some embodiments, the antigen-binding protein comprises a TCR α chain comprising an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 78; and/or a TCR β chain comprising an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 83. In some embodiments, the antigen-binding protein comprises a TCR α chain as shown in SEQ ID NO: 78 and a TCR β chain as shown in SEQ ID NO: 83.

In some embodiments, the antigen-binding protein comprises a TCR α chain comprising an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 79; and/or a TCR β chain comprising an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 84. In some embodiments, the antigen-binding protein comprises a TCR α chain as shown in SEQ ID NO: 79 and a TCR β chain as shown in SEQ ID NO: 84.

In some embodiments, the antigen-binding protein comprises a TCR α chain comprising an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 80; and/or a TCR β chain comprising an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 85. In some embodiments, the antigen-binding protein comprises a TCR α chain as shown in SEQ ID NO: 80 and a TCR β chain as shown in SEQ ID NO: 85.

In some embodiments, the antigen-binding protein comprises a TCR α chain comprising an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 81; and/or a TCR β chain comprising an amino acid sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 86. In some embodiments, the antigen-binding protein comprises a TCR α chain as shown in SEQ ID NO: 81 and a TCR β chain as shown in SEQ ID NO: 86.

In some embodiments, the antigen binding protein further comprises an intracellular signaling region. In some embodiments, the antigen binding protein further comprises one or more antigen-binding regions that bind other antigens or epitopes.

In some embodiments, the antigen binding protein is isolated or purified.

As used herein, the term "isolated or purified" means that the "isolated or purified" antigen binding protein has been identified, isolated and/or recovered from components of the environment in which it is produced, such that the "isolated or purified" antigen binding protein is free or substantially free of other contaminant components from the environment in which it is produced that may interfere with its therapeutic or diagnostic use. Contaminant components may include enzymes, hormones, and other proteins or non-protein solutes. Thus, the "isolated or purified" antigen binding protein may be prepared by at least one purification step that removes or substantially removes these contaminant components.

In some embodiments, the antigen-binding protein provided in the present disclosure has one or more of the following properties:
1. capable of binding to the target antigenic peptide with an EC50 of less than or equal to about 1.0×10⁻⁷ M;
2. high membrane stability;
3. specific killing activity against antigen-positive tumor cells; and
4. absence of alloreactivity to different HLA subtypes.

Effector cells expressing the antigen-binding protein of the present disclosure, such as a TCR, bind their antigen targets with high affinity (preferably an antigen epitope presented by an antigen-presenting cell on HLA-A*11). The term "affinity" or "binding affinity" refers to the capacity of a T cell expressing the TCR of the present disclosure to respond to a given concentration of ligand in vitro, and the ability is considered to correlate with the in vivo effector capacity of the cells expressing the TCR. By definition, the cells expressing the TCR having high binding affinity respond to very low doses of antigen in vitro, whereas such cells having lower binding affinity require higher amounts of antigen if the immune response similar to that of the cells expressing the TCR having high affinity is desired. Thus, binding affinity can be regarded as a quantitative determinant of the activation threshold of the cells expressing the TCR. For example, if the EC50 for activating target cells expressing target antigen peptide-HLA-A*11 by cells expressing TCR upon co-culture is not greater than 10⁻⁵ M (e.g., 10⁻⁵ M or lower, such as 10⁻⁶ M, 10⁻⁷ M, 10⁻⁸ M, or 10⁻⁹ M), then the cells expressing TCR are generally considered to bind their antigen targets with "high" affinity. In some embodiments, the antigen-binding protein binds the target antigen peptide with an EC50 of less than or equal to about 1.0×10⁻⁷ M. In some embodiments, the EC50 can be determined by introducing the TCR provided by the present disclosure into Jurkat cells carrying an NFAT-GFP reporter gene, co-incubating with T2 cells loaded with different concentrations of target antigen peptides, and detecting the activation level of the reporter gene in Jurkat cells.

In some embodiments, the antigen-binding protein provided by the present disclosure exhibits high membrane stability. As used herein, the term "high membrane stability" refers to the characteristics of stable expression of the antigen-binding protein, such as a TCR, on the cell membrane. In certain embodiments, "high membrane stability" refers to the characteristics that TCR is stably and abundantly expressed on the cell membrane, which may be characterized by expression rate of TCR on membrane. The expression rate may be determined using methods described in the examples of the present disclosure. "High membrane stability" may refer to that the expression rate of the TCR on membrane is ≥35%, ≥40%, ≥45%, ≥50%, ≥55%, ≥60%, ≥65%, ≥70%, ≥75%, ≥76%, ≥77%, ≥78%, ≥79%, ≥80%, ≥81%, ≥82%, ≥83%, ≥84%, ≥85%, ≥86%, ≥87%, ≥88%, ≥89%, or ≥90%.

In some embodiments, the antigen-binding protein provided by the present disclosure exhibits specific killing activity against antigen-positive tumor cells. As used herein, the term "specific killing activity" refers to the ability of effector cells (e.g., TCR-T cells) expressing an effective amount of a specific TCR to contact the antigen-positive target cells and kill the antigen-positive target cells, while exhibiting no or substantially no killing activity against the antigen-negative cells. In one embodiment, "specific killing activity" refers to the ability of TCR-T cells expressing an effective amount of a specific TCR to contact and kill KRAS G12 (e.g., KRAS G12V or KRAS G12D) mutant-positive target cells, while exhibiting no or substantially no killing activity against non-KRAS G12 (e.g., KRAS G12V or KRAS G12D) mutation-positive target cells. In one embodiment, "specific killing activity" is measured using methods described in the examples of the present disclosure. Specifically, TCR-T cells expressing the TCR or a binding fragment thereof of the present disclosure are used as effector cells, while non-TCR-transduced PBMCs after parallel amplification culture are used as control effector cells; KRAS G12-positive and HLA-A*11:01-positive cells are used as HLA-antigen peptide matched positive target cells; KRAS G12-negative and HLA-A*11:01-negative cells are used as HLA-antigen peptide mismatched negative target cells. Co-cultures are performed at various effector-to-target (E:T) ratios for 16 hours, followed by addition of luciferase substrate to detect surviving target cells. The proportion of lysed target cells is inferred from the number of surviving target cells.

In one embodiment, the term "substantially no" refers to that the response value of target antigen-negative cells to effector cells is equal to or lower than the background response value. In another embodiment, "substantially no" refers to that TCR-expressing cells (effector cells) show a lysis rate below 25%, below 24%, below 23%, below 22%, below 21%, below 20%, below 19%, below 18%, below 17%, below 16%, below 15%, below 14%, below 13%, below 12%, below 11%, below 10%, below 9%, below 8%, below 7%, below 6%, below 5%, below 4%, below 3%, below 2%, below 1%, below 0.8%, below 0.5%, or below 0.3% against KRAS G12 (e.g., KRAS G12V or KRAS G12D) mutation-negative target cells at a given E:T ratio (e.g., E:T is 9:1). The lysis rate may be determined using methods disclosed in the examples of the present disclosure.

In some embodiments, the antigen-binding protein provided in the present disclosure exhibits no alloreactivity to different HLA subtypes, i.e., does not induce alloreactivity.

In some embodiments, the antigen-binding protein provided in the present disclosure does not cause non-specific activation against cells from different tissue origins, i.e., the antigen-binding protein provided in the present disclosure has high antigen specificity.

In another aspect, the present disclosure provides a multispecific antibody comprising the antigen-binding protein of the present disclosure.

As used herein, a "multispecific antibody" refers to an antibody that can bind to at least one target antigen. For example, the multispecific antibody may comprise at least two antigen-binding proteins of the present disclosure; or the multispecific antibody may comprise at least one antigen-binding protein of the present disclosure and at least one TCR or a binding fragment thereof specifically binding to a different target antigen; or the multispecific antibody may comprise at least one antigen-binding protein of the present disclosure and at least one immunoeffector polypeptide.

In some embodiments, examples of TCRs or binding fragments thereof that specifically bind to other antigen targets may include, but are not limited to, TCRs or binding fragments thereof that specifically bind to HPV E6 or E7, TCRs or fragments thereof that specifically bind to gp100, or TCRs or fragments thereof that specifically bind to MAGE (e.g., MAGE-A1, MAGE-A4).

In some embodiments, the immunoeffector polypeptides are known. The immunoeffector polypeptides are molecules that directly or indirectly activates the humoral or cellular components of the immune system , for example, molecules that induce or stimulate an immune response through T cell activation". Examples of the immunoeffector polypeptides include but are not limited to antibodies and cytokines.

Examples of the antibodies include but are not limited to anti-CD2 antibody, anti-CD3 antibody, anti-CD4 antibody, anti-CD8 antibody, anti-CD44 antibody, anti-CD45RA antibody, anti-CD45RB antibody, anti-CD45RO antibody, anti-CD49a antibody, anti-CD49b antibody, anti-CD49c antibody, anti-CD49d antibody, anti-CD49e antibody, anti-CD49f antibody, anti-CD16 antibody, anti-CD28 antibody, and anti-IL-2R antibodies. In some embodiments, the antibody available is an scFv antibody, such as an anti-CD3 scFv antibody. Anti-CD3 antibodies include but are not limited to OKT3, UCHT-1, BMA031, and 12F6.

Examples of cytokines include but are not limited to IL-1, IL-1a, IL-3, IL-4, IL-5, IL-6, IL-7, IL-10, IL-11, IL-12, IL-13, IL-15, IL-21, IL-23, TGF-β, IFN-γ, and TNFα.

Other types of immunoeffector polypeptides may include but are not limited to viral proteins or viral peptides, or bacterial proteins or bacterial peptides.

In a preferred embodiment, the immunoeffector polypeptide is an antibody. In some embodiments, the antibody is an scFv, such as an anti-CD3 scFv. In some embodiments, the anti-CD3 antibodies include but are not limited to OKT3, UCHT-1, BMA031, and 12F6. In a particularly preferred embodiment, the antibody is linked to either the N-terminus or C-terminus of the antigen-binding protein. In some embodiments, the antigen-binding protein is a TCR or a fragment thereof, and in some embodiments, the Vα and Vβ of the TCR or a fragment thereof are comprised on different polypeptide chains (e.g., the TCR is an αβ TCR). In some embodiments, the Vα and Vβ of the TCR or a fragment thereof are comprised on a single polypeptide chain, e.g., a scTCR.

In another aspect, the present disclosure provides a nucleic acid encoding the antigen-binding protein or the multispecific antibody of the present disclosure.

In some embodiments, the nucleic acid comprises a nucleotide sequence having at least 80%, at least 85%, at least 90%, at least 95%, or 100% sequence identity to any of SEQ ID NOs: 87-91, and/or a nucleotide sequence having at least 80%, at least 85%, at least 90%, at least 91%, at least 92%, at least 93%, at least 94%, at least 95%, at least 96%, at least 97%, at least 98%, at least 99%, or 100% sequence identity to any of SEQ ID NOs: 92-96.

In some embodiments, the nucleic acid comprises a nucleotide sequence having at least 80%, at least 85%, at least 90%, at least 95%, or 100% sequence identity to SEQ ID NO: 87, and/or a nucleotide sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 92. In some embodiments, the nucleic acid comprises a nucleotide sequence of SEQ ID NO: 87, and/or a nucleotide sequence of SEQ ID NO: 92. In some embodiments, the nucleic acid comprises a nucleotide sequence of SEQ ID NO: 87 and a nucleotide sequence of SEQ ID NO: 92.

In some embodiments, the nucleic acid comprises a nucleotide sequence having at least 80%, at least 85%, at least 90%, at least 95%, or 100% sequence identity to SEQ ID NO: 88, and/or a nucleotide sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 93. In some embodiments, the nucleic acid comprises a nucleotide sequence of SEQ ID NO: 88, and/or a nucleotide sequence of SEQ ID NO: 93. In some embodiments, the nucleic acid comprises a nucleotide sequence of SEQ ID NO: 88 and a nucleotide sequence of SEQ ID NO: 93.

In some embodiments, the nucleic acid comprises a nucleotide sequence having at least 80%, at least 85%, at least 90%, at least 95%, or 100% sequence identity to SEQ ID NO: 89, and/or a nucleotide sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 94. In some embodiments, the nucleic acid comprises a nucleotide sequence of SEQ ID NO: 89, and/or a nucleotide sequence of SEQ ID NO: 94. In some embodiments, the nucleic acid comprises a nucleotide sequence of SEQ ID NO: 89 and a nucleotide sequence of SEQ ID NO: 94.

In some embodiments, the nucleic acid comprises a nucleotide sequence having at least 80%, at least 85%, at least 90%, at least 95%, or 100% sequence identity to SEQ ID NO: 90, and/or a nucleotide sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 95. In some embodiments, the nucleic acid comprises a nucleotide sequence of SEQ ID NO: 90, and/or a nucleotide sequence of SEQ ID NO: 95. In some embodiments, a nucleic acid comprises the nucleotide sequence of SEQ ID NO: 90 and a nucleotide sequence of SEQ ID NO: 95.

In some embodiments, the nucleic acid comprises a nucleotide sequence having at least 80%, at least 85%, at least 90%, at least 95%, or 100% sequence identity to SEQ ID NO: 91, and/or a nucleotide sequence having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NO: 96. In some embodiments, the nucleic acid comprises a nucleotide sequence of SEQ ID NO: 91, and/or a nucleotide sequence of SEQ ID NO: 96. In some embodiments, the nucleic acid comprises a nucleotide sequence of SEQ ID NO: 91 and a nucleotide sequence of SEQ ID NO: 96.

In some embodiments, the nucleic acid comprises nucleotide sequences having at least 80%, at least 85%, at least 90%, at least 95%, or 100% sequence identity to SEQ ID NOs: 87 and 97 respectively, and/or nucleotide sequences having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NOs: 92 and 98 respectively. In some embodiments, the nucleic acid comprises nucleotide sequences of SEQ ID NOs: 87 and 97, and/or nucleotide sequences of SEQ ID NOs: 92 and 98. In some embodiments, the nucleic acid comprises nucleotide sequences of SEQ ID NOs: 87 and 97 and nucleotide sequences of SEQ ID NOs: 92 and 98.

In some embodiments, the nucleic acid comprises nucleotide sequences having at least 80%, at least 85%, at least 90%, at least 95%, or 100% sequence identity to SEQ ID NOs: 88 and 97 respectively, and/or nucleotide sequences having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NOs: 93 and 98 respectively. In some embodiments, the nucleic acid comprises nucleotide sequences of SEQ ID NOs: 88 and 97, and/or the nucleotide sequences of SEQ ID NOs: 93 and 98. In some embodiments, the nucleic acid comprises nucleotide sequences of SEQ ID NOs: 88 and 97, and nucleotide sequences of SEQ ID NOs: 93 and 98.

In some embodiments, the nucleic acid comprises nucleotide sequences having at least 80%, at least 85%, at least 90%, at least 95%, or 100% sequence identity to SEQ ID NOs: 89 and 97 respectively, and/or nucleotide sequences having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NOs: 94 and 98 respectively. In some embodiments, the nucleic acid comprises nucleotide sequences of SEQ ID NOs: 89 and 97, and/or nucleotide sequences of SEQ ID NOs: 94 and 98. In some embodiments, the nucleic acid comprises nucleotide sequences of SEQ ID NOs: 89 and 97 and nucleotide sequences of SEQ ID NOs: 94 and 98.

In some embodiments, the nucleic acid comprises nucleotide sequences having at least 80%, at least 85%, at least 90%, at least 95%, or 100% sequence identity to SEQ ID NOs: 90 and 97 respectively, and/or nucleotide sequences having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NOs: 95 and 98 respectively. In some embodiments, the nucleic acid comprises nucleotide sequences of SEQ ID NOs: 90 and 97, and/or nucleotide sequences of SEQ ID NOs: 95 and 98. In some embodiments, the nucleic acid comprises nucleotide sequences of SEQ ID NOs: 90 and 97, and nucleotide sequences of SEQ ID NOs: 95 and 98.

In some embodiments, the nucleic acid comprises nucleotide sequences having at least 80%, at least 85%, at least 90%, at least 95%, or 100% sequence identity to SEQ ID NOs: 91 and 97 respectively, and/or nucleotide sequences having at least 80%, at least 85%, at least 90%, at least 91%, at least 92%, at least 93%, at least 94%, at least 95%, at least 96%, at least 97%, at least 98%, at least 99%, or 100% sequence identity to SEQ ID NOs: 96 and 98 respectively. In some embodiments, the nucleic acid comprises nucleotide sequences of SEQ ID NOs: 91 and 97, and/or nucleotide sequences of SEQ ID NOs: 96 and 98. In some embodiments, the nucleic acid comprises nucleotide sequences of SEQ ID NOs: 91 and 97, and nucleotide sequences of SEQ ID NOs: 96 and 98.

In another aspect, the present disclosure provides a vector comprising the nucleic acid of the present disclosure.

As used herein, the term "vector" is a nucleic acid molecule used as a vehicle for transferring (exogenous) genetic materials into a host cell in which the nucleic acid molecule as a vector can, for example, be replicated and/or expressed. The term "vector" encompasses, but is not limited to, plasmids, viral vectors (including retroviral vectors, lentiviral vectors, adenoviral vectors, cowpox virus vectors, polyomavirus vectors and adenovirus-associated vectors (AAVs)), phages, phasmids, cosmids and artificial chromosomes (including BACs and YACs). The vector itself is usually a nucleotide sequence, usually comprises a DNA sequence comprising an insert (transgene) and a larger sequence that serves as the "backbone" of the vector. The engineered vector usually comprises an origin for self-replication in the host cell (if stable expression of polynucleotides is desired), a selection marker and a restriction enzyme cleavage site (e.g., a polyclonal site, MCS). The vector may additionally comprise a promoter, a genetic marker, a reporter gene, a targeting sequence, and/or a protein purification tag. As known to those skilled in the art, a large number of suitable vectors are known to those skilled in the art, and many are commercially available. Examples of suitable vectors are provided in J. Sambrook et al, Molecular Cloning: a Laboratory Manual (4th ed.), Cold Spring Harbor Laboratory, Cold Spring Harbor Laboratory Press, New York (2012), which is incorporated herein by reference in their entirety.

In some embodiments, the vector is preferably selected from the group consisting of lentiviral vectors, retroviral vectors, plasmids, and DNA vectors, mRNA vectors, transposon-based vectors, and artificial chromosomes.

In another aspect, the present disclosure provides a cell comprising the antigen-binding protein, or multispecific antibody, nucleic acid, or vector of the present disclosure.

As used herein, the term "cell" refers to any type of cell capable of expressing the antigen binding protein of the present disclosure. The cell may be a eukaryotic cell, e.g., a vegetation (which does not have the potential to develop into a plant), animal, fungus, or algae cell, or may be a prokaryotic cell, e.g., a bacterium or protozoan cell. The cell may be a cultured cell or a primary cell, i.e. directly isolated from an organism, e.g. a human. The cell may be an adherent cell or suspension cell, i.e. cell grown in suspension. Suitable host cell is known in the art and includes, for example, a DH5α E. coli cell, Chinese hamster ovary cell, monkey VERO cell, COS cell, HEK293 cell, and the like. For the purpose of producing the antigen binding protein of the present disclosure, the cell is preferably a mammalian cell. Most preferably, the host cell is a human cell.

In some embodiments, the cell is selected from the group consisting of lymphocytes (e.g., T cells, NK cells), monocytes (e.g., PBMCs), and stem cells. As used herein, the term "stem cell" is a stem cell used for expressing the antigen binding protein (particularly the TCR) of the present disclosure. For example, a stem cell may be a lymphoid progenitor cell, an induced pluripotent stem cell (iPSC), or a hematopoietic stem cell (HSC). In some embodiments, the stem cell does not include embryonic stem cells obtained by destroying a human embryo, and/or does not include totipotent stem cells that are used to develop and form an individual animal. Transferring a gene to stem cells does not typically result in expression of a TCR on the surface of the cells, as stem cells does not express CD3 molecule on the surface. However, when stem cells differentiate into lymphoid precursors that migrate to thymus, expression of CD3 molecules will initiate expression of the introduced TCR molecules on the surface of thymocytes.

In some embodiments, the stem cell is a lymphoid progenitor cell or an induced pluripotent stem cell (iPSC).

In some embodiments, the cell is a T cell. The T cell may be any T cell, such as a cultured T cell, e.g., a primary T cell or a T cell from a cultured T cell line, e.g., Jurkat, SupTl, etc., or a T cell obtained from a mammal. If obtained from a mammal, the T cell may be obtained from many sources including, but not limited to, blood, bone marrow, lymph node, thymus or other tissues or fluids. The T cell may also be enriched or purified. Preferably, the T cell is a human T cell. More preferably, the T cell is a T cell isolated from human. The T cell may be any type of T cells and may be at any developmental stage, including but not limited to CD4+/CD8+ double positive T cells, CD4+ helper T cells, such as Th1 and Th2 cells, CD4+ T cells, CD8+ T cells (e.g., cytotoxic T cells), tumor infiltrating lymphocytes (TILs), memory T cells (e.g., central memory T cells and effector memory T cells), naive T cells, and the like. In some embodiments, the T cell does not express an endogenous TCR.

In another aspect, the present disclosure provides a method for preparing the cell of the present disclosure, comprising a step of transducing or transfecting a cell with the vector of the present disclosure.

As used herein, the term "transfection" is a process of intentionally introducing nucleic acid molecules or polynucleotides (including vectors) into target cells. One example is RNA transfection, that is, a process of introducing RNA (e.g., in vitro transcribed RNA, ivtRNA) into host cells. This term is primarily used for non-viral methods in eukaryotic cells. The term "transduction" is commonly used to describe virus-mediated transfer of nucleic acid molecules or polynucleotides. Transfection of animal cells typically involves the opening of transient pores or "holes" in the cell membrane to allow uptake of the materials. Transfection can be performed by using calcium phosphate, electroporation, cell extrusion, or by mixing cationic lipids with the materials to produce liposomes that are fused with the cell membrane and deposit their cargos into the interior. Exemplary techniques for transfecting eukaryotic host cells include lipid vesicle-mediated uptake, heat shock-mediated uptake, calcium phosphate-mediated transfection (calcium phosphate/DNA co-precipitation), microinjection, and electroporation.

In some embodiments, the method further comprises a step of amplifying and/or activating the cells prior to or after the transduction or transfection.

In another aspect, the present disclosure provides a conjugate comprising the antigen-binding protein and/or multispecific antibody of the present disclosure, and an active agent coupled or conjugated to the antigen binding protein.

In some embodiments, the active agent is selected from the group consisting of detectable markers, immunostimulatory molecules, and therapeutic agents. Preferably, the detectable marker is selected from the group consisting of biotins, streptavidin, enzymes or catalytically active fragments thereof, radionuclides, nanoparticles, paramagnetic metal ions, nucleic acid probes, contrast agents, and fluorogenic, phosphorescent or chemiluminescent molecules. Preferably, the immunostimulatory molecule is selected from the group consisting of cytokines (e.g. IL-2 and IFN-γ), chemokines (e.g. IL-8), platelet factors (e.g. platelet factor 4), and complement initiators. Preferably, the therapeutic agent is selected from the group consisting of immunomodulators, radioactive compounds, enzymes, chemotherapeutic agents and toxins. Other suitable therapeutic agents include small molecule cytotoxic agents, i.e. compounds with a molecular weight of less than 700 daltons having the ability to kill mammalian cells. Such compounds may also contain toxic metals having cytotoxic effects. In addition, it should be understood that these small molecule cytotoxic agents also include precursor drugs, i.e. compounds that decay or are transformed under physiological conditions to release cytotoxic agents. Examples of such agents include cisplatin, maytenin derivatives, rachelmycin, calicheamicin, docetaxel, etoposide, gemcitabine, isocyclic phosphorylamine, irinotecan, melphalan, mitoxantrone, sorfimer sodiumphotofrin II, temozolomide, topotecan, trimetreate glucuronate, auristatin E, vincristine, and doxorubicin; peptide cytotoxin, i.e., proteins or the fragments thereof having the ability to kill mammalian cells; e.g., ricin, diphtheria toxin, Pseudomonas bacterial exotoxin A, Dnase, and RNase; radionuclides, i.e., unstable isotopes of elements that emit one or more alpha or beta particles or gamma rays while decaying, e.g., iodine-131, rhenium-186, indium-111, yttrium-90, bismuth-210 and bismuth-213, actinium-225, and astatine-213; chelating agents which can be used to facilitate the binding of these radionuclides to molecules or multimers thereof; or heterologous protein domains, homologous protein domains, viral/bacterial protein domains, viral/bacterial peptides.

In another aspect, the present disclosure provides a composition comprising the antigen-binding protein, the multispecific antibody, the nucleic acid, the vector, or the cell of the present disclosure, preferably the composition further comprises a pharmaceutically acceptable carrier or excipient.

The term "composition" refers in particular to a composition suitable for the administration to humans. However, the term generally also encompasses a composition suitable for the administration to non-human animals. The composition and its components (i.e. active agents and optionally carriers or excipients) are preferably pharmaceutically acceptable, i.e. capable of eliciting the desired therapeutic effect without causing any undesired local or systemic effects in the recipient. The pharmaceutically acceptable compositions of the present invention may be, for example, sterile. Specifically, the term "pharmaceutically acceptable" may indicate approval by a regulatory agency or other recognized pharmacopoeia for use in animals, and more particularly in humans.

The term "excipient" includes fillers, adhesives, disintegrants, coating agents, adsorbents, anti-adhesives, flow aids, preservatives, antioxidants, flavoring agents, coloring agents, sweeteners, solvents, co-solvents, buffers, chelating agents, viscosity imparting agents, surfactants, diluents, wetting agents, carriers, diluents, preservatives, emulsifiers, stabilizers and tension regulators. It is known to those skilled in the art to select suitable excipients to prepare the composition of the present invention. Exemplary carriers for use in the composition of the present invention include saline, buffered saline, glucose, and water. Typically, the selection of a suitable excipient depends in particular on the active agent used, the disease to be treated and the desired dosage form of the composition.

Depending on the active agent employed (e.g. soluble TCR), the composition of the present disclosure may be formulated to various forms, such as solid, liquid, gaseous or lyophilized forms, and in particular may be in the form of ointments, creams, transdermal patches, gels, powders, tablets, solutions, aerosols, granules, pills, suspensions, emulsions, capsules, syrups, liquids, elixirs, infusions, tinctures or fluid extracts, or a form particularly suitable for the desired administration method. The processes of pharmaceutical production known to the present invention are shown in the 22nd edition of Remington's Pharmaceutical Sciences (Ed. MaackPublishing Co, Easton, Pa., 2012) and may include, for example, the conventional processes of mixing, lysing, granulating, sugar-coating, grinding, emulsifying, encapsulating, embedding, or lyophilizing. Compositions comprising, for example, the host cell or the soluble TCR as described herein are typically provided in liquid form and preferably comprise pharmaceutically acceptable buffering agents.

In some embodiments, the composition of the present disclosure further comprises a second therapeutic agent, preferably, the second therapeutic agent is selected from the group consisting of antibodies, chemotherapeutic agents, and small molecule drugs.

Preferred examples of the second therapeutic agent include known anti-cancer drugs such as cisplatin, maytenin derivatives, rachelmycin, calicheamicin, docetaxel, etoposide, gemcitabine, isocyclic phosphorylamine, irinotecan, melphalan, mitoxantrone, sorfimer sodiumphotofrin II, temozolomide, topotecan, trimetreate glucuronate, auristatin E, vincristine, and doxorubicin; and peptide cytotoxin, such as ricin, diphtheria toxin, Pseudomonas bacterial exotoxin A, Dnase, and RNase; radionuclides, e.g. iodine 131, rhenium 186, indium 111, iridium 90, bismuth 210 and 213, actinium 225 and astatine 213; prodrugs, e.g. antibody-directed enzyme prodrugs; immunostimulants, e.g. IL-2, chemokines e.g. IL-8, platelet factor 4; antibodies or fragments thereof, e.g. anti-CD3 antibodies or fragments thereof; complement initiators; heterologous protein domains, homologous protein domains, viral/bacterial protein domains, and viral/bacterial peptides.

In yet another aspect, the present disclosure provides a method for treating or preventing a KRAS mutation-positive disease and/or condition in a subject, comprising administering to the subject an effective amount of the antigen-binding protein of the present disclosure.

In another aspect, the present disclosure provides a method for treating or preventing a KRAS mutation-positive disease and/or condition in a subject, comprising administering to the subject an effective amount of the cell of the present disclosure.

As used herein, the term "treatment", "treat" or "treating" includes therapeutic or prophylactic treatment in a subject in need thereof. The term "therapeutic or prophylactic treatment" includes prophylactic treatment intended to completely prevent clinical and/or pathological manifestations or therapeutic treatment intended to improve or alleviate clinical and/or pathological manifestations. Thus, the term "treatment" also includes improvement or prevention of diseases.

As used herein, the term "effective amount" refers to an amount of a therapeutic agent sufficient to achieve such treatment or prevention when administered to a subject for the treatment or prevention of a disease. The "effective amount" may vary depending on the compound, the disease and its severity, and the age, weight, etc. of the subject to be treated. A "therapeutically effective amount" refers to an effective amount for the therapeutic treatment. A "prophylactic effective amount" refers to an effective amount for prophylactic treatment.

Therapeutic efficacy and toxicity can be determined by standard procedures such as ED50 (a dose that is therapeutically effective in 50% of the population) and LD50 (a dose that is lethal to 50% of the population) in such as cell cultures or experimental animals. The dose ratio between therapeutic and toxic effects is a therapeutic index and can be expressed as a ratio of ED50/LD50. The pharmaceutical composition exhibiting a large therapeutic index is preferred.

The exact dose of the antigen binding protein or cell administered can be determined by those skilled in the art using known techniques. A suitable dose provides a sufficient amount of the active agent of the invention and is preferably therapeutically effective, i.e., sufficient to elicit, for example, a therapeutic or prophylactic response in a subject or animal within a reasonable time period. For example, the dose of the antigen binding protein of the present invention such as TCR should be sufficient to bind to a cancer antigen or detect, treat, or prevent a cancer within a time period of about 2 hours or more, e.g., 12 hours to 24 hours or more from the time of administration (e.g., 1 month, 2 months, 3 months, 6 months, 12 months, 24 months, etc.). In some embodiments, the time period may even be longer. As known in the art, adjustments may be necessary depending on therapeutic purpose (such as alleviation of the acute onset of a disease), dosing route, time and frequency, time and frequency for administrating the formulation, age, weight, general health condition, gender, diet, severity of disease condition, drug combination, response sensitivity and tolerance/response to treatment.

A number of assays for determining the dose to be administered are known in the art. For the purposes of the present invention, the assays, including after administration of a given dose of T cells expressing the antigen binding protein (e.g., TCR) of the present disclosure to a group of mammals (each being administered a different dose of T cells), comparing the extent of target cell lysis or IFN-γ secretion achieved by such T cells, may be used to determine a starting dose to be administered to a mammal. The extent of target cell lysis or IFN-γ secretion achieved after administration of a given dose can be determined by methods known in the art. The dose of the antigen binding protein or the cell of the present disclosure is also determined by the presence, nature and extent of any adverse side effects that may accompany the administration of the antigen binding protein or the cell of the present disclosure. Typically, the dose of the antigen binding protein or the cell of the present disclosure to be administered to each individual patient is determined by the attending physician, in consideration of a variety of factors such as age, weight, general health, diet, gender, active agent to be administered, route of administration, and severity of the treated medical condition. In some embodiments of the treatment method of the present disclosure, the number of cells administered per infusion may vary, for example, from about 1 x 10⁶ to about 1 x 10¹² cells or more. In some embodiments, less than 1x 10⁶ cells may be administered.

It should be recognized that treatment may require a single administration or multiple administrations of a therapeutically effective dose of the active agent of the invention. For example, depending on the formulation, half-life and clearance of a particular composition, some compositions may be administered every 3 to 4 days, weekly, or every two weeks, or administered once over a period of one month.

The composition of the present disclosure may be suitable for the administration by a variety of routes. Typically, administration is accomplished parenterally. Parenteral delivery methods include topical, intra-arterial, intramuscular, subcutaneous, intramedullary, intrathecal, intracardiac, intravenous, intraperitoneal, intrauterine, intravaginal, sublingual, or intranasal administration.

The terms "subject" or "individual" or "animal" or "patient" are used interchangeably herein and refer to any subject in need of treatment, in particular mammalian subjects. In general, mammalian subjects include humans, non-human primates, dogs, cats, guinea pigs, rabbits, rats, mice, horses, cows, dairy cattle, etc. However, it is readily understood that it is particularly contemplated that the TCRs, nucleic acids, vectors, host cells, and pharmaceutical compositions provided herein are used to treat human subjects, particularly those are HLA-A*11 positive, such as HLA-A*11:01 positive.

In some embodiments of the treatment method of the present disclosure, the cells are autologous or allogeneic to the subject.

In some embodiments, the method comprises the following steps: (i) isolating a sample containing cells from the subject; (ii) transducing or transfecting the cells with the vector of the present disclosure; and (iii) administering the cells obtained in step (ii) to the subject. In some embodiments, the method further comprises a step of knocking out an endogenous TCR in the cells after step (i) and prior to step (ii). In some embodiments, the method further comprises administering a second therapeutic agent. Preferably the second therapeutic agent is selected from the group consisting of antibodies, chemotherapeutic agents and small molecule drugs. Preferred examples of the second therapeutic agent are as described above.

In another aspect, the present disclosure provides a method for detecting (e.g., diagnosing) a KRAS mutation-positive disease and/or condition in a subject, the method comprising: (i) contacting a sample obtained from the subject with the antigen-binding protein, the cell, or the conjugate of the present disclosure; and (ii) detecting the presence of a KRAS mutant antigen in the sample, wherein the presence of the KRAS mutant antigen is indicative of the KRAS mutation-positive disease and/or condition.

In some embodiments, the sample obtained from the subject may be a blood sample, urine sample, tissue sample, or cell sample. In certain embodiments, the method is performed in vitro. In some embodiments, the method comprises: (i) contacting the sample obtained from the subject with a conjugate of the present disclosure, wherein the conjugate comprises a detectable label; and (ii) detecting the presence of the KRAS mutant antigen in the sample by detecting the detectable label. Examples of detectable labels include, but are not limited to, biotins, streptavidin, enzymes or catalytically active fragments thereof, radionuclides, nanoparticles, paramagnetic metal ions, nucleic acid probes, contrast agents, and fluorogenic, phosphorescent, or chemiluminescent molecules; preferably enzymes or catalytically active fragments thereof, radionuclides, fluorogenic, phosphorescent, or chemiluminescent molecules.

In yet another aspect, the present disclosure provides a kit comprising the antigen-binding protein, multispecific antibody, or conjugate of the present disclosure.

In some embodiments, the kit is used to detect the presence of KRAS-positive epitopes in a sample to be tested.

In some embodiments, the kit is a kit used for detecting (e.g., diagnosing) KRAS mutation-positive diseases and/or conditions in a subject, and the kit comprises the antigen-binding protein or conjugate of the present disclosure.

In some embodiments, the conjugate comprises a detectable label. Examples of the detectable labels include, but are not limited to, biotins, streptavidin, enzymes or catalytically active fragments thereof, radionuclides, nanoparticles, paramagnetic metal ions, nucleic acid probes, contrast agents, and fluorogenic, phosphorescent, or chemiluminescent molecules; preferably enzymes or catalytically active fragments thereof, radionuclides, fluorogenic, phosphorescent, or chemiluminescent molecules. In some embodiments, the kit may also include an instruction manual describing how to use the kit.

In another aspect, the present disclosure provides the use of the antigen-binding protein, the multispecific antibody, the nucleic acid, the vector, the cell, the conjugate, or the composition of the present disclosure in the preparation of a medicament for the treatment or prevention of KRAS mutation-positive diseases and/or conditions in a subject.

In yet another aspect, the present disclosure provides the antigen-binding protein, the multispecific antibody, the nucleic acid, the vector, the cell, the conjugate, or the composition of the present disclosure for use in the treatment or prevention of KRAS mutation-positive diseases and/or conditions in a subject.

In another aspect, the present disclosure provides the use of the antigen-binding protein, the multispecific antibody, or the conjugate of the present disclosure in the preparation of a kit for detecting the presence of a positive epitope in a sample to be tested, wherein the positive epitope is a KRAS mutation-positive epitope.

In some embodiments, the present disclosure provides the use of the antigen-binding protein, the multispecific antibody, or the conjugate of the present disclosure in the preparation of a kit for detecting (e.g., diagnosing) KRAS mutation-positive diseases and/or conditions in a subject.

In yet another aspect, the present disclosure provides the antigen-binding protein, the multispecific antibody, or the conjugate of the present disclosure for use in detecting the presence of a positive epitope in a sample to be tested, wherein the positive epitope is a KRAS mutation-positive epitope, for example, for use in detecting (e.g., diagnosing) KRAS mutation-positive diseases and/or conditions in a subject.

In some embodiments of the uses of the present disclosure, the subject has an HLA-A*11 type, for example, HLA-A*11:01.

In embodiments of the present disclosure, the disease and/or condition may include KRAS mutation-positive diseases and/or conditions. In some embodiments, the KRAS mutation may include a G12 mutation epitope, for example, KRAS G12V or KRAS G12D. In some embodiments, the KRAS mutation-positive disease and/or condition may include tumors. In some embodiments, the tumor is selected from solid tumors and hematologic malignancies. More preferably, the tumor includes at least one of pancreatic cancer, colon cancer, rectal cancer, colorectal cancer, endometrial cancer, lung cancer, melanoma, prostate cancer, cholangiocarcinoma, cervical cancer, bladder cancer, liver cancer, and breast cancer.

The present invention is further elaborated by the following specific examples. It should be understood that these examples are intended to illustrate the invention only and are not intended to limit the scope of the invention. Experimental methods for which specific conditions are not indicated in the following examples are performed through conventional conditions in the art, e.g., those described in Sambrook and Russeii et al, Molecular Cloning: a Laboratory Manual (Third Edition) (2001), CSHL Press, or through those as recommended by the manufacturer. Unless otherwise stated, the experimental materials and reagents used in the following examples are commercially available.

### Example

### Example 1. Design and Identification of T Cell Epitopes for KRAS G12 Mutations

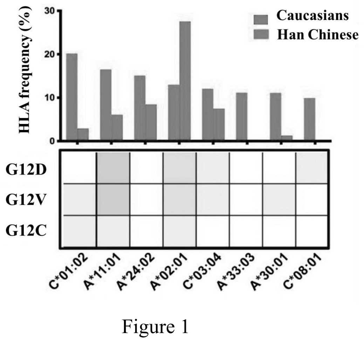

To expand the market potential for each KRAS mutation epitope-specific TCR product, multiple HLA genotypes with the highest distribution frequencies in the target market population were identified based on large-scale HLA genotyping data. On the basis of prioritizing high-frequency HLAs, T cell epitopes of KRAS mutant antigens presented by specific HLA genotypes were identified through bioinformatics analysis and experimental approaches. Systematic bioinformatics and immunological studies were conducted on antigen epitopes formed by mutations at the KRAS G12 position, confirming 12 distinct T cell epitopes. MHC tetramers (Tetramer) and ELISpot assays were developed to specifically detect T cells specific for these antigen epitopes. Subsequent drug development processes, such as TCR cloning, affinity optimization, and non-specific exclusion, were conducted for HLA target antigen epitopes (Figure 1).

### Example 2. Cloning of TCRs That Specifically Recognize KRAS G12 Presented by HLA-A*11:01

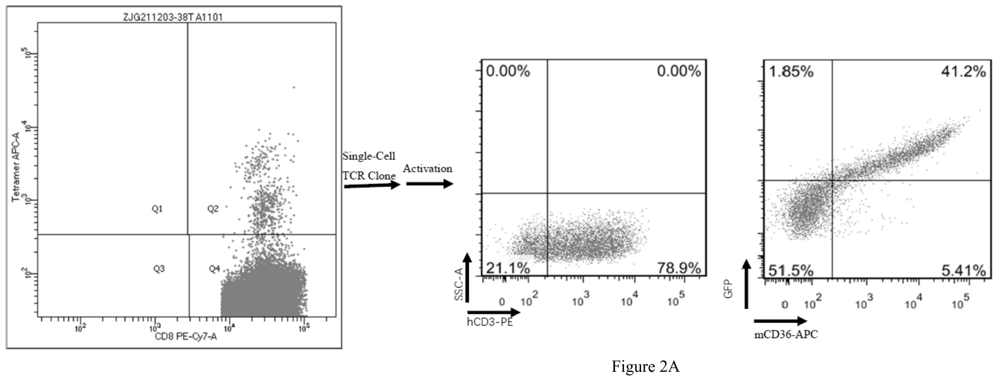

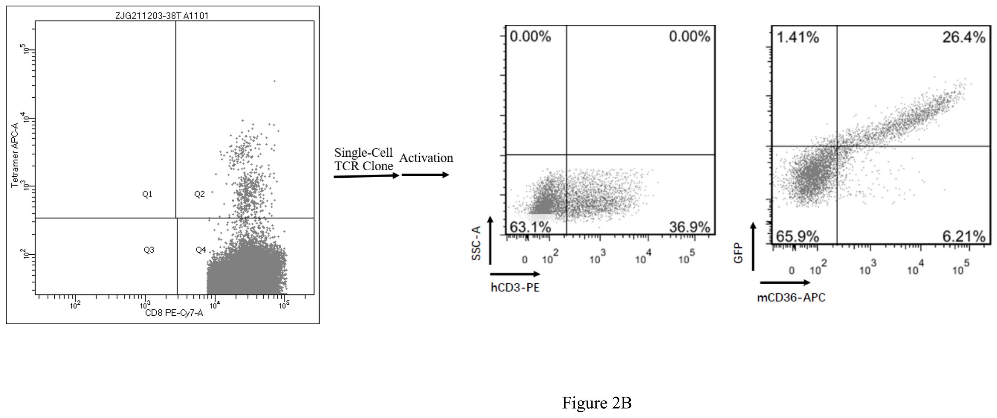

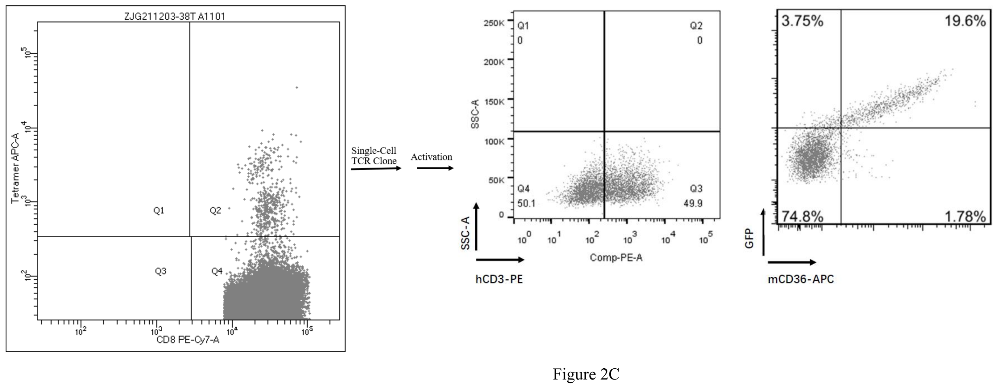

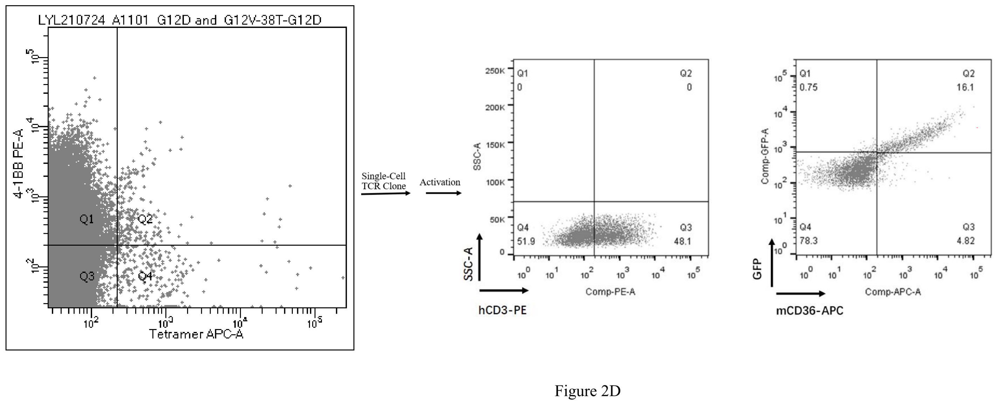

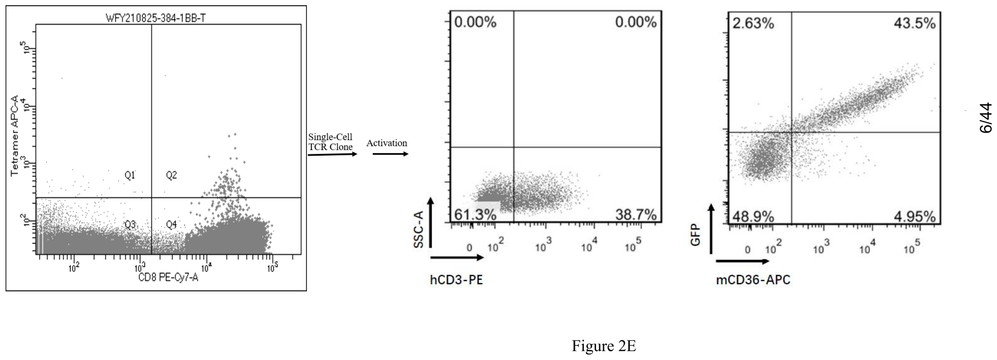

After obtaining ethical approval and informed consent from patients, post-operative tumor tissues from patients with KRAS G12 positive colorectal cancer patients (HLA-A*11:01) were obtained and tumor infiltrating lymphocytes (TILs) were cultured and amplified from the tumor tissues. After adequate amplification of TILs, CD8 and Tetramer (HLA-A*11:01, KRAS G12V 8-16, VVGA***V***GVGK, designated as KV1, SEQ ID NO: 101; HLA-A*11:01, KRAS G12V 7-16, VVVGA***V***GVGK, designated as KV2, SEQ ID NO: 102; HLA-A*11:01, KRAS G12D 8-16, VVGA***D***GVGK, designated as KD1, SEQ ID NO: 103; HLA-A*11:01, KRAS G12V 7-16, VVVGA***D***GVGK, designated as KD2, SEQ ID NO: 104) strongly-stained positive cells were sorted using flow cytometry. The sorted CD8+/tetramer+ cells were subjected to cellular mRNA reverse transcription and amplification to obtain the TCR α chain and β chain variable region (V region) genes. The obtained TCR α chain and β chain V region genes were further constructed into a lentiviral vector containing the TCR constant region (C region) gene. The cloned vector could express the complete TCR α chain and β chain, respectively (Figure 2). Each candidate TCR was paired and introduced into established T cell reporter cell lines, and antigen-specific activation assays were used to confirm antigen specificity and affinity of the TCR pairs. Through the cloning and validation strategy described above, five TCRs presented by HLA-A*11:01 were obtained, as shown in Figure 2.

These five TCRS specifically include:
TCR specific for KRAS G12V 7~16 epitope: KV A11-N02 (Figure 2A);
TCR specific for KRAS G12V 8~16 epitope: KVA11-N03 (Figure 2B);
TCR specific for KRAS G12V 7~16 epitope: KVA11-N04 (Figure 2C);
TCR specific for KRAS G12D 7~16 epitope: KDA11-N01 (Figure 2D);
TCR specific for KRAS G12D 7~16 epitope: KDA11-N02 (Figure 2E).

Amino acid sequences of the variable regions of the TCRs obtained above and their coding sequences are shown in Table 1. The CDR sequences of the α and β chain variable regions are shown in Table 2. The constant region sequences of the recombinant TCRs are shown in Table 3. Amino acid sequences of the α and β chains of the recombinant TCRs are shown in Table 4.

**Table 1. Amino Acid Sequences and Coding Sequences of TCR Variable Regions**

| TCR | | KVA11-N02 | KVA11-N02 | KVA11-N02 | KVA11-N02 | KVA11-N02 |
|---|---|---|---|---|---|---|
| Vα SEQ ID | amino acid sequences | 52 | 53 | 54 | 55 | 56 |
| NO: | nucleotide sequences | 87 | 88 | 89 | 90 | 91 |
| Vβ SEQ ID NO: | amino acid sequences | 57 | 58 | 59 | 60 | 61 |
| | nucleotide sequences | 92 | 93 | 94 | 95 | 96 |

**Table 2. CDR Sequences of the Variable Regions of TCR α and β Chains**

| TCR | | KVA11-N02 | KVA11-N03 | KVA11-N04 | KDA11-N01 | KDA11-N02 |
|---|---|---|---|---|---|---|
| α chain SEQ ID NO: | CDR1 | 27 | 28 | 29 | 30 | 31 |
| | CDR2 | 32 | 33 | 34 | 35 | 36 |
| | CDR3 | 22 | 23 | 24 | 25 | 26 |
| β chain SEQ ID NO: | CDR1 | 42 | 43 | 44 | 45 | 46 |
| | CDR2 | 47 | 48 | 49 | 50 | 51 |
| | CDR3 | 37 | 38 | 39 | 40 | 41 |

**Table 3. Constant Region Sequences of Recombinant TCR α and β Chains**

| α chain | |
|---|---|
| Amino acid sequence of WT human TRAC | SEQ ID NO:62 |
| Amino acid sequence of human TRAC variant 1 (T48C) | SEQ ID NO:63 |
| Amino acid sequence of human TRAC variant 2 (T48C + LVL mutation) | SEQ ID NO:64 |
| Amino acid sequence of WT murine TRAC | SEQ ID NO:65 |
| Amino acid sequence of murine TRAC variant 1 (T48C) | SEQ ID NO:105 |
| Amino acid sequence of murine TRAC variant 2 (T48C + LIV mutation) | SEQ ID NO:66 |
| Coding sequence of murine TRAC variant 2 (T48C + LIV mutation) | SEQ ID NO:97 |

| β chain | |
|---|---|
| Amino acid sequence of WT human TRBC1 | SEQ ID NO:67 |
| Amino acid sequence of human TRBC1 variant (S57C) | SEQ ID NO:68 |
| Amino acid sequence of WT human TRBC2 | SEQ ID NO:69 |
| Amino acid sequence of human TRBC2 variant (S57C) | SEQ ID NO:70 |
| Amino acid sequence of WT murine TRBC1 | SEQ ID NO:71 |
| Amino acid sequence of murine TRBC1 variant (S57C) | SEQ ID NO:72 |
| Coding sequence of murine TRBC1 variant (S57C) | SEQ ID NO:98 |
| Amino acid sequence of WT murine TRBC1' | SEQ ID NO:73 |
| Amino acid sequence of murine TRBC1' variant (S57C) | SEQ ID NO:74 |
| Amino acid sequence of WT murine TRBC2 | SEQ ID NO:75 |
| Amino acid sequence of murine TRBC2 variant (S57C) | SEQ ID NO:76 |

**Table 4. Amino Acid Sequences of Recombinant TCR α and β Chains**

| TCR | | KVA11-N02 | KVA11-N02 | KVA11-N02 | KVA11-N02 | KVA11-N02 |
|---|---|---|---|---|---|---|
| α chain SEQ ID NO: | amino acid sequences | 77 | 78 | 79 | 80 | 81 |
| β chain SEQ ID NO: | amino acid sequences | 82 | 83 | 84 | 85 | 86 |

The α chain and β chain variable regions of KVA11-N02, KVA11-N03, KVA11-N04, KDA11-N01, and KDA11-N02 were respectively fused to the murine constant regions (SEQ ID NO:66 (α chain) and SEQ ID NO:72 (β chain)) and constructed in tandem in β-T2A-α order into the lentiviral vector for subsequent use.

### Example 3.1 Affinity Testing

KVA11-N02, KVA11-N03, KVA11-N04, and KDA11-N01 were respectively introduced into Jurkat cells carrying an NFAT-GFP reporter gene (Jurkat-NFAT, in which the endogenous TCR had been knocked out), and co-incubated with T2 cells loaded with various concentrations of target antigen peptides (e.g., 1.0×10^-5 M, 1.0×10^-6 M, 1.0×10^-7 M, 1.0×10^-8 M, 1.0×10^-9 M, or 0 M). The activation level of the reporter gene in Jurkat cells was measured. The EC50 values of each TCR are shown in Table 5.

**Table 5**

| TCR | KVA11-N02 | KVA11-N03 | KVA11-N04 | KDA11-N01 |
|---|---|---|---|---|
| EC50 | 1.22*10^-7M | 2.11*10^-8M | 2.89*10^-8M | 7.65*10^-8M |

### Example 3.2 Affinity Testing

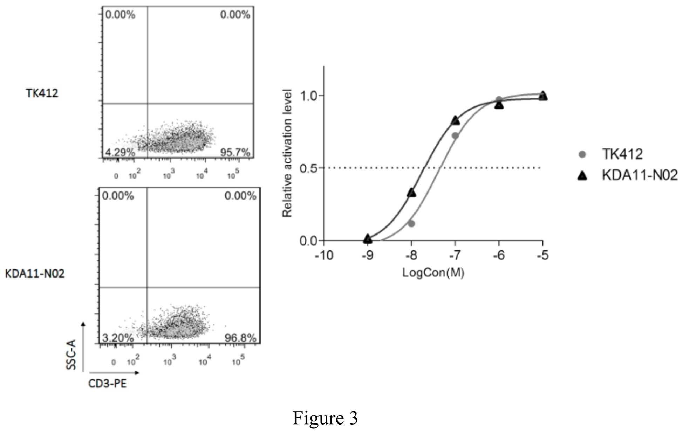

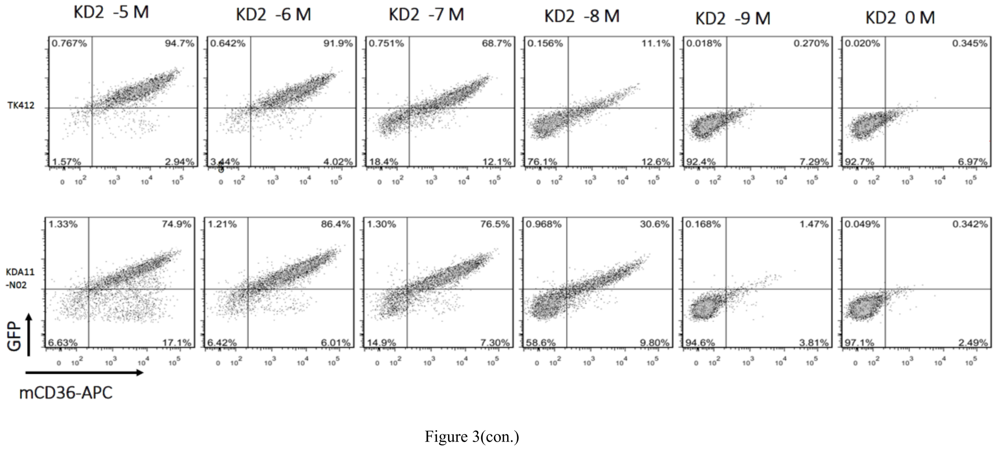

KDA11-N02 and TK412 (Identification of T-cell Receptors Targeting KRAS-Mutated Human Tumors, Qiong J. Wang, et al., Published OnlineFirst December 23, 2015; DOI: 10.1158/2326-6066.CIR-15-0188) were respectively introduced into Jurkat cells carrying an NFAT-GFP reporter gene (Jurkat-NFAT, in which the endogenous TCR had been knocked out), and co-incubated with T2 cells loaded with various concentrations of target antigen peptides (e.g., 1.0×10^-5 M, 1.0×10^-6 M, 1.0×10^-7 M, 1.0×10^-8 M, 1.0×10^-9 M, or 0 M). The activation level of the reporter gene in Jurkat cells was measured. As shown in Figure 3, the EC50 of KDA11-N02 was 1.73×10^-8 M, and the EC50 of TK412 in control group was 4.62×10^-8 M.

### Example 4 Membrane Stability

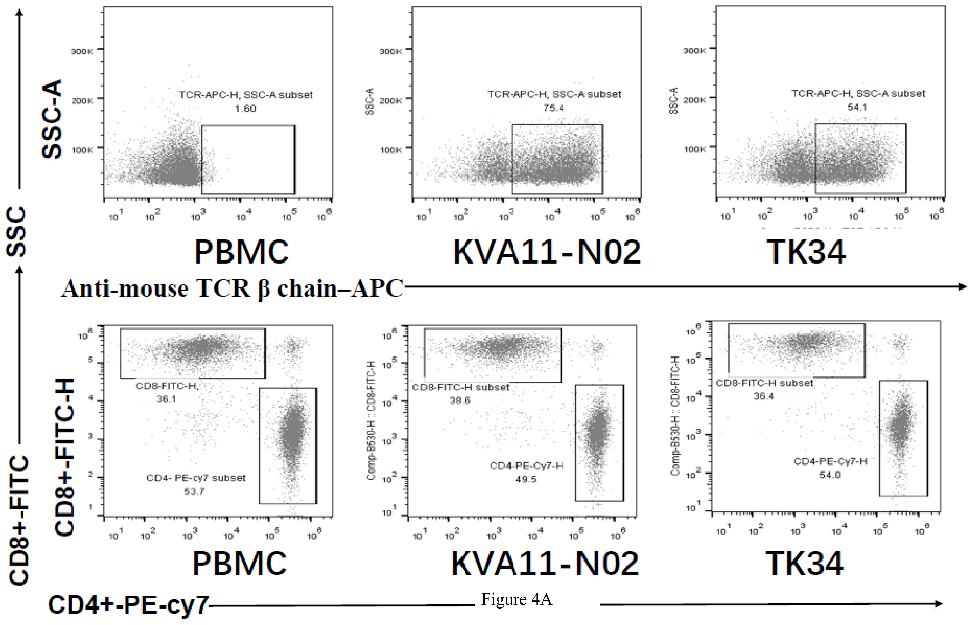

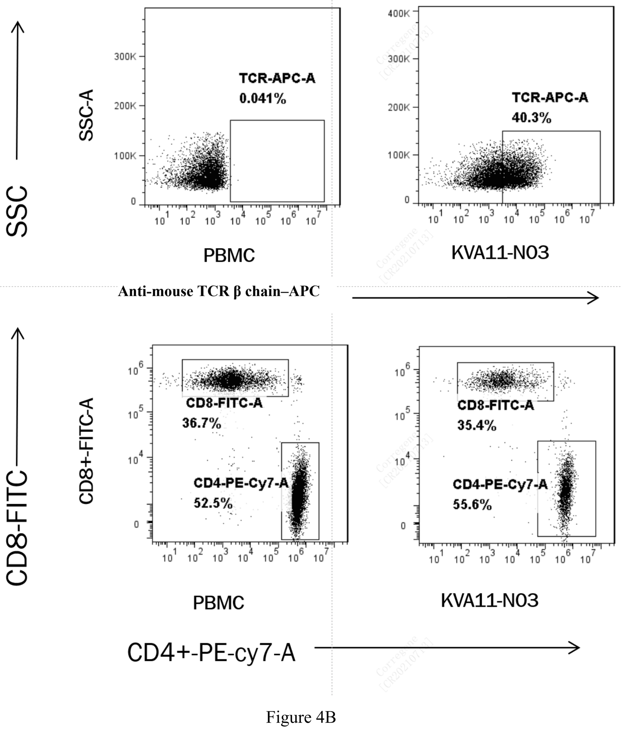

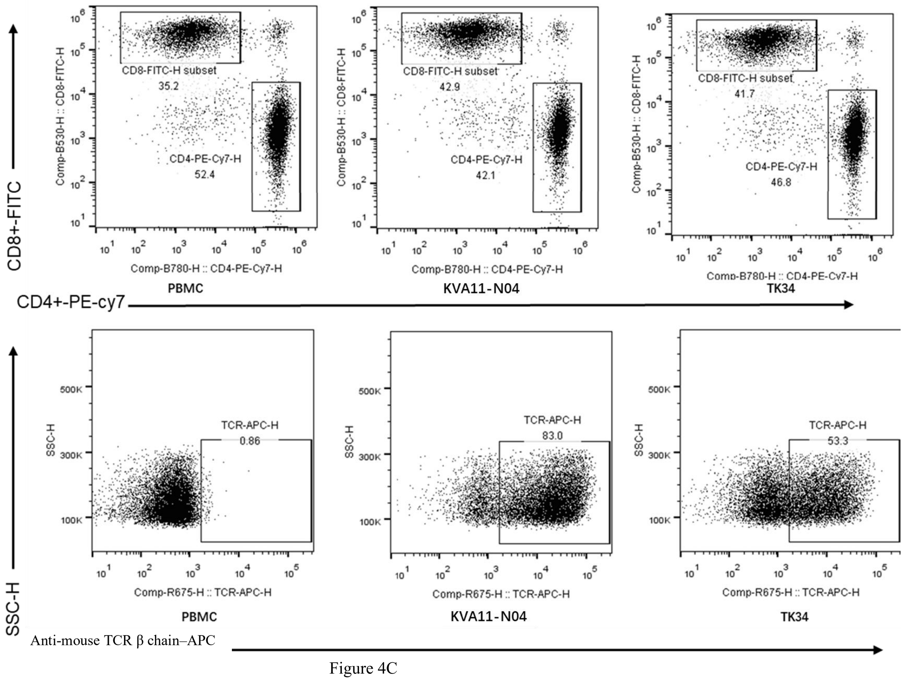

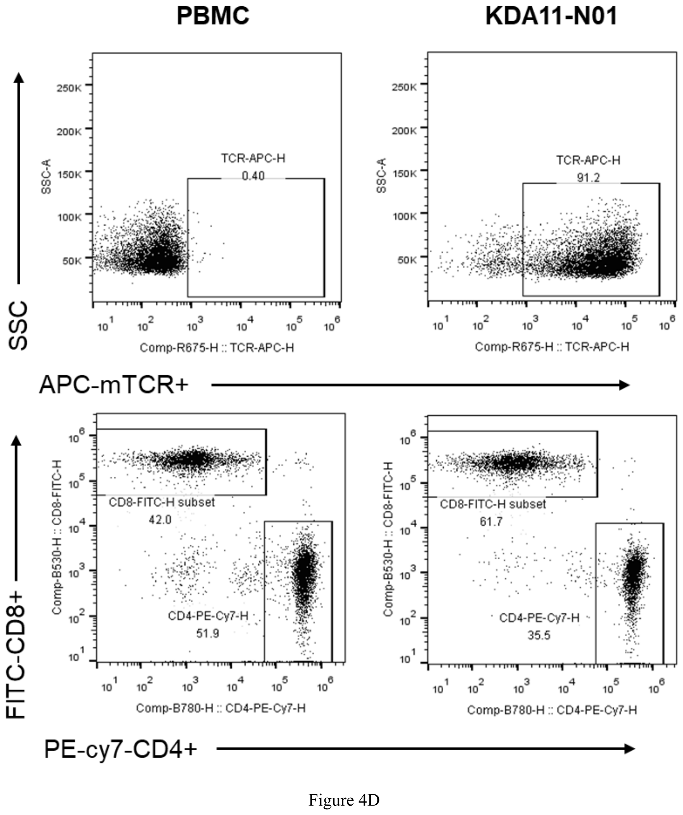

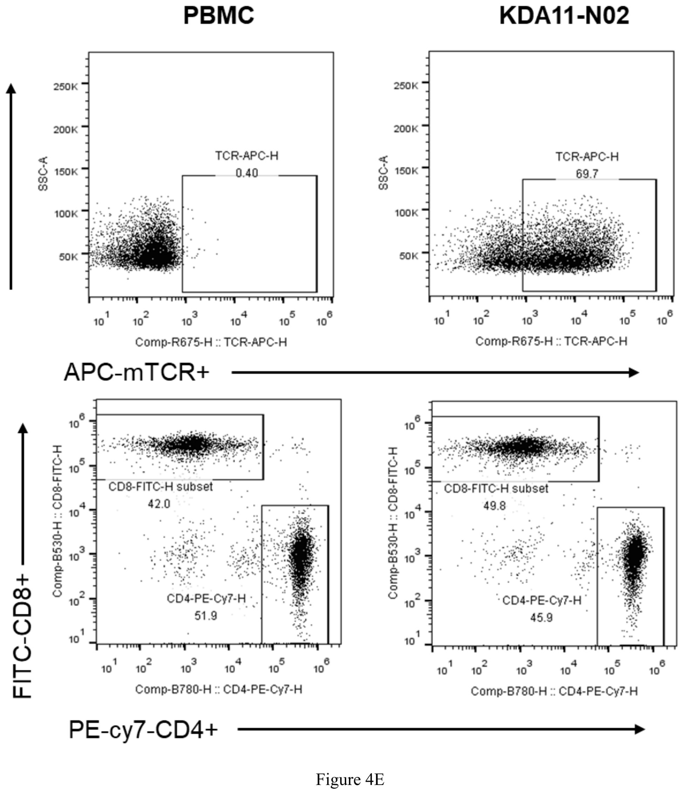

Equal amounts of TK34 (a TCR generated in mice against the same target as reported in literature, TRAV3-3*01/BV4*01, hereafter referred to as TK34, Identification of T-cell Receptors Targeting KRAS-Mutated Human Tumors, Qiong J. et al., Cancer Immunology Research)), KVA11-N02, KVA11-N03, KVA11-N04, KDA11-N01, and KDA11-N02 were lentivirally transduced into PBMCs and then cultured and expanded to Day 9. Anti-TCR β-APC antibody was used to stain and detect the expression rate of TCR-T; human CD3-PE, CD4-PE-Cy7, and CD8-FITC antibodies were used to stain and measure the CD4/CD8 ratio. Flow cytometry analysis showed:
1) KVA11-N02 (Figure 4A) and KVA11-N04 (Figure 4C): under the same transduction and culture conditions, the CD4/CD8 ratios expressed in cells after transduction of TK34, KVA11-N02, and KVA11-N04 were basically the same, and the TCR expression rates of KVA11-N02 and KVA11-N04 were significantly better than TK34, indicating that KVA11-N02 and KVA11-N04 protein had better membrane stability, suggesting that KVA11-N02 and KVA11-N04 potentially had better antigen reactivity and anti-tumor activity;
2) KVA11-N03 (Figure 4B): the TCR expression rate of KVA11-N03 was above 40% (Figure 4B), showing that the KVA11-N03 protein molecule could be stably expressed on the membrane;
3) KDA11-N01 (Figure 4D): the TCR expression rate of KDA11-N01 reached 90%, and the mean fluorescence intensity of TCR expression reached 25851, suggesting that the KDA11-N01 TCR protein molecule had good membrane stability, indicating that KDA11-N01 potentially had better antigen reactivity and anti-tumor activity;
4) KDA11-N02 (Figure 4E): the TCR expression rate of KDA11-N02 was about 70%, and the mean fluorescence intensity of TCR expression reached 6719, suggesting that the KDA11-N02 TCR protein had good membrane stability, indicating that KDA11-N02 potentially had better antigen reactivity and anti-tumor activity.

### Example 5 The TCRs of the Present Disclosure Specifically Kill Tumor Antigens and the Killing Activity Is Positively Correlated with Antigen Expression Levels

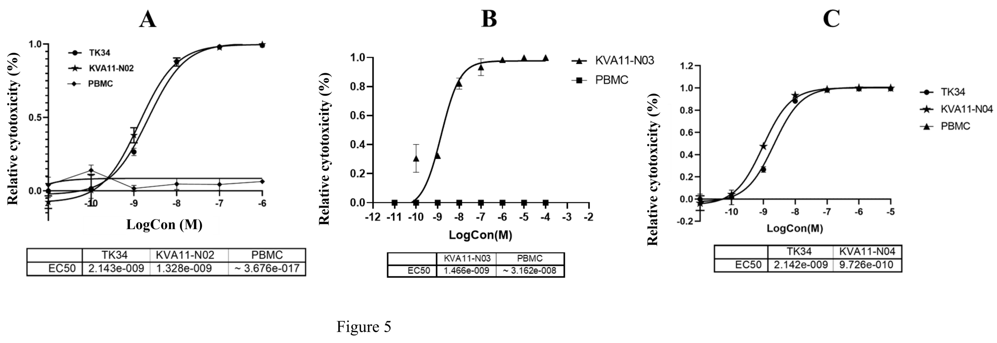

KVA11-N02 and KVA11-N04 can specifically recognize the KRAS G12V VVVGAVGVGK epitope peptide, while KVA11-N03 and TK34 can specifically recognize the KRAS G12V VVGAVGVGK epitope peptide. TCR-T cells expressing KVA11-N02, KVA11-N03, KVA11-N04, or TK34 were used as effector cells, and PBMCs that were not transduced with TCR were cultured and expanded in parallel and used as a control of the effector cells; T2KO-A1101 (HLA-A*11:01) cells loaded with KRAS G12V 7~16 epitope peptide or 8~16 epitope peptide at concentrations of 10^-5 M, 10^-6 M, 10^-7 M, 10^-8 M, 10^-9 M, 10^-10 M, or 10^-11 M were used as HLA antigen peptide matched positive target cells; T2KO-A*1101 cells not loaded with peptide were used as HLA matched but antigen peptide mismatched negative target cells; all T2 cells stably express the luciferase gene. Effector cells were incubated with target cells of different antigen concentrations at an effector-to-target (E:T) ratio of 9:1 for 20 hours, and then luciferase substrate was added to detect surviving target cells. The proportion of target cells killed was calculated based on the remaining target cells. The EC50 value of each TCR-T was calculated based on the killing ratio of effector cells against target cells loaded with different concentrations of antigen peptides, whereby to plot EC50 curves. The results showed: 1) TK34 and KVA11-N02 exhibited significant killing activity against the G12V7~16 epitope peptide presented by HLA-A*11:01, which increased with antigen expression levels and eventually reached a plateau, approaching 100% killing. It can be obviously observed that, KVA11-N02 showed stronger killing ability than TK34 against T2 cells with lower antigen density. EC50 curves is plotted based on killing ratios, and the results show that:
1) the EC50 values of TK34 and KVA11-N02 were 2.14E-9 and 1.32E-9, respectively, with KVA11-N02 showing stronger killing activity (Figure 5A);
2) KVA11-N03 exhibited significant killing activity against the G12V8~16 epitope peptide presented by HLA-A*11:01, which increased with antigen expression levels and eventually reached a plateau, approaching 100% killing. The EC50 curve plotted from the killing ratios showed an EC50 of 1.466E-9 for KVA11-N03, indicating that KVA11-N03 exhibited strong killing activity (Figure 5B);
3) TK34 and KVA11-N04 exhibited significant killing activity against both the G12V_{8∼16} and G12V_{7∼16} epitope peptides presented by HLAA11:01, which increased with antigen expression levels and eventually reached a plateau, approaching 100% killing. It can be obviously observed that, KVA11-N04 showed stronger killing ability than TK34 against T2 cells with lower antigen density. Based on the EC50 curves plotted from killing ratios, the EC50 values of TK34 and KVA11-N04 were 2.14E-9 and 9.726E-10, respectively, with KVA11-N04 showing stronger killing activity (Figure 5C).

### Example 6.1 The TCR-T Cells of the Present Disclosure Can Specifically Recognize Tumor Antigens and Release Cytokines, and the Amount of Cytokine Secreted Is Positively Correlated with Antigen Expression Level

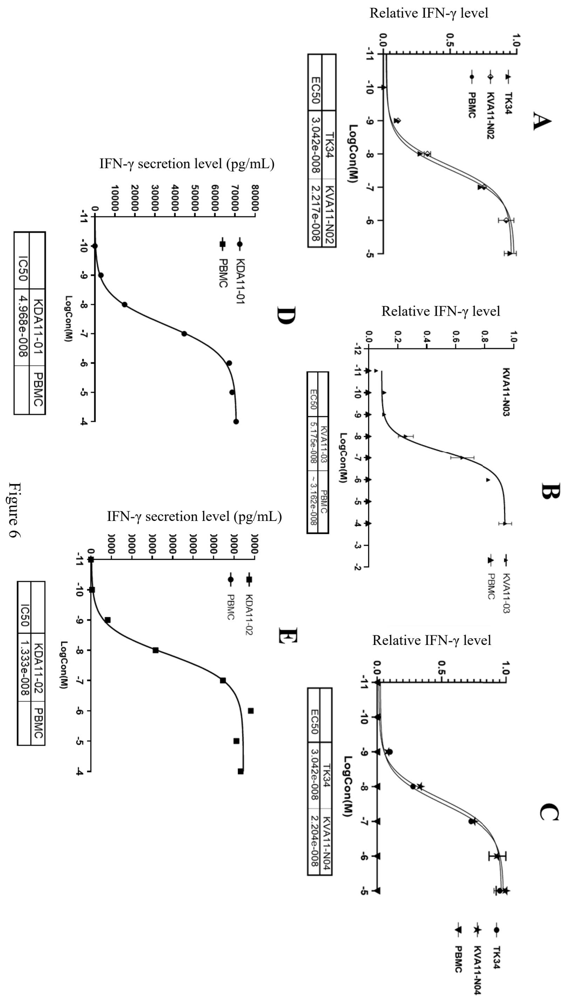

KVA11-N02 and KVA11-N04 can specifically recognize the KRAS G12V VVVGAVGVGK epitope peptide, KVA11-N03 can specifically recognize the KRAS G12V VVGAVGVGK epitope peptide, and TK34 can specifically recognize the KRAS G12V VVGAVGVGK epitope peptide. TCR-T cells expressing KVA11-N02, KVA11-N03, KVA11-N04 or TK34 were used as effector cells, and PBMCs that were not transduced with TCR were cultured and expanded in parallel as a control of the effector cells. T2KO-A1101 (HLA-A*11:01) cells loaded with KRAS G12V peptide at concentrations of 10^{^-5} M, 10^{^-6} M, 10^{^-7} M, 10^{^-8} M, 10^{^-9} M, 10^{^-10} M, or 10^{^-11} M were used as HLA antigen peptide matched positive target cells, and T2KO-A1101 cells not loaded with peptides were used as HLA matched but antigen peptide mismatched negative target cells. Effector cells were incubated with target cells of different antigen concentrations at an effector-to-target (E:T) ratio of 1:1 for 20 hours. IFN-γ secretion in the supernatant was measured using IFN-γ ELISA Kits (ExCell Bio, Cat# EH008-96). EC50 values for each TCR-T were calculated based on the amount of IFN-γ secreted upon activation, and EC50 curves were plotted. The results showed:
1) TK34 and KVA11-N02 exhibited strong specific responses to the G12V_{8∼16/7∼16} epitope peptides presented by HLA-A*11:01, which increased with antigen expression levels. It can be obviously observed that, compared with TK34, KVA11-N02 showed stronger cytokine secretion capacity against T2 cells with lower antigen density. According to EC50 curves plotted based on IFN-γ secretion, the EC50 values for TK34 and KVA11-N02 were 3.024E-8 and 2.217E-8, respectively, indicating that KVA11-N02 exhibited stronger specific response (Figure 6A);
2) KVA11-N03 exhibited strong specific response to the G12V_{8∼16} epitope peptide presented by HLA-A*11:01, which increased with antigen expression levels. According to EC50 curves plotted based on IFN-γ secretion, the EC50 value of KVA1 1-N03 was 5.175E-8, indicating that KVA11-N03 exhibited strong specific response (Figure 6B);
3) TK34 and KVA11-N04 exhibited strong specific responses to G12V_{8∼16/7∼16} epitope peptides presented by HLA-A*11:01, which increased with antigen expression levels. It can be obviously observed that, compared with TK34, KVA11-N04 showed stronger cytokine secretion against T2 cells with lower antigen density. According to EC50 curves plotted based on IFN-γ secretion, the EC50 values of TK34 and KVA11-N04 were 3.042E-8 and 2.204E-8, respectively, indicating that KVA11-N04 exhibited stronger specific response (Figure 6C).

### Example 6.2 The TCR-T Cells of the Present Disclosure Can Specifically Recognize Tumor Antigens and Release Cytokines, and the Amount of Cytokine Secreted Is Positively Correlated with Antigen Expression Level

KDA11-N01 and KDA11-N02 can specifically recognize the KRAS G12D_{7∼16} epitope peptide VVVGADGVGK presented by HLA-A*11:01. TCR-T cells expressing KDA11-N01 were used as effector cells, and PBMCs that were not transduced with TCR were cultured and expanded in parallel as a control of the effector cells. T2KO-A1101 (HLA-A*11:01) cells loaded with KRAS G12D peptide at concentrations of 10^{^-5} M, 10^{^-6} M, 10^{^-7} M, 10^{^-8} M, 10^{^-9} M, 10^{^-10} M, or 10^{^-11} M were used as HLA antigen peptide matched positive target cells, and T2KO-A*1101 cells not loaded with peptides were used as HLA matched but antigen peptide mismatched negative target cells. Effector cells were co-incubated with target cells of different antigen concentrations at an effector-to-target (E:T) ratio of 1:1 for 20 hours. IFN-γ secretion in the supernatant was measured using IFN-γ ELISA Kits (ExCell Bio, Cat# EH008-96). EC50 values for each TCR-T were calculated based on the amount of IFN-γ secreted upon activation, and EC50 curves were plotted. The results showed:
1) KDA11-N01 exhibited strong specific response to the G12D_{7∼16} epitope peptide presented by HLA-A*11:01, which increased with antigen expression levels. It can be obviously observed that, KDA11-N01 showed strong cytokine secretion capacity against T2 cells with lower antigen density. According to EC50 curve plotted based on IFN-γ secretion, the EC50 value for KDA11-N01 was 4.96E-8, indicating that KDA11-N01 exhibited strong specific response (Figure 6D);
2) KDA11-N02 exhibited strong specific response to the G12D_{7∼16} epitope peptide presented by HLA-A*11:01, which increased with antigen expression levels. It can be obviously observed that, KDA11-N02 showed strong cytokine secretion capacity against T2 cells with lower antigen density. According to EC50 curve plotted based on IFN-γ secretion, the EC50 value for KDA11-N02 was 1.33E-8, indicating that KDA11-N02 exhibited strong specific response (Figure 6E).

### Example 7.1 The TCR-T Cells of the Present Disclosure Exhibit Efficient and Specific Killing Activity Against Antigen-Positive Tumor Cells

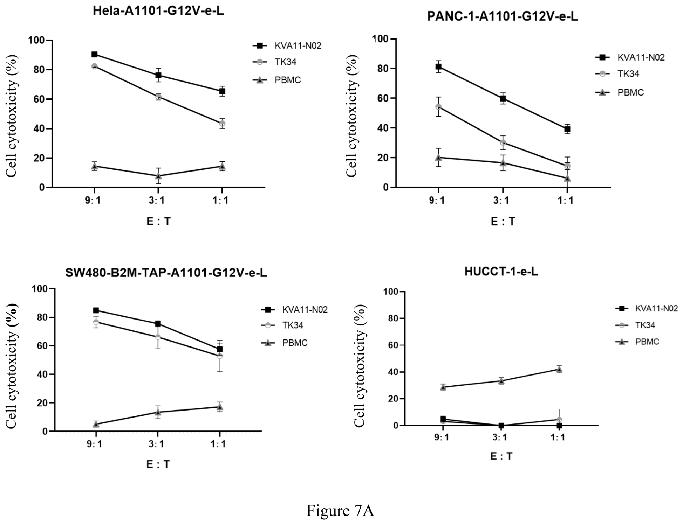

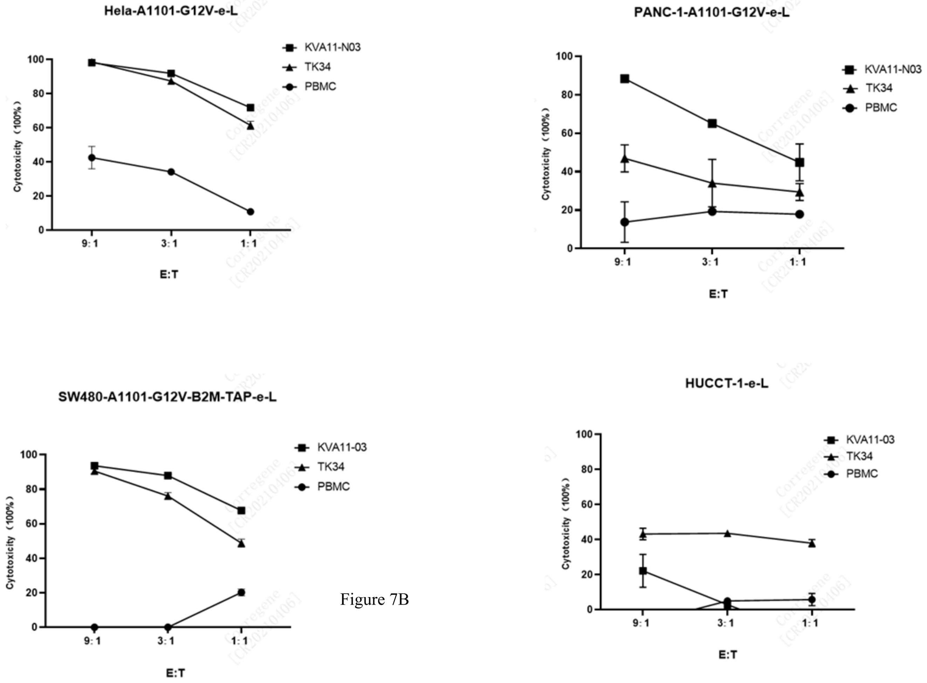

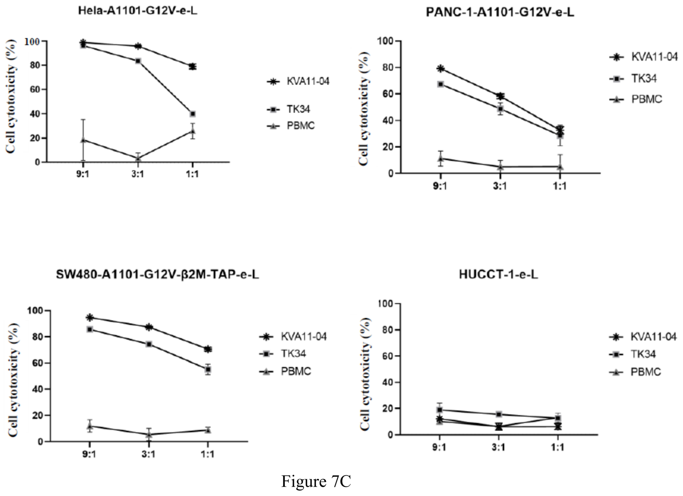

KVA11-N02 and KVA11-N04 can specifically recognize the KRAS G12V VVVGAVGVGK epitope peptide, and KVA11-N03 and TK34 can both specifically recognize the KRAS G12V_{8∼16} VVGAVGVGK epitope peptide. TCR-T cells expressing KVA11-N02 or TK34 were used as effector cells, and PBMCs that were not transduced with TCR were cultured and expanded in parallel as a control of the effector cells. SW480-B2M-A1101-G12V (KRAS G12V-positive, HLA-A*11:01 positive), Hela-A1101-G12V (KRAS G12V positive, HLA-A*11:01 positive), and PANC-1-A1101-G12V (KRAS G12V positive, HLA-A*11:01 positive) were used as HLA antigen peptide matched positive target cells; HUCCT-1 (KRAS G12V negative, HLA-A*11:01 positive) was used as HLA matched but antigen peptide mismatched negative target cell. All target cells stably expressed luciferase gene. Effector and target cells were co-incubated at effector-to-target (E:T) ratios shown in the figure for 16 hours, and the surviving target cells were measured by adding luciferase substrate. The proportion of killed target cells was calculated based on the remaining target cells. The results showed:
1) Both TK34 and KVA11-N02 exhibited clear killing activity, but it can be obviously observed that, compared with TK34, KVA11-N02 showed stronger killing activity against adherent positive target cells SW480, Hela, and PANC-1, and in a dose-dependent manner. No obvious cytotoxicity was observed against the negative target cell HUCCT-1 (Figure 7A), indicating that KVA11-N02 exhibited better killing activity and specificity;
2) Both TK34 and KVA11-N03 exhibited clear killing activity in a dose-dependent manner, and no obvious cytotoxicity was observed against the negative target cell HUCCT-1, indicating that KVA11-N03 exhibited better killing activity and specificity, and significantly better killing function than TK34 (Figure 7B);
3) Compared with TK34, KVA11-N04 showed stronger killing activity against adherent positive target cells SW480, Hela, and PANC-1, in a dose-dependent manner, and no obvious cytotoxicity was observed against the negative target cell HUCCT-1 (Figure 7C), indicating that KVA11-N04 exhibited better killing activity and specificity.

### Example 7.2 The TCR-T cells of the Present Disclosure Exhibit High-Efficiency and Specific Killing Activity Against Antigen-Positive Tumor Cells

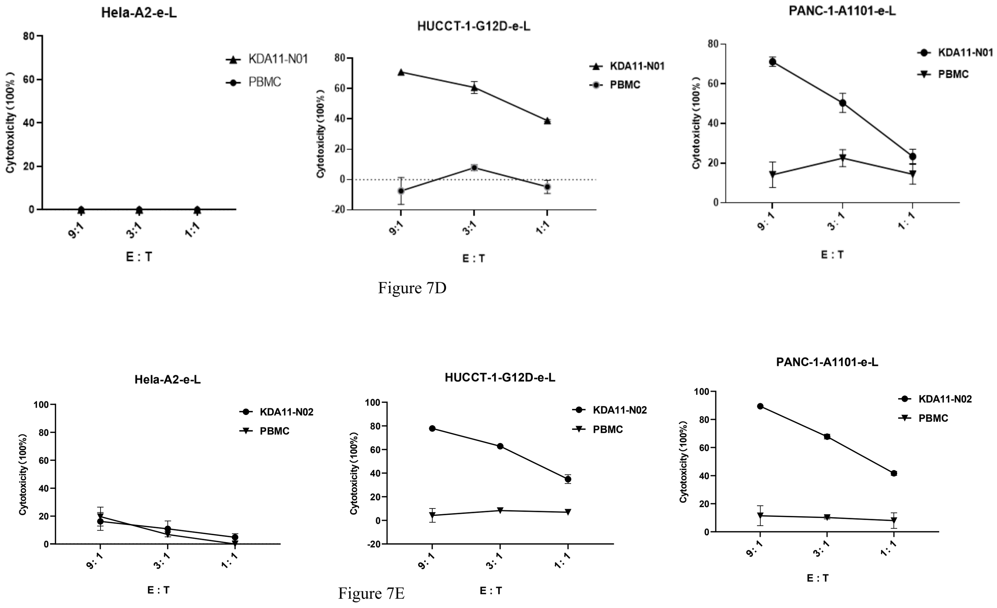

TCR-T cells expressing KDA11-N01 were used as effector cells, and PBMCs that were not transduced with TCR were cultured and expanded in parallel as a control of the effector cells. PANC-1-A1101 (KRAS G12D positive, HLA-A*11:01 positive) and HUCCT-1-A1101-G12D (KRAS G12D positive, HLA-A*11:01 positive) were used as HLA antigen peptide matched positive target cells. Hela-A2 (KRAS G12D negative, HLA-A*11:01 negative) was used as HLA antigen peptide mismatched negative target cell. All target cells stably expressed luciferase gene. Effector and target cells were co-cultured at effector-to-target (E:T) ratios shown in the figure for 16 hours, and the surviving target cells were measured by adding luciferase substrate. The proportion of killed target cells was calculated based on the remaining target cells. The results showed:
1. KDA11-N01 exhibited clear killing activity against several positive target cells, and in a dose-dependent manner. But it can be obviously observed that, KDA11-N01 showed stronger cytotoxicity against adherent positive target cells PANC-1, possibly due to higher antigen expression; no obvious cytotoxicity was observed against the negative target cell Hela (Fig. 7D), indicating that KDA11-N01 exhibited good cytotoxicity and specificity;
2. KDA11-N02 exhibited clear killing activity against several positive target cells in a dose-dependent manner. But it can be obviously observed that, KDA11-N02 showed stronger cytotoxicity against the adherent positive target cell PANC-1, possibly due to higher antigen expression; no obvious cytotoxicity was observed against the negative target cell Hela (Fig. 7E), indicating that KDA11-N02 exhibited good cytotoxicity and specificity.

### Example 8.1 TCR-T Cells of the Present Disclosure Exhibit Specific Responses to Antigen-Positive Target Cells: IFN-Γ Secretion Assay

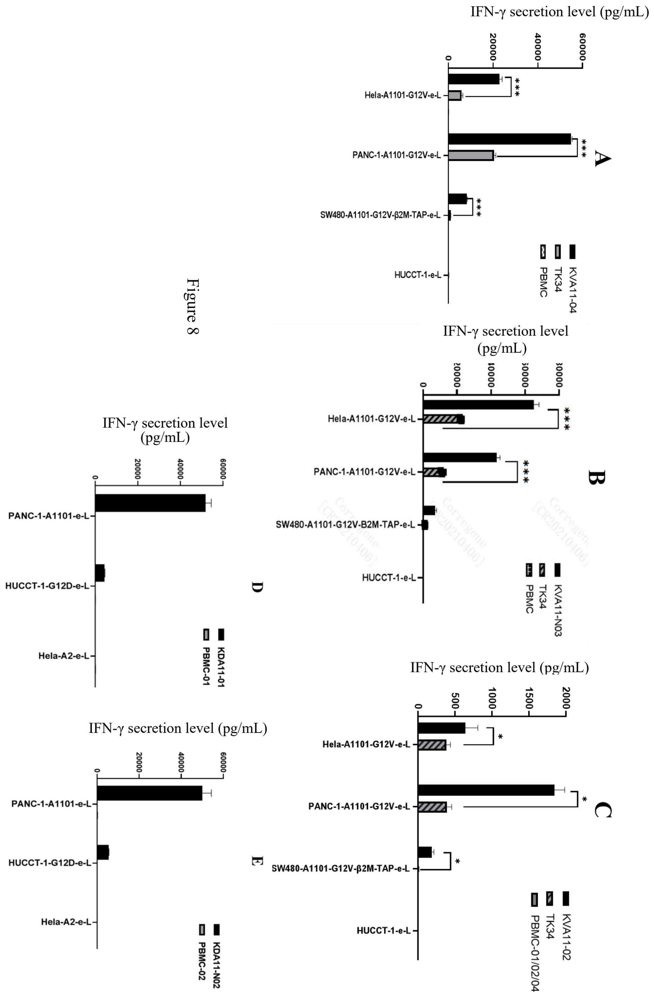

TCR-T cells expressing KVA11-N02, KVA11-N03, KVA11-N04, or TK34 were used as effector cells, and PBMCs that were not transduced with TCR were cultured and expanded in parallel as a control of the effector cells. SW480-B2M-A1101-G12V (KRAS G12V positive, HLA-A*11:01 positive), Hela-A1101-G12V (KRAS G12V positive, HLA-A*11:01 positive), and PANC-1-A1101-G12V (KRAS G12V positive, HLA-A*11:01 positive) were used as HLA antigen peptide matched positive target cells. HUCCT-1 (KRAS G12V negative, HLA-A*11:01 positive) was used as HLA matched but antigen peptide mismatched negative target cell. TCR-T positive effector cells were provided at 1E5 and co-cultured with different target cells at an effector-to-target ratio of 1:1 for 20 hours. IFN-γ secretion in the supernatant was detected using IFN-γ ELISA Kits (ExCell Bio, Cat# EH008-96). The results showed:
1. KVA11-N02 can be activated by adherent cells Hela-A1101-G12V, PANC-1-A1101-G12V, and SW480-A1101-B2M-TAP-G12V, and secreted IFN-γ; KVA11-N02 cannot be activated by antigen-negative adherent cells HUCCT-1. Compared with TK34, KVA11-N02 secreted significantly higher levels of IFN-γ and responded more strongly to KRAS G12V-positive cell lines (Fig. 8A), indicating that the specificity of KVA11-N02 is superior to that of TK34.
2. Both KVA11-N03 and TK34 can be activated by adherent cells Hela-A1101-G12V, PANC-1-A1101-G12V, and SW480-A1101-B2M-TAP-G12V, and secreted IFN-γ; KVA11-N03 can not be activated by antigen-negative adherent cells HUCCT-1, indicating that KVA11-N03 exhibited good specificity. Moreover, KVA11-N03 secreted significantly higher IFN-γ than TK34 (Fig. 8B).
3. KVA11-N04 can be activated by adherent cells Hela-A1101-G12V, PANC-1-A1101-G12V, and SW480-A1101-B2M-TAP-G12V, and secreted IFN-γ; KVA11-N04 can not be activated by antigen-negative adherent cells HUCCT-1. Compared with TK34, KVA11-N04 secreted significantly higher levels of IFN-γ and responded more strongly to KRAS G12V-positive cell lines (Fig. 8C), indicating that the specificity of KVA11-N04 is superior to that of TK34.

### Example 8.2 The TCR-T Cells of the Present Disclosure Exhibit Specific Responses to Antigen-Positive Target Cells: IFN-Γ Secretion Assay

TCR-T cells expressing KDA11-N01 and KDA11-N02 were used as effector cells, and PBMCs that were not transduced with TCR were cultured and expanded in parallel as a control of the effector cells. PANC-1-A1101 (KRAS G12D positive, HLA-A*11:01 positive), HPAF-II-A1101 (KRAS G12D positive, HLA-A*11:01 positive), and HUCCT-1-A1101-G12D (KRAS G12D positive, HLA-A*11:01 positive) were used as HLA antigen peptide matched positive target cells; Hela-A2 (KRAS G12D negative, HLA-A*11:01 negative) was used as HLA matched but antigen peptide mismatched negative target cell. TCR-T positive effector cells were provided at 1E5 and co-cultured with different target cells at an effector-to-target ratio of 1:1 for 20 hours. IFN-γ secretion in the supernatant was detected using IFN-γ ELISA Kits (ExCell Bio, Cat# EH008-96). The results showed:
1) KDA11-N01 can be activated by adherent cells PANC-1-A1101, HUCCT-1-G12D, and HPAF-II-A1101, and secreted IFN-γ, but KDA11-N01 cannot be activated by the antigen-negative adherent cells Hela-A2 (Figure 8D). These results suggest that KDA11-N01 has strong specificity.
2) KDA11-N02 can be activated by PANC-1-A1101, HUCCT-1-G12D, and HPAF-II-A1101, and secreted IFN-γ, but KDA11-N02 cannot be activated by the antigen-negative adherent cells Hela-A2 (Figure 8E). These results suggest that KDA11-N02 has strong specificity.

### Example 9 The TCRs of the Present Disclosure Efficiently Mediate Antigen-Specific T Cell Proliferation

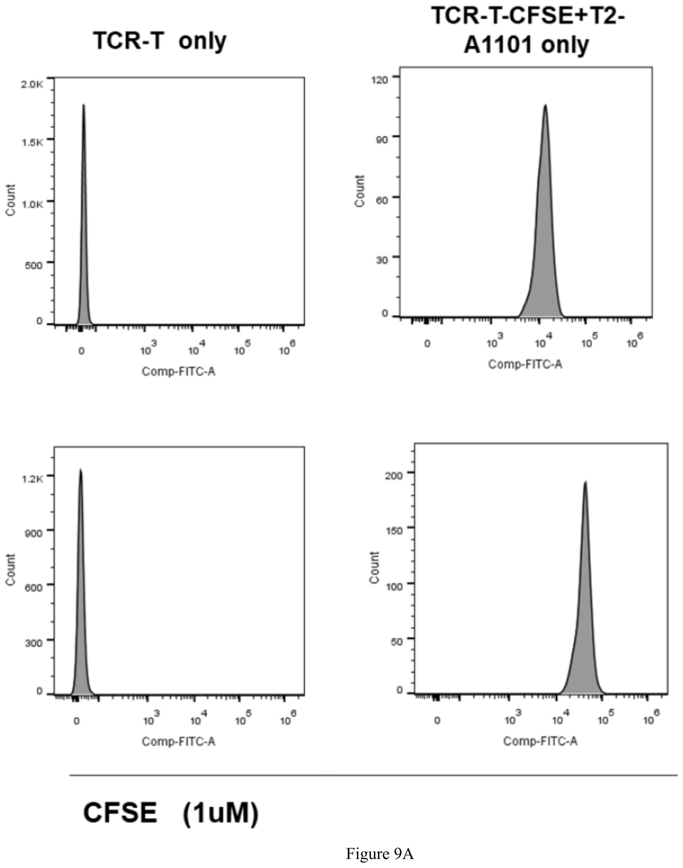

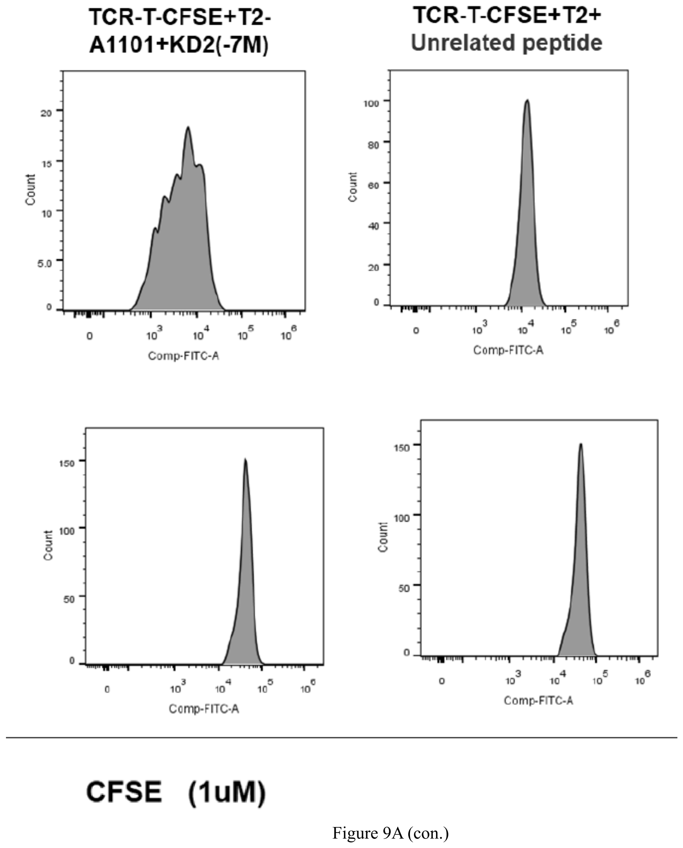

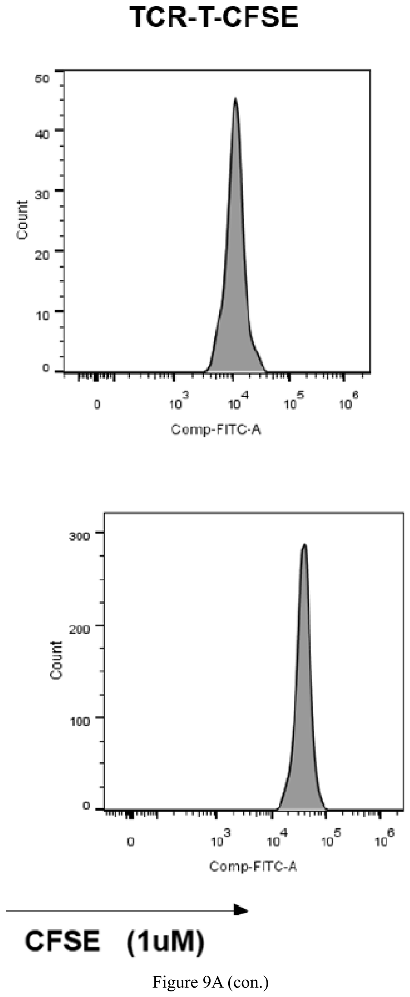

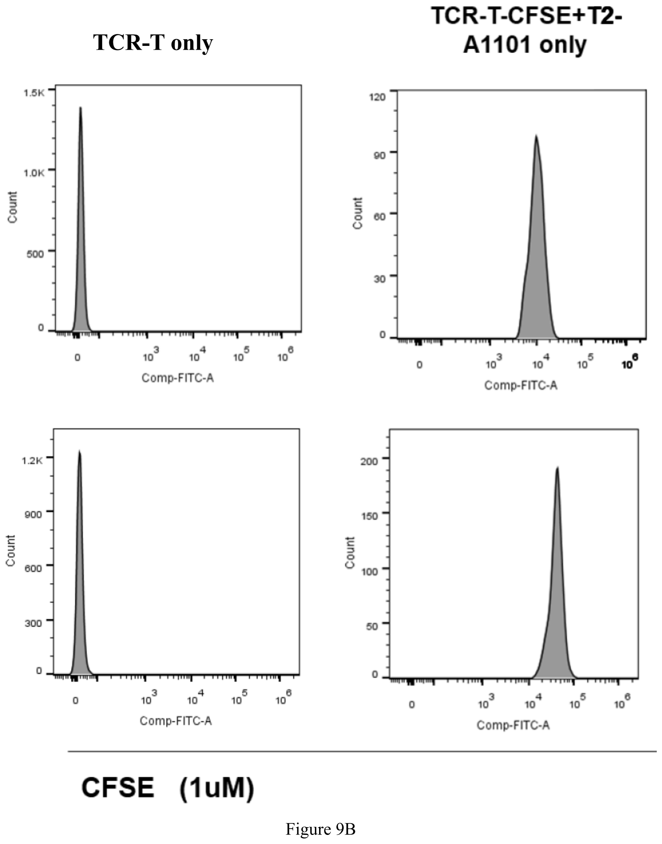

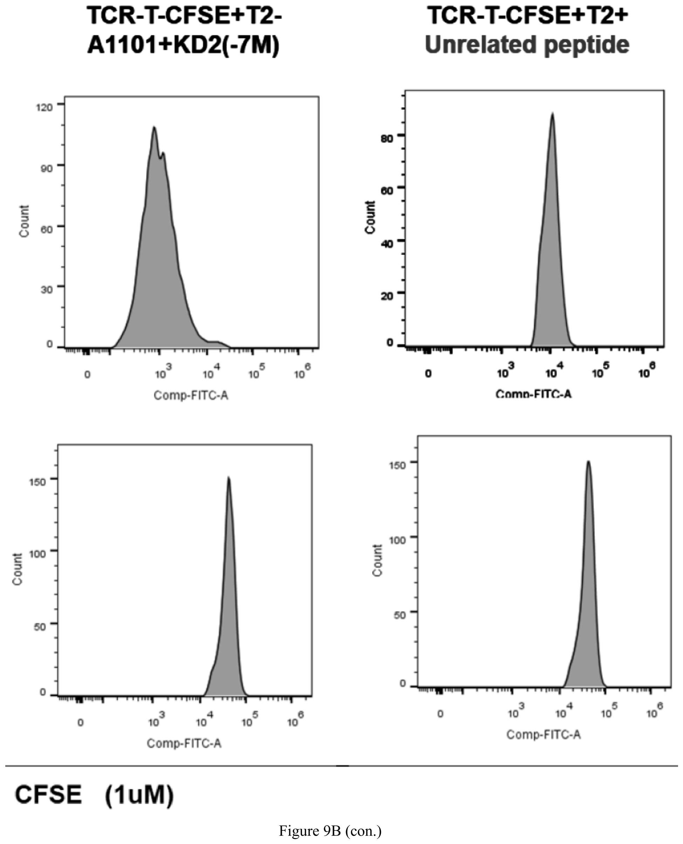

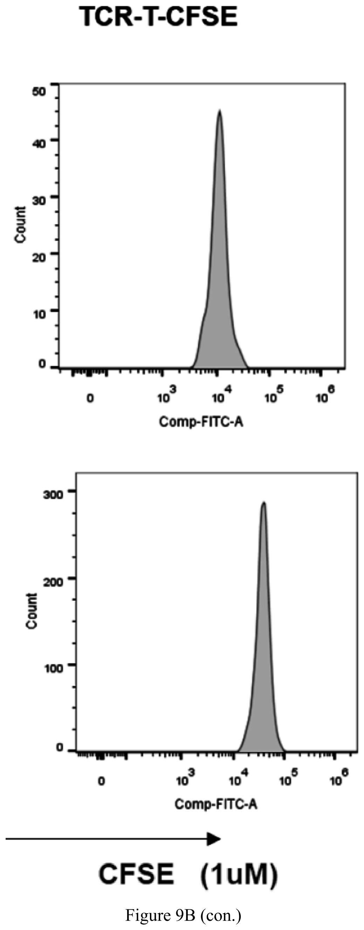

KDA11-N01 TCR-T cells, KDA11-N02 TCR-T cells, and non-transduced PBMCs were rested for 24 hours in T cell culture medium without IL-2 respectively, then stained and labeled with CFSE (C34554, Invitrogen) and co-cultured with T2 cells loaded with KRAS G12D 7~16 peptide for 3 days. The mixed cells were then stained with mouse TCRβ-APC and human CD8α-PE Cy7, and CD8 and mouse TCRβ double-positive cells were selected to analyze proliferation. T2 cells loaded with unrelated peptides served as peptide controls, and PBMCs not transduced with TCR served as negative controls. The results indicated that the KRAS G12D 7~16 peptide specifically stimulated the proliferation of both KDA11-N01 TCR-T cells and KDA11-N02 TCR-T cells, further confirming the antigen specificity of KDA11-N01 TCR-T cells and KDA11-N02 TCR-T cells (Figures 9A and 9B).

### Example 10 The TCRs of the Present Disclosure do not Exhibit Non-Specific Activation Against T2KO-TAP1 Cell Lines Expressing Different HLA Subtypes

To evaluate whether KVA11-N02, KVA11-N03, KVA11-N04, KDA11-N01, and KDA11-N02 induce alloreactivity, i.e., antigen-independent cross-reactivity induced with mismatched HLA molecules, T2KO-TAP1 cell lines (HLA knockout with TAP1 expression) were constructed to express 66 different HLA alleles respectively. These selected high-frequency HLA-A, HLA-B, and HLA-C can cover over 90% of the population. After transduction with different HLA genes, T2KO-TAP1 cells were sorted via flow cytometry to ensure HLA expression levels exceeded 95%. 1E5 TCR-T cells expressing the TCRs of the present disclosure were used as effector cells, and were co-cultured with 1E5 target cells for 16~24 hours, and then supernatant was collected for measuring IFN-γ levels. T2-A1101 cells loaded with 10^-6 M KRAS G12V 7~16 peptide, KRAS G12V 8~16 peptide, or KRAS G12D 7~16 peptide served as positive controls.

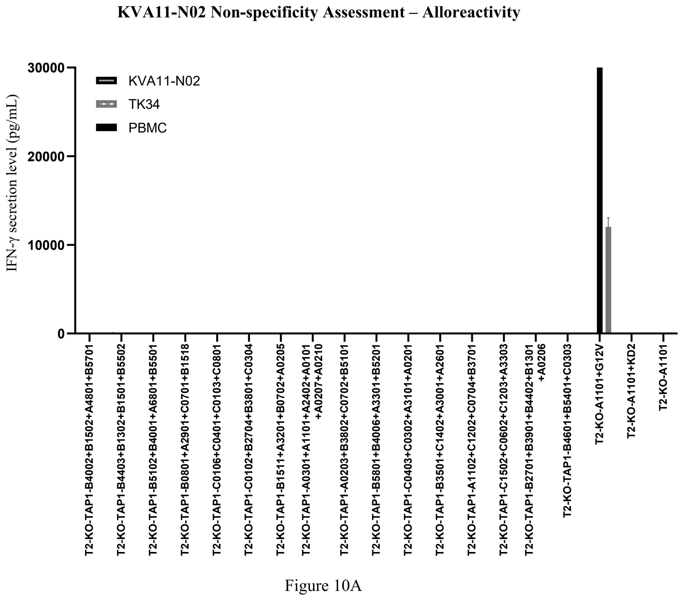

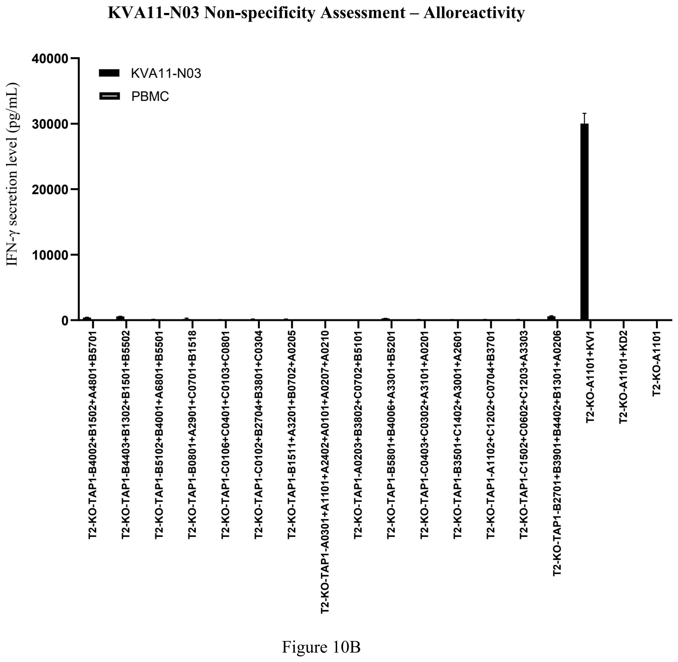

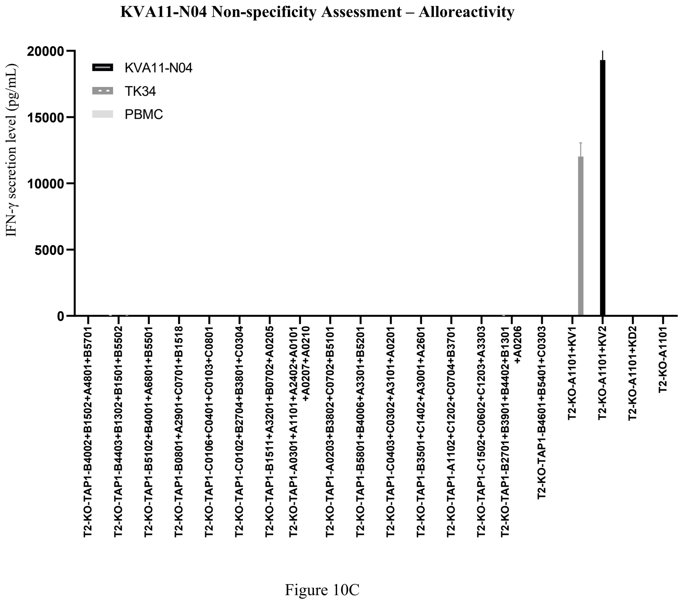

The results showed that none of the target cells had an obvious effect on stimulating IFN-γ secretion by KVAH-N02, KVA11-N03, or KVA11-N04 (Figures 10A-10C), indicating that KVAH-N02, KVA11-N03, and KVA11-N04 of the present disclosure do not exhibit alloreactivity toward different HLA subtypes, suggesting that they will not induce alloreactivity.

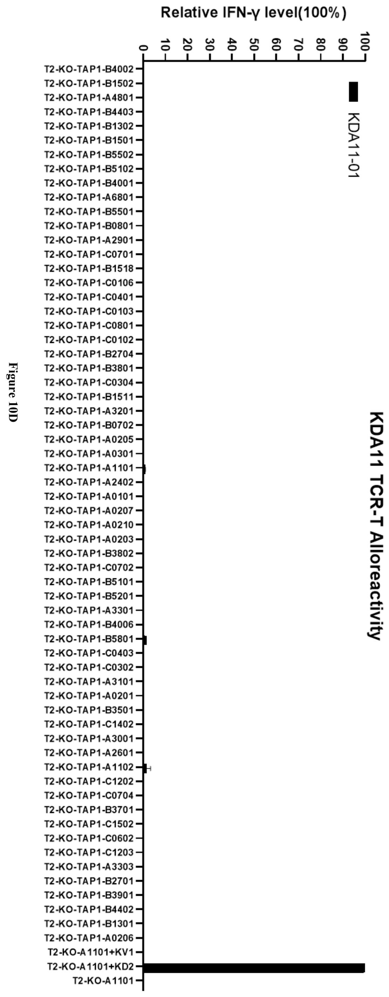

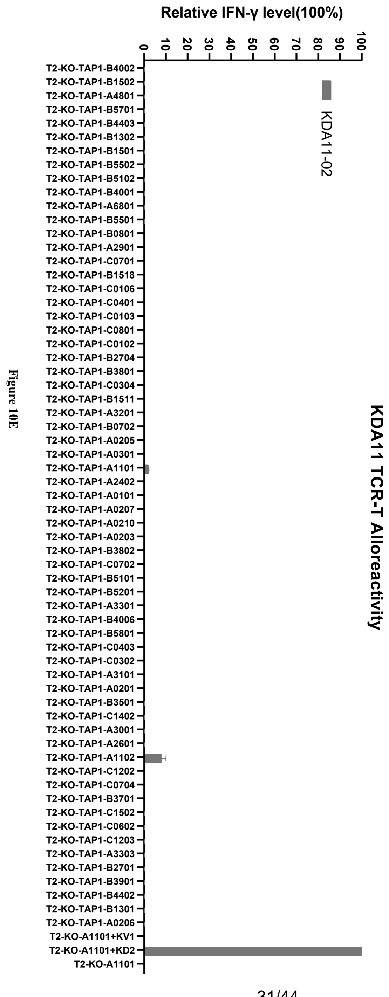

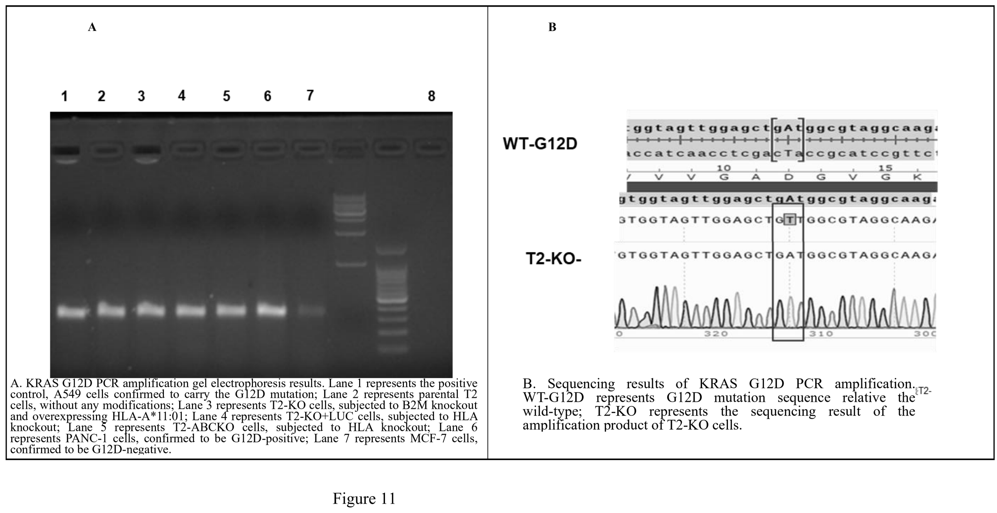

Similarly, none of the target cells had an obvious effect on stimulating IFN-γ secretion by KDA11-N01 and KDA11-N02 (Figures 10D-10E). However, T2-TAP-A1101, T2-TAP-A1102, and T2-TAP-B5801 cells induced IFN-γ secretion by KDA11-N01 (Figure 10D), and T2-TAP-A1101 and T2-TAP-A1102 induced IFN-γ secretion by KDA11-N02 (Figure 10E), suggesting that KDAH-N01 and KDA11-N02 may exhibit reactivity to a certain antigen peptide presented by HLA*A11:02. This antigen could be KRAS G12D or another unrelated peptide. Then, PCR was performed to detect the expression of the target antigen in T2 cells, followed by genomic sequencing of the amplification product. The results showed that T2 cells inherently express KRAS G12D (Figure 11).

### Example 11 The TCRs of the Present Disclosure do not Exhibit Non-Specific Activation Toward Cells from Different Tissue Origins

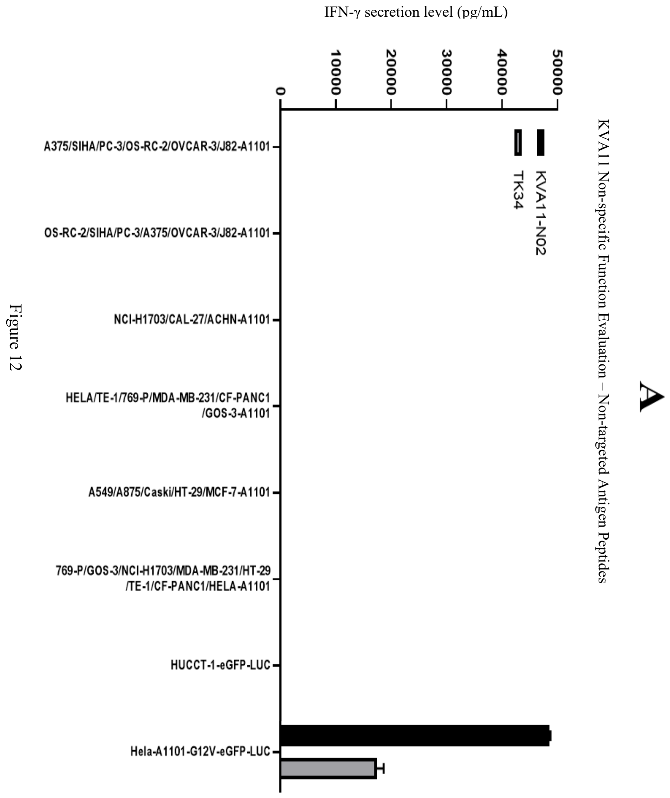

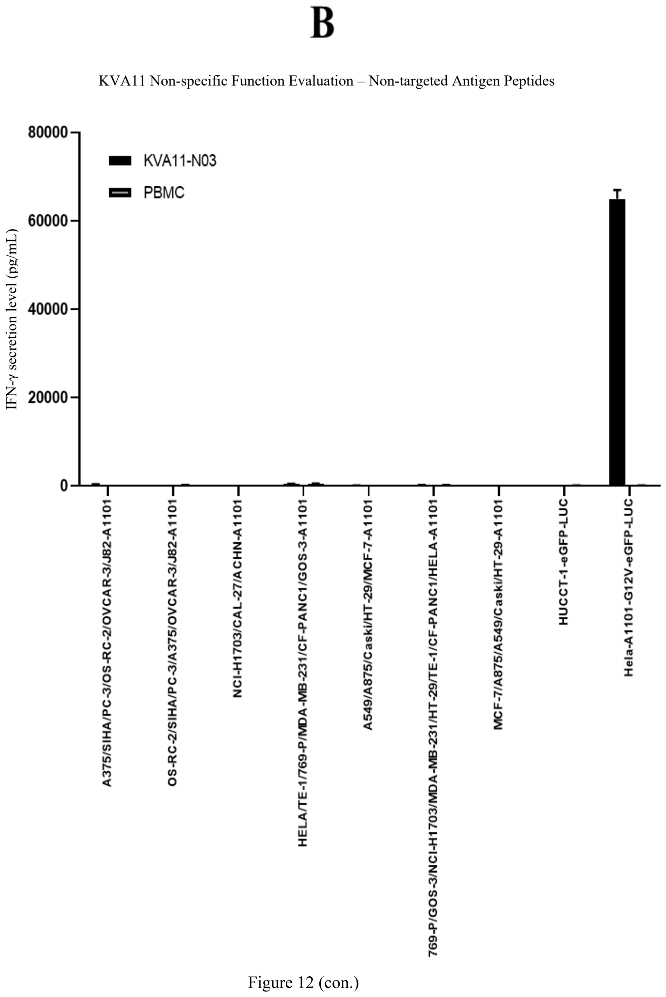

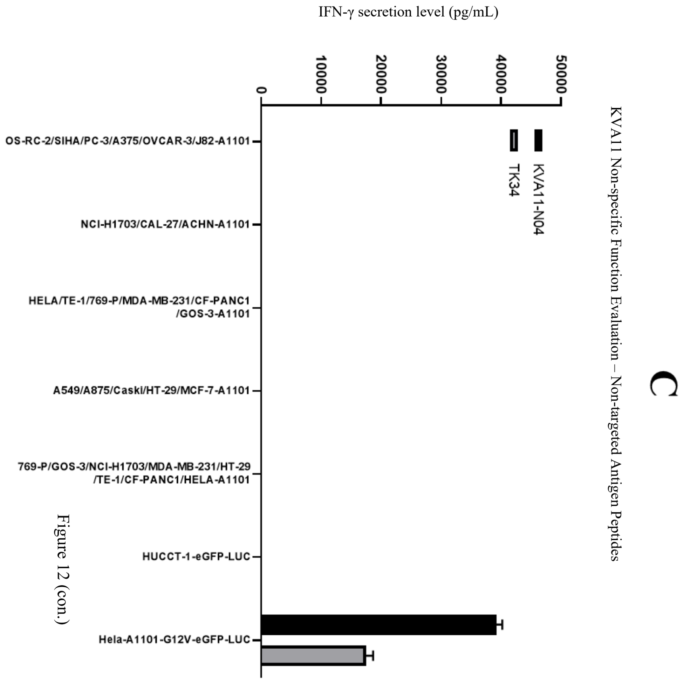

To evaluate the safety of KVA11-N02, KVA11-N03, and KVA11-N04, firstly, tumor cell lines derived from various human tissues were transduced with the HLA-A*11:01 gene and sorted via flow cytometry to ensure HLA-A*11:01 expression rate exceeded 90%. These cells were used as non-specific antigen-presenting cells to determine whether KVA11-N02, KVA11-N03, or KVA11-N04 responded to non-specific antigen. TCR-T cells expressing KVA11-N02, KVA11-N03, or KVA11-N04 were used as effector cells, and PBMCs that were not transduced with TCR were cultured and expanded in parallel as a control of the effector cells. Hela-G12V-1101 (overexpressing KRAS G12V and HLA-A*11:01) was used as a positive target cell. Tumor cell lines transduced with HLA-A*11:01 were served as target cells. 1E5 effector cells were co-incubated with different target cells at an effector-to-target (E:T) ratio of 1:1 for 24 hours. IFN-γ levels in the supernatant were measured using IFN-γ ELISA Kits (ExCell Bio, Cat# EH008-96). The results showed that all of KVA11-N02 TCR-T cells, KVA11-N03 TCR-T cells, and KVA11-N04 TCR-T cells did not secrete IFN-γ beyond control PBMC levels when exposed to non-specific antigen-presenting target cells, indicating no non-specific activation toward HLA-A*11:01-positive tumor cells from different tissue origins and demonstrating excellent antigen specificity (Figures 12A-12C).

### Example 12. Study on Non-specific Reactivity of Target Antigen Peptides for the TCRs of the Present Disclosure: X-SCAN: No Off-target Sites Identified

The peptide response fingerprint characteristics of the candidate TCRs were summarized by stimulating TCR-T cells to secrete interferon or activating reporter cell lines using antigen-presenting cells loaded with a mutated peptide library. The binding ability of peptides to MHC was determined using software; the ability of the synthesized peptides to activate TCR-T cells was detected; gene expression vectors were constructed and transduced into antigen-presenting cells to detect their antigen presentation ability; and the expression profiles of the antigens in different tissues were queried. Based on the results, the non-specific reactivity characteristics of the TCR-T cells were determined.

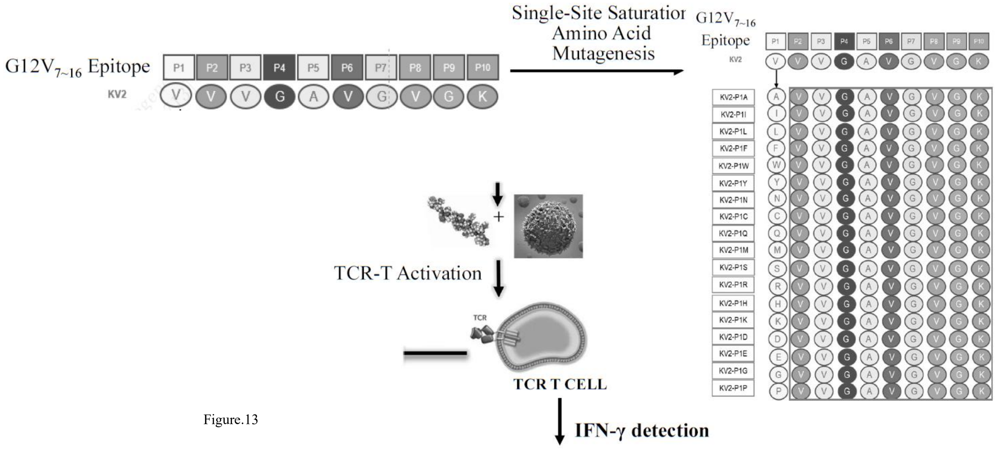

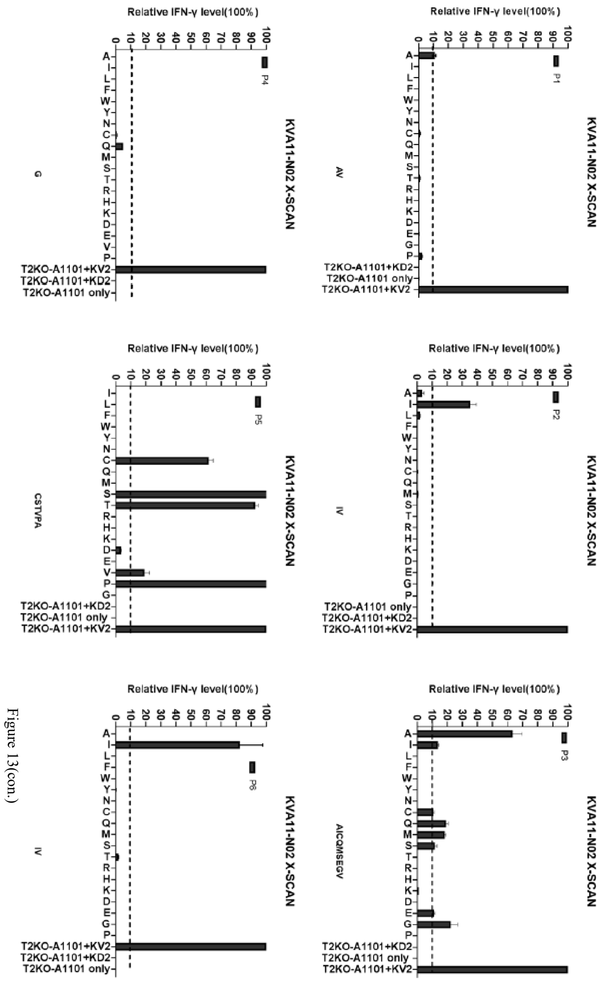

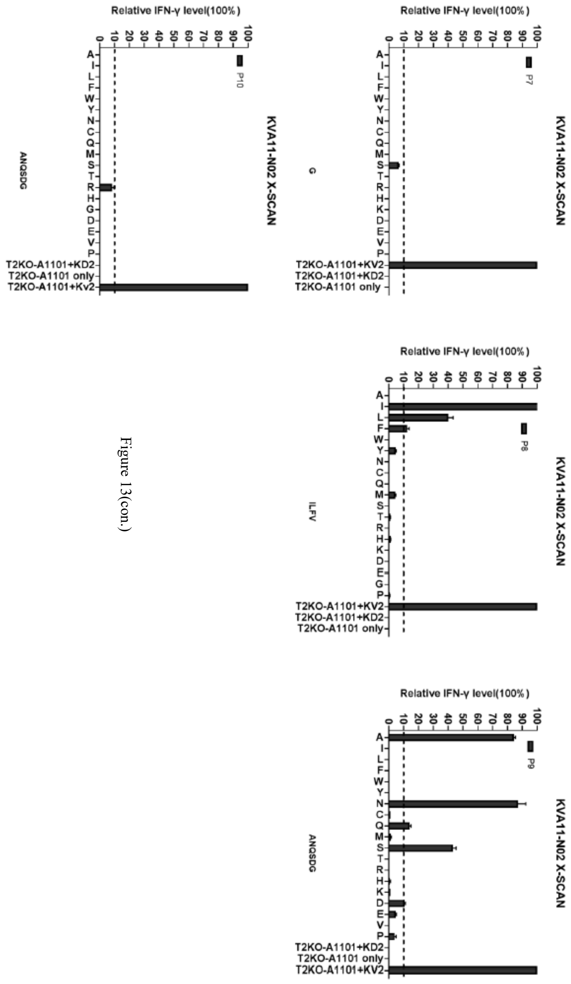

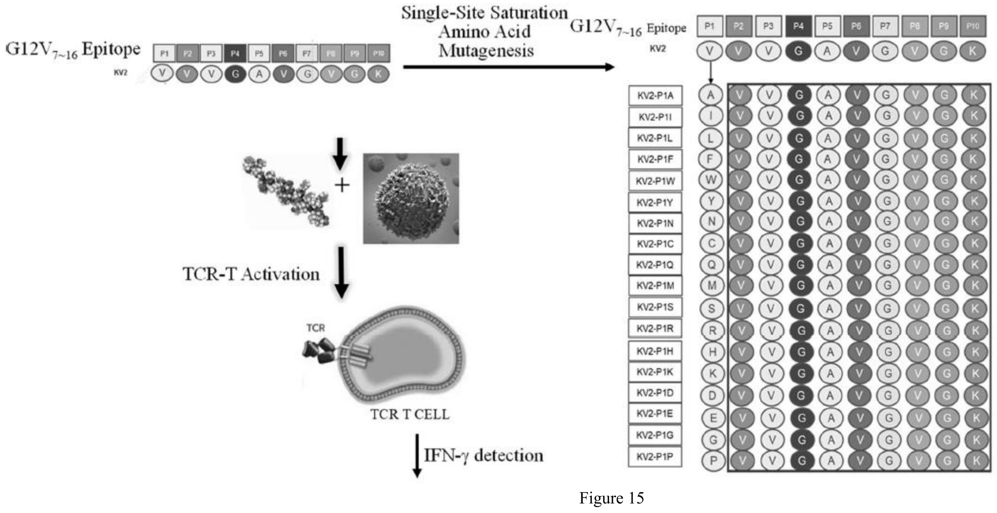

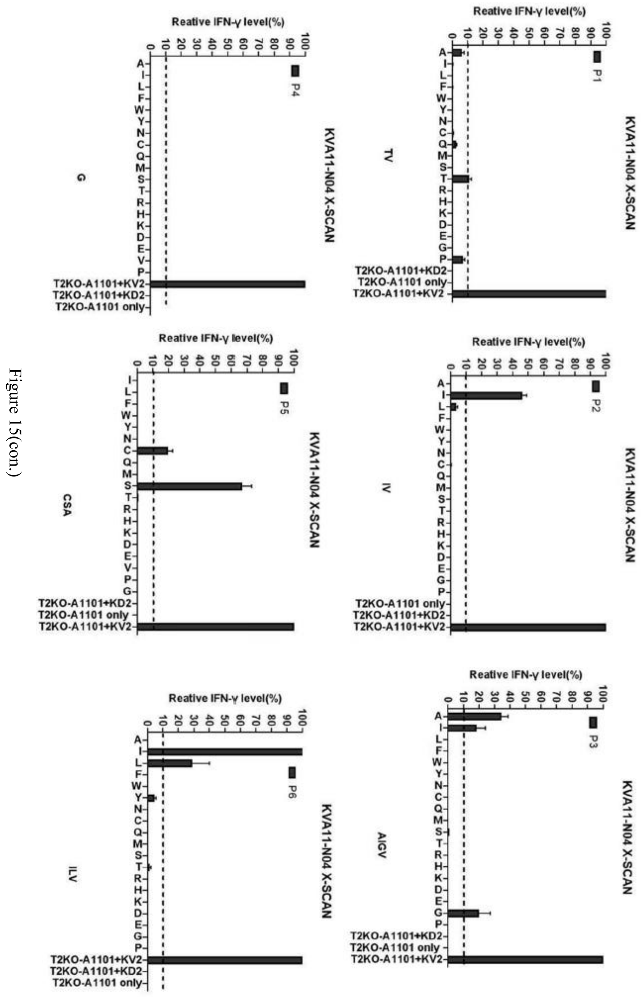

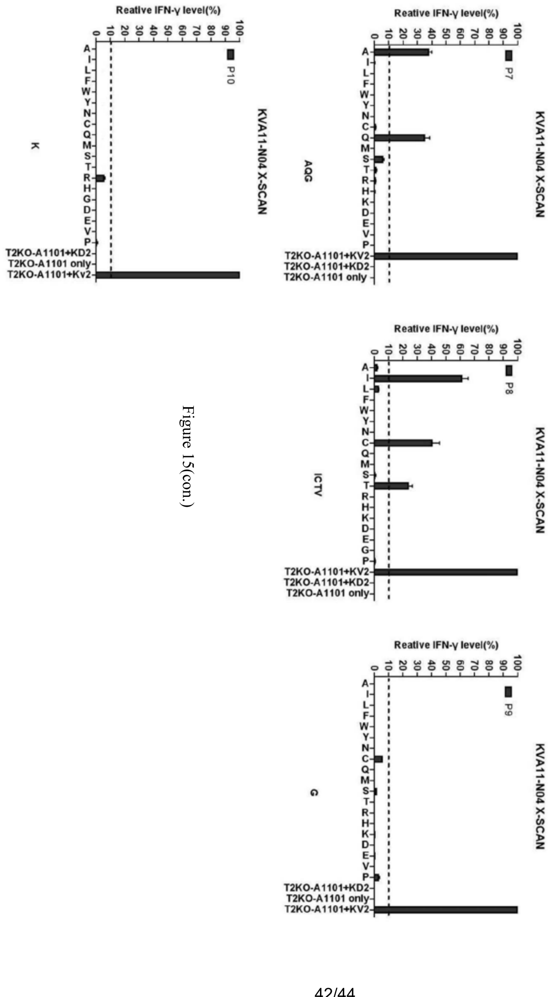

To comprehensively evaluate the non-specific reactivity of KVA11-N02 and KVA11-N04, a single-point mutation was performed on the target antigen peptide KRAS G12V 7~16, VVVGAVGVGK of KVA11-N02 and KVA11-N04. Mutation principle: keeping other amino acids of the target peptide unchanged, each amino acid at the mutation site was sequentially substituted with the other 19 amino acids, resulting in a total of 19×9 (number of amino acids in the peptide) mutated peptides. The peptide positions were labeled as P1, P2, P3, ... starting from the N-terminus. Take the G12V₈₋₁₆ peptide VVGAVGVGK for example, mutating P1 amino acid V to G, A, L, I, S, P, F, M, W, Q, T, C, N, Y, D, E, K, R, H, respectively, yielded 19 P1 mutated peptides, named KV1-P1X (X representing the mutated amino acid without further meaning), with sequences of XVGAVGVGK (wherein X represents the mutated amino acid, indicating any mutated amino acid), and individual peptides were named as KV1-P1G, KV1-P1A, etc. Adaptimmune used peptides with 90% purity; we ordered peptides of standard 95% purity from GenScript to comprehensively evaluate the specificity of KVA11-N02 and KVA11-N04. A total of 191 mutated peptides were tested. T2 cells were loaded with 10⁻⁷ M of each mutated peptide and co-incubated overnight with 1E5 KVA11-N02 TCR-T cells and KVA11-N04 TCR-T cells at a 1:1 ratio, respectively. IFN-γ secretion was measured using IFN-γ ELISA Kits. The reactivity of the point-mutated peptides was summarized: mutations that failed to stimulate TCR-T cells to produce IFN-γ were identified as critical amino acid, while the amino acids on the remaining positions were identified as non-critical amino acids. The detection results of KVA11-N02 TCR-T cells are shown in Figure 13; the detection results of KVA11-N04 TCR-T cells are shown in Figure 15.

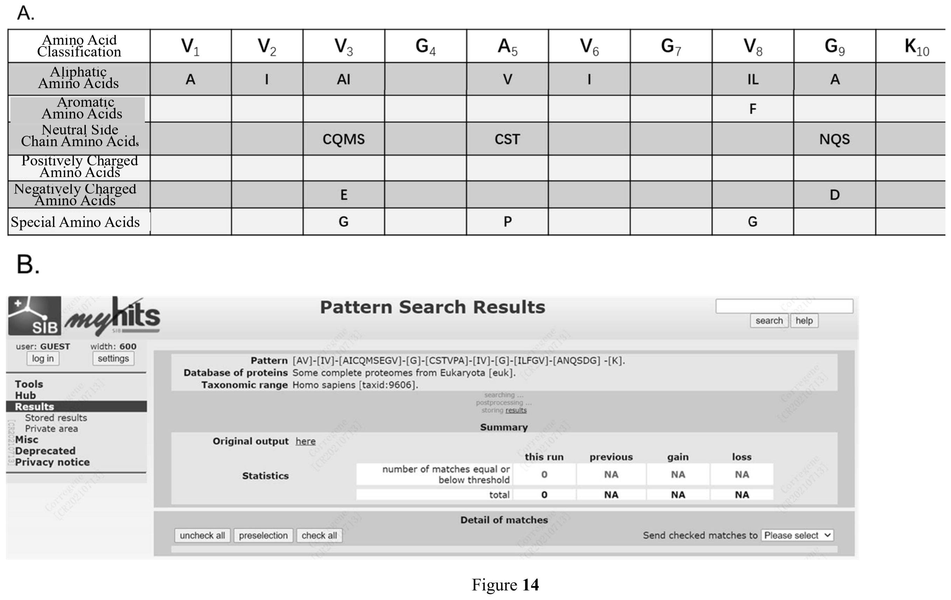

Summary of the positively reactive amino acids at each position that elicited IFN-γ response upon stimulation of TCR-T cells by KVA11-N02: P1[A**V**]-P2[I**V**]-P3[AICQMSEG**V**]-P4[**G**]-P5[CSTVP**A**]-P6[I**V**]-P7[**G**]-P8[ILFG**V**]-P9[ANQSD **G**]-P10[**K**] (Target peptide residues shown in bold.) A motif search in the human protein database (https://myhits.sib.swiss/cgi-bin/pattern_search) yielded zero potential reactive peptides (Figure 14). The more comprehensive evaluation results from the X-Scan clearly indicated that KVA11-N02 does not exhibit non-specific reactivity with endogenous human peptides, demonstrating good specificity.

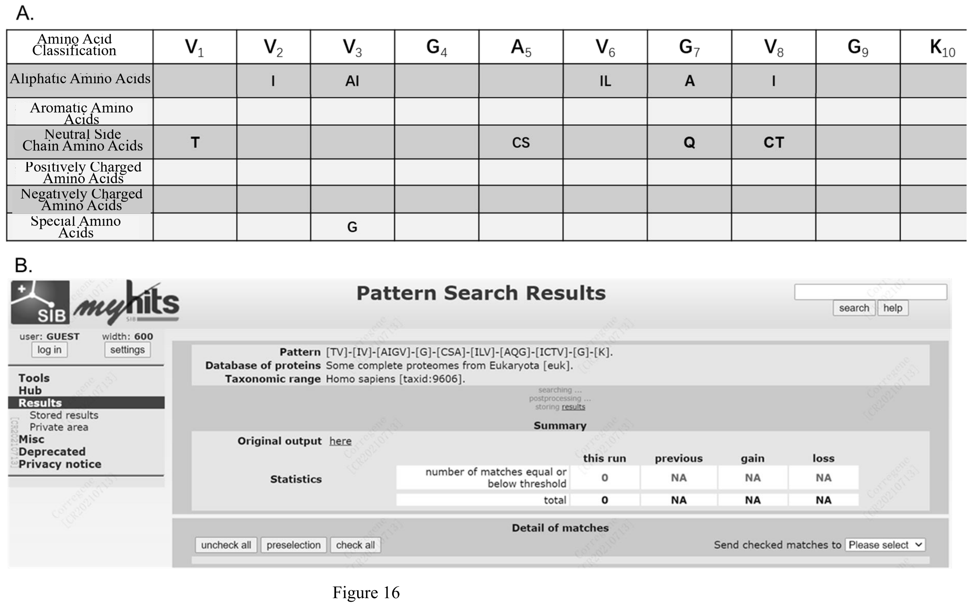

Summary of the positively reactive amino acids at each position that elicited IFN-γ response upon stimulation of TCR-T cells by KVA11-N04: P1[T**V**]-P2[I**V**]-P3[AIG**V**]-P4[**G**]-P5[CS**A**]-P6[IL**V**]-P7[AQ**G**]-P8[ICT**V**]-P9[**G**]-P10[**K**] (Target peptide residues shown in bold.) A motif search in the human protein database (https://myhits.sib.swiss/cgi-bin/pattern_search) yielded zero potential reactive peptides (Figure 16). The more comprehensive evaluation results from the X-Scan clearly indicated that KVA11-N04 does not exhibit non-specific reactivity with endogenous human peptides, demonstrating good specificity.

### Example 13. In Vivo Antitumor Activity

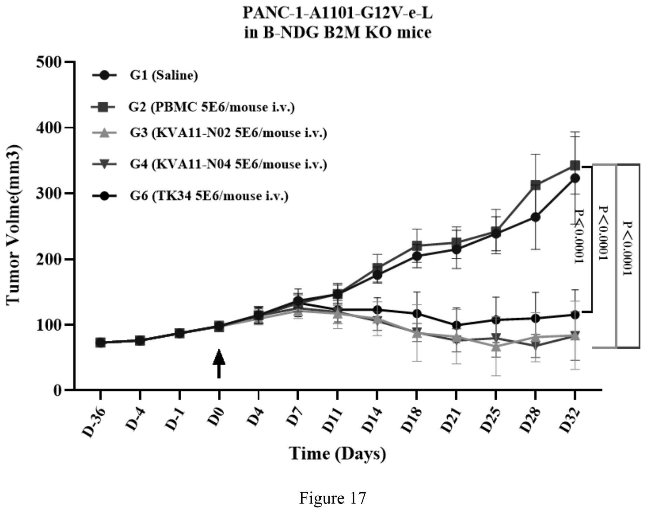

A tumor model was established using the pancreatic cancer cell line PANC-1 (KRAS G12V, A1101) in B-NDG B2M KO immunodeficient mice (5-6 weeks old, female). Subsequently, 5×10⁶ KVA11-N02 TCR-T cells and 5×10⁶ KVA11-N04 TCR-T cells were each administered via tail vein injection. As controls, 5×10⁶ PBMCs were administered as a negative control, 5×10⁶ TK34 TCR-T cells were administered as a positive control, and an equal volume of saline was administered as a blank control. The subcutaneous tumor growth in each group of mice was monitored, and the results are shown in Figure 17. As shown in Figure 17, compared with TK34, the TCR-T cells provided in the present disclosure more effectively inhibited tumor cell growth.

While the present invention has been described with reference to particular embodiments thereof, it should be understood by those skilled in the art that various changes can be made and equivalents can be substituted without departing from the true spirit and scope of the present invention. In addition, many modifications may be made to make particular instances, materials, compositions, methods, steps of methods suitable for the purpose, spirit and scope of the present invention. All such modifications are intended to be within the scope of the claims.

## Claims

1. An antigen-binding protein comprising an antigen binding domain or a fragment thereof of a T cell receptor (TCR), the antigen binding domain or fragment thereof comprising a T cell receptor (TCR) alpha chain variable region (Va) and a TCR beta chain variable region (Vβ), wherein the Vα comprises
a CDR3 having an amino acid sequence of X1X2X3X4X5X6X7X8X9X10X11X12X13LX15 (SEQ ID NO: 1), wherein:
X1 is A or V,
X2 is E or V,
X3 is R, P, N or null,
X4 is D, G, S, L or null,
X5 is I, G, D or null,
X6 is E, T or null,
X7 is G or null,
X8 is A, G, T or null,
X9 is G, S, A or null,
X10 is N, Y or G,
X11 is N, G, A or null,
X12 is R or null,
X13 is K or R,
X15 is I, T or M; or
a CDR3 having an amino acid sequence of X1X2X3X4X5X6GX8X9X10X11X12KLX15 (SEQ ID NO: 2), wherein:
X1 is A or V,
X2 is E or V,
X3 is R, P or N,
X4 is D, G or S,
X5 is I, G or D,
X6 is E, T or null,
X8 is A, G or T,
X9 is G or S,
X10 is N or Y,
X11 is N, G or null,
X12 is R or null,
X15 is I or T; or
a CDR3 having an amino acid sequence of X1VX3X4X6X7X8X9X10X11X13LX15 (SEQ ID NO: 3), wherein:
X1 is A or V,
X3 is N or null,
X4 is L or null,
X6 is T or null,
X7 is G or null,
X8 is A or null,
X9 is A or null,
X10 is N or G,
X11 is N or A,
X13 is K or R,
X15 is T or M; or
a CDR3 having an amino acid sequence of SEQ ID NOs: 22, 23, 24, 25 or 26, or a functional variant thereof formed by insertion, deletion, or substitution of one or more amino acids;
and/or
the Vβ comprises a CDR3 having an amino acid sequence of ASX3X4X5X6X7X8X9X10X11X12X13X14 (SEQ ID NO: 10), wherein:
X3 is S or T,
X4 is E, H, L, F or S,
X5 is G, W, V, S or I,
X6 is F, G, D or V,
X7 is Y, A or S,
X8 is T, Q, P, Y or S,
X9 is E, D, N or G,
X10 is Y, S, R or null,
X11 is G or null,
X12 is T, E, P or null,
X13 is A, Q or L,
X14 is F, Y or H; or
a CDR3 having an amino acid sequence of ASSX4X5X6X7X8X9X10X12X13X14 (SEQ ID NO: 11), wherein:
X4 is E, H or L,
X5 is G, W or V,
X6 is F or G,
X7 is Y, A or S,
X8 is T, Q or P,
X9 is E or D,
X10 is Y or null,
X12 is T, E or null,
X13 is A or Q,
X14 is F or Y; or
a CDR3 having an amino acid sequence of ASX3X4X5X6SX8X9X10X11X12X13X14 (SEQ ID NO: 12), wherein:
X3 is S or T,
X4 is F or S,
X5 is S or I,
X6 is D or V,
X8 is Y or S,
X9 is N or G,
X10 is S or R,
X11 is G or null,
X12 is E or P,
X13 is Q or L,
X14 is F or H; or
a CDR3 having an amino acid sequence of SEQ ID NOs: 37, 38, 39, 40 or 41, or a functional variant thereof formed by insertion, deletion, or substitution of one or more amino acids;
and wherein the antigen-binding domain or fragment thereof is antigen-specific for a mutated KRAS G12 epitope.

2. The antigen-binding protein according to claim 1, wherein the Vα comprises
a CDR1 having an amino acid sequence of X1X2X3X4X5X6 (SEQ ID NO: 4), wherein X1 is V, N or D; X2 is S or R; X3 is G or A; X4 is N or S; X5 is P, Q or D; X6 is Y or S; or
a CDR1 having an amino acid sequence of X1SX3X4X5X6 (SEQ ID NO: 5), wherein X1 is V or N; X3 is G or A; X4 is N or S; X5 is P, Q or D; X6 is Y or S; or
a CDR1 having an amino acid sequence of X1X2X3SQS (SEQ ID NO: 6), wherein X1 is N or D; X2 is S or R; X3 is G or A; or
a CDR1 having an amino acid sequence of SEQ ID NOs: 27, 28, 29, 30 or 31, or a functional variant thereof formed by insertion, deletion, or substitution of one or more amino acids;
and/or
the Vα comprises:
a CDR2 having an amino acid sequence of X1X2X3X4X5NX7X8X9 (SEQ ID NO: 7), wherein X1 is Y, V or null; X2 is I or Y; X3 is T, S, R or Y; X4 is G or S; X5 is D, G or null; X7 is L, M, G or null; X8 is V, D or null; X9 is K or null; or
a CDR2 having an amino acid sequence of X1X2X3X4X5NX7X8X9 (SEQ ID NO: 8), wherein X1 is Y, V or null; X2 is I or Y; X3 is T, S or R; X4 is G or S; X5 is D, G or null; X7 is L, M or null; X8 is V, D or null; X9 is K or null; or
a CDR2 having an amino acid sequence of X1X2X3SX5NX7X8 (SEQ ID NO: 9), wherein X1 is V or null; X2 is I or Y; X3 is S or Y; X5 is G or null; X7 is G or null; X8 is D or null; or
a CDR2 having an amino acid sequence of SEQ ID NOs: 32, 33, 34, 35 or 36, or a functional variant thereof formed by insertion, deletion, or substitution of one or more amino acids.

3. The antigen-binding protein according to claim 1 or 2, wherein the Vβ comprises
a CDR1 having an amino acid sequence of X1X2HX4X5 (SEQ ID NO: 13), wherein X1 is S, L or M; X2 is G or N; X4 is N, D, A or E; X5 is S, T or Y; or
a CDR1 having an amino acid sequence of X1GHX4X5 (SEQ ID NO: 14), wherein X1 is S or L; X4 is N, D or A; X5 is S or T; or
a CDR1 having an amino acid sequence of X1X2HX4X5 (SEQ ID NO: 15), wherein X1 is S or M; X2 is G or N; X4 is A or E; X5 is T or Y; or
a CDR1 having an amino acid sequence of SEQ ID NOs: 42, 43, 44, 45 or 46, or a functional variant thereof formed by insertion, deletion, or substitution of one or more amino acids;
and/or
the Vβ comprises
a CDR2 having an amino acid sequence of X1X2NX4X5X6 (SEQ ID NO: 16), wherein X1 is F, Y or S; X2 is N, Q or M; X4 is N, K or V; X5 is V, E or G; X6 is P, L or V; or
a CDR2 having an amino acid sequence of X1X2NX4X5X6 (SEQ ID NO: 17), wherein X1 is F or Y; X2 is N or Q; X4 is N or K; X5 is V, E or G; X6 is P, L or V; or
or a CDR2 having an amino acid sequence of X1X2NX4X5V (SEQ ID NO: 18), wherein X1 is F or S; X2 is Q or M; X4 is N or V; X5 is E or G; or
a CDR2 having an amino acid sequence of SEQ ID NOs: 47, 48, 49, 50 or 51, or a functional variant thereof formed by insertion, deletion, or substitution of one or more amino acids.

4. The antigen-binding protein according to any one of claims 1 to 3, wherein:
the Vα comprises a CDR3 having an amino acid sequence of X1X2X3X4X5X6GX8X9X10X11X12KLX15 (SEQ ID NO: 2), wherein: X1 is A or V, X2 is E or V, X3 is R, P or N, X4 is D, G or S, X5 is I, G or D, X6 is E, T or null, X8 is A, G or T, X9 is G or S, X10 is N or Y, X11 is N, G or null, X12 is R or null, X15 is I or T; or a CDR3 having an amino acid sequence of SEQ ID NOs: 22, 23 or 24, or a functional variant thereof formed by insertion, deletion or substitution of one or more amino acids; and
the Vα comprises a CDR1 having an amino acid sequence of X1SX3X4X5X6 (SEQ ID NO: 5), wherein: X1 is V or N, X3 is G or A, X4 is N or S, X5 is P, Q or D, X6 is Y or S; or a CDR1 having an amino acid sequence of SEQ ID NOs: 27, 28 or 29, or a functional variant thereof formed by insertion, deletion or substitution of one or more amino acids; and
the Vα comprises a CDR2 having an amino acid sequence of X1X2X3X4X5NX7X8X9 (SEQ ID NO: 8), wherein: X1 is Y, V or null, X2 is I or Y, X3 is T, S or R, X4 is G or S, X5 is D, G or null, X7 is L, M or null, X8 is V, D or null, X9 is K or null; or a CDR2 having an amino acid sequence of SEQ ID NOs: 32, 33 or 34, or a functional variant thereof formed by insertion, deletion or substitution of one or more amino acids;
and/or
the Vβ comprises a CDR3 having an amino acid sequence of ASSX4X5X6X7X8X9X10X12X13X14 (SEQ ID NO: 11), wherein X4 is E, H or L, X5 is G, W or V, X6 is F or G, X7 is Y, A or S, X8 is T, Q or P, X9 is E or D, X10 is Y or null, X12 is T, E or null, X13 is A or Q, X14 is F or Y; or a CDR3 having an amino acid sequence of SEQ ID NOs: 37, 38 or 39, or a functional variant thereof formed by insertion, deletion or substitution of one or more amino acids; and
the Vβ comprises a CDR1 having an amino acid sequence of X1GHX4X5 (SEQ ID NO: 14), wherein X1 is S or L, X4 is N, D or A, X5 is S or T; or a CDR1 having an amino acid sequence of SEQ ID NOs: 42, 43 or 44, or a functional variant thereof formed by insertion, deletion or substitution of one or more amino acids; and
the Vβ comprises a CDR2 having an amino acid sequence of X1X2NX4X5X6 (SEQ ID NO: 17), wherein: X1 is F or Y, X2 is N or Q, X4 is N or K, X5 is V, E or G, X6 is P, L or V; or a CDR2 having an amino acid sequence of SEQ ID NOs: 47, 48 or 49, or a functional variant thereof formed by insertion, deletion or substitution of one or more amino acids;
and wherein the antigen-binding domain or fragment thereof is antigen-specific for a mutated KRAS G12V epitope.

5. The antigen-binding protein according to any one of claims 1 to 3, wherein:
the Vα comprises a CDR3 having an amino acid sequence of X1VX3X4X6X7X8X9X10X11X13LX15 (SEQ ID NO: 3), wherein X1 is A or V, X3 is N or null, X4 is L or null, X6 is T or null, X7 is G or null, X8 is A or null, X9 is A or null, X10 is N or G, X11 is N or A, X13 is K or R, X15 is T or M; or a CDR3 having an amino acid sequence of SEQ ID NOs: 25 or 26, or a functional variant thereof formed by insertion, deletion or substitution of one or more amino acids; and
the Vα comprises a CDR1 having an amino acid sequence of X1X2X3SQS (SEQ ID NO: 6), wherein X1 is N or D, X2 is S or R, X3 is G or A; or a CDR1 having an amino acid sequence of SEQ ID NOs: 30 or 31, or a functional variant thereof formed by insertion, deletion or substitution of one or more amino acids; and
the Vα comprises a CDR2 having an amino acid sequence of X1X2X3SX5NX7X8 (SEQ ID NO: 9), wherein X1 is V or null, X2 is I or Y, X3 is S or Y, X5 is G or null, X7 is G or null, X8 is D or null; or a CDR2 having an amino acid sequence of SEQ ID NOs: 35 or 36, or a functional variant thereof formed by insertion, deletion or substitution of one or more amino acids;
and/or
the Vβ comprises a CDR3 having an amino acid sequence of ASX3X4X5X6SX8X9X10X11X12X13X14 (SEQ ID NO: 12), wherein X3 is S or T, X4 is F or S, X5 is S or I, X6 is D or V, X8 is Y or S, X9 is N or G, X10 is S or R, X11 is G or null, X12 is E or P, X13 is Q or L, X14 is F or H; or a CDR3 having an amino acid sequence of SEQ ID NOs: 40 or 41, or a functional variant thereof formed by insertion, deletion or substitution of one or more amino acids; and
the Vβ comprises a CDR1 having an amino acid sequence of X1X2HX4X5 (SEQ ID NO: 15), wherein X1 is S or M, X2 is G or N, X4 is A or E, X5 is T or Y; or a CDR1 having an amino acid sequence of SEQ ID NOs: 45 or 46, or a functional variant thereof formed by insertion, deletion or substitution of one or more amino acids; and
the Vβ comprises a CDR2 having an amino acid sequence of X1X2NX4X5V (SEQ ID NO: 18), wherein X1 is F or S, X2 is Q or M, X4 is N or V, X5 is E or G; or a CDR2 having an amino acid sequence of SEQ ID NOs: 50 or 51, or a functional variant thereof formed by insertion, deletion or substitution of one or more amino acids;
and wherein the antigen-binding domain or fragment thereof is antigen-specific for a mutated KRAS G12D epitope.

6. An antigen-binding protein comprising an antigen-binding domain or a fragment thereof of a T cell receptor (TCR), wherein the antigen-binding domain or fragment thereof comprises a TCR alpha chain variable region (Va) and a TCR beta chain variable region (Vβ),
wherein the Vα comprises a CDR3 having an amino acid sequence of SEQ ID NOs: 22, 23, 24, 25, or 26, or a functional variant thereof formed by insertion, deletion or substitution of one or more amino acids; and/or
the Vβ comprises a CDR3 having an amino acid sequence of SEQ ID NOs: 37, 38, 39, 40, or 41, or a functional variant thereof formed by insertion, deletion or substitution of one or more amino acids;
and wherein the antigen-binding domain or fragment thereof is antigen-specific for a mutated KRAS G12 epitope.

7. The antigen-binding protein according to claim 6, wherein the Vα comprises a CDR1 having an amino acid sequence of SEQ ID NOs: 27, 28, 29, 30, or 31, or a functional variant thereof formed by insertion, deletion or substitution of one or more amino acids; and/or
the Vα comprises a CDR2 having an amino acid sequence of SEQ ID NOs: 32, 33, 34, 35, or 36, or a functional variant thereof formed by insertion, deletion or substitution of one or more amino acids.

8. The antigen-binding protein according to claim 6 or 7, wherein the Vβ comprises a CDR1 having an amino acid sequence of SEQ ID NOs: 42, 43, 44, 45, or 46, or a functional variant thereof formed by insertion, deletion or substitution of one or more amino acids; and/or
the Vβ comprises a CDR2 having an amino acid sequence of SEQ ID NOs: 47, 48, 49, 50, or 51, or a functional variant thereof formed by insertion, deletion or substitution of one or more amino acids.

9. An antigen-binding protein comprising an antigen-binding domain or a fragment thereof of a T cell receptor (TCR), wherein the antigen-binding domain or fragment thereof comprises a TCR alpha chain variable region (Va) and a TCR beta chain variable region (Vβ);
wherein the Vα comprises a CDR1 having an amino acid sequence of SEQ ID NOs: 27, 28, 29, 30, or 31, or a functional variant thereof formed by insertion, deletion or substitution of one or more amino acids; a CDR2 having an amino acid sequence of SEQ ID NOs: 32, 33, 34, 35, or 36, or a functional variant thereof formed by insertion, deletion or substitution of one or more amino acids; and a CDR3 amino acid sequence selected from SEQ ID NOs: 22, 23, 24, 25, or 26, or a functional variant thereof formed by insertion, deletion or substitution of one or more amino acids;
and/or
the Vβ comprises a CDR1 having an amino acid sequence of SEQ ID NOs: 42, 43, 44, 45, or 46, or a functional variant thereof formed by insertion, deletion or substitution of one or more amino acids; a CDR2 having an amino acid sequence of SEQ ID NOs: 47, 48, 49, 50, or 51, or a functional variant thereof formed by insertion, deletion or substitution of one or more amino acids; and a CDR3 having an amino acid sequence of SEQ ID NOs: 37, 38, 39, 40, or 41, or a functional variant thereof formed by insertion, deletion or substitution of one or more amino acids.

10. The antigen-binding protein according to claim 9, wherein:
the Vα comprises CDR1, CDR2, and CDR3 having an amino acid sequences as shown in SEQ ID NOs: 27, 32, and 22 respectively, or functional variants thereof formed by insertion, deletion or substitution of one or more amino acids; and/or the Vβ comprises CDR1, CDR2, and CDR3 having an amino acid sequences as shown in SEQ ID NOs: 42, 47, and 37 respectively, or functional variants thereof formed by insertion, deletion or substitution of one or more amino acids;
the Vα comprises CDR1, CDR2, and CDR3 having an amino acid sequences as shown in SEQ ID NOs: 28, 33, and 23 respectively, or functional variants thereof formed by insertion, deletion or substitution of one or more amino acids; and/or the Vβ comprises CDR1, CDR2, and CDR3 having an amino acid sequences as shown in SEQ ID NOs: 43, 48, and 38 respectively, or functional variants thereof formed by insertion, deletion or substitution of one or more amino acids;
the Vα comprises CDR1, CDR2, and CDR3 having an amino acid sequences as shown in SEQ ID NOs: 29, 34, and 24 respectively, or functional variants thereof formed by insertion, deletion or substitution of one or more amino acids; and/or the Vβ comprises CDR1, CDR2, and CDR3 having an amino acid sequences as shown in SEQ ID NOs: 44, 49, and 39 respectively, or functional variants thereof formed by insertion, deletion or substitution of one or more amino acids;
the Vα comprises CDR1, CDR2, and CDR3 having an amino acid sequences as shown in SEQ ID NOs: 30, 35, and 25 respectively, or functional variants thereof formed by insertion, deletion or substitution of one or more amino acids; and/or the Vβ comprises CDR1, CDR2, and CDR3 having an amino acid sequences as shown in SEQ ID NOs: 45, 50, and 40 respectively, or functional variants thereof formed by insertion, deletion or substitution of one or more amino acids; or
the Vα comprises CDR1, CDR2, and CDR3 having an amino acid sequences as shown in SEQ ID NOs: 31, 36, and 26 respectively, or functional variants thereof formed by insertion, deletion or substitution of one or more amino acids; and/or the Vβ comprises CDR1, CDR2, and CDR3 having an amino acid sequences as shown in SEQ ID NOs: 46, 51, and 41 respectively, or functional variants thereof formed by insertion, deletion or substitution of one or more amino acids.

11. The antigen-binding protein according to any one of claims 1 to 10, wherein the Vα comprises an amino acid sequence having at least 80%, at least 85%, at least 90%, at least 95%, or 100% sequence identity to any one of SEQ ID NOs: 52, 53, 54, 55, or 56; and/or the Vβ comprises an amino acid sequence having at least 80%, at least 85%, at least 90%, at least 95%, or 100% sequence identity to any one of SEQ ID NOs: 57, 58, 59, 60, or 61;
preferably, the Vα comprises an amino acid sequence having at least 80%, at least 85%, at least 90%, at least 95%, or 100% sequence identity to SEQ ID NO: 52, and/or the Vβ comprises an amino acid sequence having at least 80%, at least 85%, at least 90%, at least 95%, or 100% sequence identity to SEQ ID NO: 57; or
the Vα comprises an amino acid sequence having at least 80%, at least 85%, at least 90%, at least 95%, or 100% sequence identity to SEQ ID NO: 53, and/or the Vβ comprises an amino acid sequence having at least 80%, at least 85%, at least 90%, at least 95%, or 100% sequence identity to SEQ ID NO: 58; or
the Vα comprises an amino acid sequence having at least 80%, at least 85%, at least 90%, at least 95%, or 100% sequence identity to SEQ ID NO: 54, and/or the Vβ comprises an amino acid sequence having at least 80%, at least 85%, at least 90%, at least 95%, or 100% sequence identity to SEQ ID NO: 59; or
the Vα comprises an amino acid sequence having at least 80%, at least 85%, at least 90%, at least 95%, or 100% sequence identity to SEQ ID NO: 55, and/or the Vβ comprises an amino acid sequence having at least 80%, at least 85%, at least 90%, at least 95%, or 100% sequence identity to SEQ ID NO: 60; or
the Vα comprises an amino acid sequence having at least 80%, at least 85%, at least 90%, at least 95%, or 100% sequence identity to SEQ ID NO: 56, and/or the Vβ comprises an amino acid sequence having at least 80%, at least 85%, at least 90%, at least 95%, or 100% sequence identity to SEQ ID NO: 61.

12. The antigen-binding protein according to any one of claims 1 to 11, wherein the antigen-binding protein binds to a KRAS G12 mutant epitope or a complex of the epitope with an MHC molecule; wherein the KRAS G12 mutant epitope comprises at least one of G12V and G12D;
preferably, the MHC molecule is HLA-A*11 type, for example HLA-A*11:01.

13. The antigen-binding protein according to any one of claims 1 to 12, wherein the antigen-binding protein exhibits one or more of the following properties:
1) capable of binding to the target antigenic peptide with an ECso of less than or equal to about 1.0×10⁻⁷ M;
2) high membrane stability;
3) specific killing activity against antigen-positive tumor cells; and
4) absence of alloreactivity to different HLA subtypes.

14. The antigen-binding protein according to any one of claims 1 to 13, wherein the Vα is comprised on a first polypeptide and the Vβ is comprised on a different second polypeptide; or
the Vα and the Vβ are comprised on a single polypeptide.

15. The antigen-binding protein according to any one of claims 1 to 14, wherein the antigen-binding protein is soluble or membrane-bound.

16. The antigen-binding protein according to any one of claims 1 to 15, wherein the antigen-binding protein is selected from a TCR, a chimeric antigen receptor (CAR), an Fc polypeptide, or an antigen-binding fragment thereof;
preferably, the antigen-binding protein is a TCR or an antigen-binding fragment thereof, and the antigen-binding protein further comprises a TCR constant region or a fragment thereof; and/or the antigen-binding protein further comprises a transmembrane region and/or a cytoplasmic region; and/or the antigen-binding protein further comprises an intracellular signaling region.

17. The antigen-binding protein according to claim 16, wherein the TCR constant region is a murine constant region or a human constant region; and/or
the TCR constant region comprises a TCR α chain constant region and/or a TCR β chain constant region;
preferably, the TCR α chain constant region and/or the TCR β chain constant region comprises at least one cysteine mutation relative to a wild-type sequence, so as to form a disulfide bond between the TCR α chain and the TCR β chain.

18. The antigen-binding protein according to claim 17, wherein the TCR α chain constant region comprises an amino acid sequence having at least 80%, at least 85%, at least 90%, at least 95%, or 100% sequence identity to any one of SEQ ID NOs: 62-66 and 105, and/or, the TCR β chain constant region comprises an amino acid sequence having at least 80%, at least 85%, at least 90%, at least 95%, or 100% sequence identity to any one of SEQ ID NOs: 67-76;
preferably, the TCR α chain constant region comprises an amino acid sequence as shown in SEQ ID NO: 66 and the TCR β chain constant region comprises an amino acid sequence as shown in SEQ ID NO: 72.

19. The antigen-binding protein according to claim 16, wherein the fragment of the TCR constant region is an extracellular domain of the TCR constant region.

20. The antigen-binding protein according to any one of claims 16 to 19, wherein the antigen-binding protein comprises:
a TCR α chain comprising an amino acid sequence having at least 80%, at least 85%, at least 90%, at least 95%, or 100% sequence identity to any one of SEQ ID NOs: 77-81; and/or, a TCR β chain comprising an amino acid sequence having at least 80%, at least 85%, at least 90%, at least 95%, or 100% sequence identity to any one of SEQ ID NOs: 82-86;
preferably, the antigen-binding protein comprises a TCR α chain with an amino acid sequence having at least 80%, at least 85%, at least 90%, at least 95%, or 100% sequence identity to SEQ ID NO: 77, and a TCR β chain with an amino acid sequence having at least 80%, at least 85%, at least 90%, at least 95%, or 100% sequence identity to SEQ ID NO: 82; or
the antigen-binding protein comprises a TCR α chain with an amino acid sequence having at least 80%, at least 85%, at least 90%, at least 95%, or 100% sequence identity to SEQ ID NO: 78, and a TCR β chain with an amino acid sequence having at least 80%, at least 85%, at least 90%, at least 95%, or 100% sequence identity to SEQ ID NO: 83; or
the antigen-binding protein comprises a TCR α chain with an amino acid sequence having at least 80%, at least 85%, at least 90%, at least 95%, or 100% sequence identity to SEQ ID NO: 79, and a TCR β chain with an amino acid sequence having at least 80%, at least 85%, at least 90%, at least 95%, or 100% sequence identity to SEQ ID NO: 84; or
the antigen-binding protein comprises a TCR α chain with an amino acid sequence having at least 80%, at least 85%, at least 90%, at least 95%, or 100% sequence identity to SEQ ID NO: 80, and a TCR β chain with an amino acid sequence having at least 80%, at least 85%, at least 90%, at least 95%, or 100% sequence identity to SEQ ID NO: 85; or
the antigen-binding protein comprises a TCR α chain with an amino acid sequence having at least 80%, at least 85%, at least 90%, at least 95%, or 100% sequence identity to SEQ ID NO: 81, and a TCR β chain with an amino acid sequence having at least 80%, at least 85%, at least 90%, at least 95%, or 100% sequence identity to SEQ ID NO: 86.

21. The antigen-binding protein according to any one of claims 1 to 20, wherein the antigen-binding protein further comprises one or more antigen-binding domains binding other antigens or epitopes.

22. The antigen-binding protein according to any one of claims 1 to 21, wherein the antigen-binding protein is isolated or purified.

23. A multispecific antibody comprising the antigen-binding protein according to any one of claims 1 to 20.

24. A nucleic acid encoding the antigen-binding protein according to any one of claims 1 to 22 or the multispecific antibody according to claim 23.

25. The nucleic acid according to claim 24, wherein the nucleic acid comprises a nucleotide sequence having at least 80%, at least 85%, at least 90%, at least 95%, or 100% sequence identity to any one of SEQ ID NOs: 87-91; and/or, a nucleotide sequence having at least 80%, at least 85%, at least 90%, at least 95%, or 100% sequence identity to any one of SEQ ID NOs: 92-96.

26. A vector comprising the nucleic acid according to claim 24 or 25.

27. The vector according to claim 26, wherein the vector is selected from the group consisting of lentiviral vectors, retroviral vectors, plasmids, DNA vectors, mRNA vectors, transposon-based vectors and artificial chromosomes.

28. A cell comprising or expressing the antigen-binding protein according to any one of claims 1 to 22, the multispecific antibody according to claim 23, the nucleic acid according to claim 24 or 25, or the vector according to claim 26 or 27.

29. The cell according to claim 28, wherein the cell is selected the group consisting of lymphocytes (e.g. T cells, NK cells), monocytes (e.g. PBMCs) and stem cells;
preferably, the stem cells are lymphoid progenitor cells or induced pluripotent stem cells (iPSCs);
more preferably, the cell is a T cell.

30. The cell according to claim 28 or 29, wherein the T cell does not express an endogenous TCR.

31. A method of producing the cell according to any one of claims 28 to 30, comprising a step of transducing or transfecting a cell with the vector according to claim 26 or 27.

32. The method according to claim 31, further comprising a step of amplifying and/or activating the cell prior to or after the transduction or transfection.

33. A conjugate comprising the antigen-binding protein according to any one of claims 1 to 23 or the multispecific antibody according to claim 23, and an active agent conjugated or coupled to the antigen-binding protein or the multispecific antibody.

34. The conjugate according to claim 33, wherein the active agent is selected from the group consisting of a detectable marker, an immunostimulatory molecule and a therapeutic agent;
preferably, the detectable marker is selected from the group consisting of biotins, streptavidin, enzymes or catalytically active fragments thereof, radionuclides, nanoparticles, paramagnetic metal ions, nucleic acid probes, contrast agents, and fluorescent, phosphorescent or chemiluminescent molecules;
preferably, the immunostimulatory molecule is selected from the group consisting of cytokines, chemokines, platelet factors and complement initiators;
preferably, the therapeutic agent is selected from the group consisting of immunomodulators, radioactive compounds (e.g. nuclide), enzymes, chemotherapeutic agents and toxins.

35. A composition comprising the antigen-binding protein according to any one of claims 1 to 22, the multispecific antibody according to claim 23, the nucleic acid according to claim 24 or 25, the vector according to claim 26 or 27, or the cell according to any one of claims 28 to 30;
preferably, the composition further comprises a pharmaceutically acceptable carrier or excipient.

36. The composition according to claim 35, further comprising a second therapeutic agent;
preferably the second therapeutic agent is selected from the group consisting of antibodies, chemotherapeutic agents, and small molecule drugs.

37. A kit comprising the antigen-binding protein according to any one of claims 1 to 22, the multispecific antibody according to claim 23, or the conjugate according to claim 32 or 33.
